(19) Europäisches Patentamt
European Patent Office
Office européen des brevets

(11) Veröffentlichungsnummer : **0 044 807**
**B1**

(12) # EUROPÄISCHE PATENTSCHRIFT

(45) Veröffentlichungstag der Patentschrift : **21.11.85**

(21) Anmeldenummer : **81810281.6**

(22) Anmeldetag : **13.07.81**

(51) Int. Cl.⁴ : **A 01 N 47/36**, C 07 D251/16, C 07 D251/22, C 07 D251/46, C 07 D239/42, C 07 D239/46// C07C143/78, C07C147/06, C07C147/14, C07C149/443

(54) **N-Phenylsulfonyl-N'-pyrimidinyl- und -triazinylharnstoffe.**

(30) Priorität : **17.07.80 CH 5481/80**
**05.11.80 CH 8216/80**
**17.06.81 CH 3991/81**

(43) Veröffentlichungstag der Anmeldung : **27.01.82 Patentblatt 82/04**

(45) Bekanntmachung des Hinweises auf die Patenterteilung : **21.11.85 Patentblatt 85/47**

(84) Benannte Vertragsstaaten :
**AT BE DE FR GB IT NL SE**

(56) Entgegenhaltungen :
**EP-A- 0 001 514**
**EP-A- 0 001 515**
**EP-A- 0 007 687**
**EP-A- 0 009 419**
**EP-A- 0 023 422**
**EP-A- 0 024 215**
**EP-A- 0 035 893**
**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Postfach**
**CH-4002 Basel (CH)**

(72) Erfinder : **Meyer, Willy**
**Talstrasse 49**
**CH-4125 Riehen (CH)**
Erfinder : **Föry, Werner, Dr.**
**Benkenstrasse 65**
**CH-4054 Basel (CH)**

**EP 0 044 807 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue, herbizid wirksame und pflanzenwuchsregulierende N-Phenylsulfonyl-N'-pyrimidinyl- und -triazinyl-harnstoffe, Verfahren zu ihrer Herstellung, sie als Wirkstoffe enthaltende Mittel, sowie deren Verwendung zum Bekämpfen von Unkräutern, vor allem selektiv in Nutzpflanzenkulturen oder zum Regulieren und Hemmen des Pflanzenwachstums. Darüber hinaus betrifft die Erfindung auch als Zwischenprodukte hergestellte neue Phenylsulfonamide.

Die erfindungsgemässen N-Phenylsulfonyl-N'-pyrimidinyl- und -triazinylharnstoffe entsprechen der allgemeinen Formel I

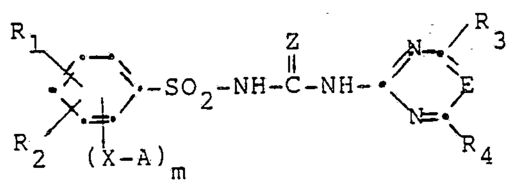

(I)

worin

A einem durch $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Halogenalkoxy, $C_1$-$C_4$-Halogenalkylthio, $C_1$-$C_4$-Halogenalkylsulfinyl oder $C_1$-$C_4$-Halogenalkylsulfonyl substituierten $C_1$-$C_6$-Alkylrest oder einen gegebenenfalls durch die aufgezählten Substituenten substituierten $C_2$-$C_6$-Alkenylrest oder einen $C_2$-$C_6$-Halogenalkenylrest,

E die Methingruppe oder Stickstoff,

X Sauerstoff, Schwefel, eine Sulfinyl- oder Sulfonylbrücke,

Z Sauerstoff oder Schwefel,

m die Zahl eins oder zwei,

$R_1$ Wasserstoff, Halogen, $C_1$-$C_5$-Alkyl, $C_2$-$C_5$-Alkenyl oder einen Rest —Y—$R_5$,

$R_2$ Wasserstoff, Halogen, $C_1$-$C_5$-Alkyl, $C_2$-$C_5$-Alkenyl, $C_1$-$C_4$-Halogen-alkyl, oder einen Rest —Y—$R_5$, —$COOR_6$, —$NO_2$ oder —CO—$NR_7R_8$,

$R_3$ und $R_4$ unabhängig voneinander Wasserstoff, $C_1$-$C_4$-Alkyl, $C_1$-$C_4$-Alkoxy, $C_1$-$C_4$-Alkylthio, $C_1$-$C_4$-Halogenalkyl, Halogen oder Alkoxyalkyl mit höchstens 4 Kohlenstoffatomen,

$R_5$ und $R_6$ je $C_1$-$C_5$-Alkyl, $C_2$-$C_5$-Alkenyl oder $C_2$-$C_6$-Alkinyl,

$R_7$ und $R_8$ unabhängig voneinander Wasserstoff, $C_1$-$C_5$-Alkyl, $C_2$-$C_5$-Alkenyl oder $C_2$-$C_6$-Alkinyl und

Y Sauerstoff, Schwefel, eine Sulfinyl- oder Sulfonylbrücke bedeuten,

mit der Massgabe, dass m die Zahl zwei bedeutet, wenn gleichzeitig X für Schwefel, eine Sulfinyl- oder Sulfonylbrücke und A für $C_3$-$C_4$-Alkenyl stehen, sowie den Salzen dieser Verbindungen.

Harnstoffverbindungen, Triazinverbindungen und Pyrimidinverbindungen mit herbizider Wirkung sind allgemein bekannt. Kürzlich wurden Arylsulfamoyl-heterocyclyl-aminocarbamoylverbindungen mit herbizider und pflanzenwuchsregulierender Wirkung, beispielsweise in den europäischen Patentpublikationen Nr. 1514, 1515, dem US Patent No. 4 127 405, in der DT-OS 2 715 786 oder in der FR-PS 1 468 747 beschrieben. In der nicht vorveröffentlichten europäischen Patentpublikation EP-A-35893 werden den erfindungsgemässen Substanzen strukturähnliche Verbindungen gleicher Wirkungsrichtung offenbart.

In den Definitionen ist unter Alkyl geradkettiges oder verzweigtes Alkyl zu verstehen ; z. B. : Methyl, Aethyl, n-Propyl, i-Propyl, die vier isomeren Butyl, n-Amyl, i-Amyl, 2-Amyl, 3-Amyl, n-Hexyl oder i-Hexyl, vorzugsweise ist der Alkylrest jedoch geradkettig.

Unter Alkoxy ist zu verstehen : Methoxy, Aethoxy, n-Propyloxy, i-Propyloxy und vier isomeren Butyloxyreste, insbesondere aber Methoxy oder Aethoxy.

Beispiele für Alkythio sind Methylthio, Aethylthio, n-Propylthio, i-Propylthio und n-Butylthio, insbesondere aber Methylthio und Aethylthio.

Beispiele für Alkenylreste sind Vinyl, Allyl, Isopropenyl, 1-Propenyl, 1-Butenyl, 2-Butenyl, 3-Butenyl, 1-Isobutenyl, 2-Isobutenyl, 1-Pentenyl, 2-Pentenyl, 3-Pentenyl und 4-Pentenyl, insbesondere aber Vinyl, Allyl und 4-Pentenyl.

Beispiele für Alkylsulfinyl sind Methylsulfinyl, Aethylsulfinyl, n-Propylsulfinyl und n-Butylsulfinyl, insbesondere aber Methylsulfinyl und Aethylsulfinyl.

Beispiele für Alkylsulfonyl sind Methylsulfonyl, Aethylsulfonyl, n-Propylsulfonyl und n-Butylsulfonyl, insbesondere aber Methylsulfonyl und Aethylsulfonyl.

Unter Halogen in den Definitionen sowie in Halogenalkoxy, -alkylsulfinyl, -alkylsulfonyl, -alkylthio und -alkenyl sind Fluor, Chlor und Brom, vorzugsweise jedoch Fluor und Chlor zu verstehen.

Die Erfindung umfasst ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxyden als Salzbildner sind die Hydroxyde von Lithium, Natrium, Kalium, Magnesium oder Calcium hervorzuheben, insbesondere aber die von Natrium oder Kalium.

Beispiele für zur Salzbildung geeignete Amine sind primäre, sekundäre und tertiäre aliphatische und aromatische Amine wie Methylamin, Aethylamin, Propylamin, i-Propylamin, die vier isomeren Butylamine,

Dimethylamin, Diäthylamin, Diäthanolamin, Dipropylamin, Diisopropylamin, Di-n-butylamin, Pyrrolidin, Piperidin, Morpholin, Trimethylamin, Triäthylamin, Tripropylamin, Chinuclidin, Pyridin, Chinolin und i-Chinolin, insbesondere aber Aethyl-, Propyl-, Diäthyl- oder Triäthylamin, vor allem aber iso-Propylamin und Diäthanolamin.

Beispiele für quaternäre Ammoniumbasen sind im allgemeinen die Kationen von Halogenamminiumsalzen, z. B. das Tetramethylammoniumkation, das Trimethylbenzylammoniumkation, das Triäthylbenzylammoniumkation, das Tetraäthylammoniumkation, das Trimethyläthylammoniumkation, aber auch das Ammoniumkation.

Unter der Verbindungen der Formel I sind diejenigen bevorzugt, in denen entweder

a) Z Sauerstoff bedeutet oder

b) die Reste $R_3$ und $R_4$ zusammen nicht mehr als 4 Kohlenstoffatome enthalten.

Die Gruppe a kann aufgeteilt werden in zwei weitere Untergruppen, die aus Verbindungen bestehen, in denen

aa) m die Zahl eins und

ab) m die Zahl zwei bedeutet.

Aus der Gruppe aa) sind die Verbindungen bevorzugt, in denen der Rest —X—A in der 2- oder 3-Position zum Sulfonylrest steht. Hierunter geniessen eine weitere Bevorzugung die Verbindungen, in denen der Rest —X—A in der 2-Position steht.

Aus der Gruppe ab) bilden die Verbindungen eine bevorzugte Gruppe, in denen die beiden Reste —X—A in 2- und 5-Stellung zur Sulfonylgruppe stehen.

Eine weitere Bevorzugung von Verbindungen der oben aufgelisteten Untergruppen der Gruppen aa) und ab) besteht darin, dass die Reste $R_3$ und $R_4$ zusammen höchstens 4 Kohlenstoffatome enthalten. Es bestehen somit die besonders bevorzugten Gruppen von Verbindungen darin, dass in Verbindungen der Formel I

aab) nur ein Rest —X—A in der 2-Stellung zum Sulfonylrest vorhanden ist, Z Sauerstoff bedeutet und die Reste $R_3$ und $R_4$ zusammen nicht mehr als 4 Kohlenstoffatome enthalten, und

abb) zwei Reste —X—A in 2- und 5-Stellung zum Sulfonylrest vorhanden sind, Z Sauerstoff bedeutet und die Reste $R_3$ und $R_4$ zusammen nicht mehr als 4 Kohlenstoffatome enthalten.

Aus der Gruppe aab) sind die Verbindungen bevorzugt, in denen $R_1$ Wasserstoff bedeutet, und der Rest $R_2$ in der 5- oder 6-Stellung zur Sulfonylgruppe steht.

Von diesen Verbindungen sind wiederum die bevorzugt, in denen $R_2$ für Wasserstoff, Halogen, $C_1$-$C_4$-Alkoxy, Nitro oder $COOR_6$ steht.

Eine weitere Bevorzugung innerhalb der letztgenannten Gruppe geniessen die Verbindungen, in denen $R_2$ Wasserstoff, Fluor, Nitro oder $C_1$-$C_4$-Alkoxy und $R_3$ und $R_4$ jeweils Wasserstoff, $C_1$-$C_4$-Alkyl, $C_1$-$C_4$-Alkoxy, $C_1$-$C_4$-Alkylthio, Halogen oder Alkoxyalkyl bedeuten, wobei $R_3$ und $R_4$ zusammen höchstens 4 Kohlenstoffatome enthalten.

Unter diesen Verbindungen sind wiederum jene bevorzugt, in denen $R_2$ Wasserstoff und $R_3$ und $R_4$ jeweils $C_1$-$C_4$-Alkyl, $C_1$-$C_4$-Alkoxy, Methylthio, Halogen oder Alkoxyalkyl bedeuten.

Eine weitere Bevorzugung innerhalb der letztgenannten Gruppe geniessen die Verbindungen, in denen X für Sauerstoff oder Schwefel steht.

Ganz besonders bevorzugt sind hiervon wiederum jene, in denen A für $C_2$-$C_8$-Alkoxyalkyl, $C_2$-$C_4$-Halogenalkenyl oder $C_2$-$C_6$-Alkenyl steht.

Drei weiterhin aus der letzten Gruppe bevorzugte Untergruppen sind Gruppen von Verbindungen, in den

α) A für $C_2$-$C_8$-Alkoxyalkyl und $R_3$ und $R_4$ jeweils für Methyl, Aethyl, Chlor oder Methoxy,

β) A für $C_2$-$C_6$-Alkenyl und $R_3$ und $R_4$ jeweils für Methyl, Aethyl, Chlor oder Methoxy und

γ) A für die durch ein bis drei Halogenatome substituiertes Vinyl und $R_3$ und $R_4$ jeweils für Methyl, Aethyl, Chlor oder Methoxy stehen.

Von den Verbindungen der Formel I, in denen Z für Schwefel steht, sind diejenigen bevorzugt, in denen X Sauerstoff, $R_3$ und $R_4$ jeweils unabhängig voneinander $C_1$-$C_3$-Alkyl, $C_1$-$C_3$-Alkoxy oder $C_1$-$C_3$-Alkylthio mit zusammen höchstens 4 Kohlenstoffatomen bedeuten und A für $—CH_2—CH=CH_2$, $—CH_2—CH=CH—CH_3$, $—CH_2—CH(CH_3)=CH_2$, Methoxyäthyl, Methoxymethyl oder $—CCl=CHCl$ steht und der Rest —X—A die 2-Stellung besetzt und m die Zahl 1 bedeutet.

Als bevorzugte Einzelverbindung ist zu nennen :

N-(2-Allyloxyphenylsulfonyl)-N'-(4-methoxy-6-methyl-1,3,5-triazin-2-yl)-harnstoff.

Die Herstellung der Verbindungen der Formel I erfolgt in einem inerten, organischen Lösungsmittel.

Nach einem ersten Verfahren werden die Verbindungen der Formel I erhalten, indem man ein Phenylsulfonamid der Formel II

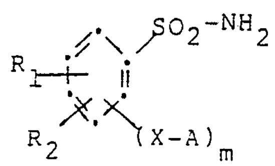

(II)

worin A, $R_1$, $R_2$, X und m die unter Formel I gegebene Bedeutung haben, in Gegenwart einer Base mit einem N-Pyrimidinyl- oder -Triazinylcarbamat der Formel III

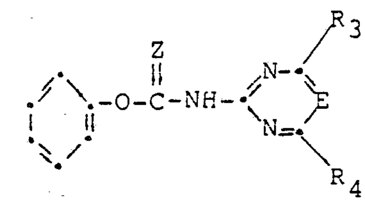

(III)

worin E, $R_3$, $R_4$ und Z die unter Formel I gegebene Bedeutung haben, umsetzt.

Nach einem zweiten Verfahren gelangt man zu Verbindungen der Formel I, indem man ein Phenylsulfonylisocyanat oder -isothiocyanat der Formel IV

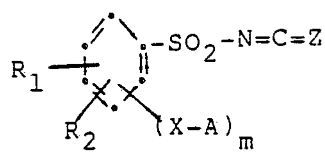

(IV)

worin A, $R_1$, $R_2$, m, X und Z die unter Formel I gegebene Bedeutung haben, gegebenenfalls in Gegenwart einer Base, mit einem Amin der Formel V

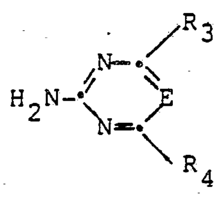

(V)

worin E, $R_3$ und $R_4$ die unter Formel I gegebene Bedeutung haben, umsetzt.

Nach einem weiteren Verfahren werden die Verbindungen der Formel I hergestellt, indem man ein Sulfonamid der oben angegebenen Formel II gegebenenfalls in Gegenwart einer Base mit einem Isocyanat oder Isothiocyanat der Formel VI

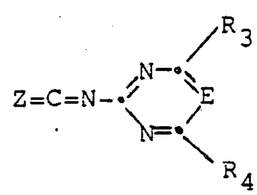

(VI)

worin E, $R_3$, $R_4$ und Z die unter Formel I gegebenen Bedeutung haben, umsetzt.

Schliesslich kann man die Verbindungen der Formel I auch erhalten, indem man ein N-Phenylsulfonylcarbamat der Formel VII

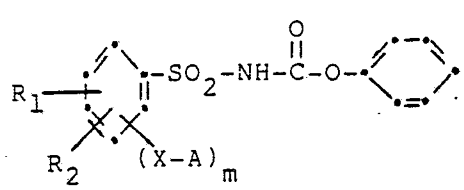

(VII)

worin A, $R_1$, $R_2$, m und X die unter Formel I gegebene Bedeutung haben, mit einem Amin der oben angegebenen Formel V umsetzt.

Die erhaltenen Harnstoffe der Formel I können gewünschtenfalls mittels Aminen, Alkalimetall- oder Erdalkalimetallhydroxiden oder quaternären Ammoniumbasen in Salze übergeführt werden. Dieses geschieht beispielsweise durch Umsetzen mit der äquimolaren Menge Base und Verdampfen des Lösungsmittels.

Die Ausgangsstoffe der Formeln II, IV und VII sind zum Teil neu und können nach folgenden Methoden hergestellt werden :

Die neuen und als Zwischenprodukte verwendeten Sulfonamide der Formel II werden aus den entsprechenden Anilinen durch Diazotierung und Austausch der Diazogruppe mit Schwefeldioxid in

Gegenwart eines Katalysators wie Kupfer-I-chlorid in Salzsäure oder Essigsäure und Umsetzen des entstandenen Phenylsulfonylchlorids mit Ammoniumhydroxid-Lösung erhalten.

Die Verbindungen der Formel II können auch durch Sauerstoff- oder Schwefel-Alkylierung resp. Alkelnylierung von Hydroxy- oder Mercaptophenylsulfonamiden mit den entsprechenden Halogeniden resp. Schwefelsäureestern oder durch Umsatz von ortho-Halogenphenylsulfonamiden mit Metall-Alkoholaten resp. Mercaptiden und gegebenenfalls durch deren Oxidation z. B. mit Perjodaten resp. Persäuren zu den entsprechenden Sulfoxiden und Sulfonen, erhalten werden.

Substituierte ortho-Hydroxyphenyl- resp. ortho-substituierte Hydroxy- oder Mercaptophenylsulfonamide der Formel VIII,

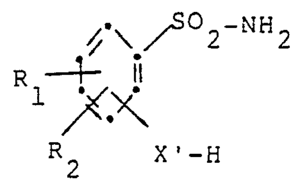

(VIII)

worin $R_1$ und $R_2$ die unter Formel I angegebene Bedeutung haben und X' für Sauerstoff oder Schwefel steht, als Ausgangsprodukte bestimmter Sulfonamid-Vertreter der Formel II sind mit Ausnahme von ortho-Hydroxyphenylsulfonamid neu. Sie können durch Aetherspaltung der entsprechenden $C_1$-$C_4$-Alkoxyphenylsulfonamiden z. B. mit Bortrihalogeniden (solche Umsetzungen sind in der US-Patentschrift 3,904,680 und in J. Am. Chem. Soc. *64*, 1128 (1942) beschrieben) oder durch Hydrogenolyse der entsprechenden Benzyloxyphenylsulfonamiden erhalten werden, wie in J. Chem. Soc. *1958*, 2903 beschrieben ist.

Die Alkoxyphenylsulfonamide wiederum können aus den entsprechenden Alkoxyaniliden wie bereits erwähnt oder durch Chlorsulfonylierung von Alkoxybenzolen und Umsatz der erhaltenen Phenylsulfonylchloriden mit Ammoniumhydroxid-Lösung gewonnen werden. Solche Reaktionen sind aus J. Am. Chem. Soc. *62*, 603 (1940) bekannt geworden.

Die als Zwischenprodukte verwendeten Verbindungen der Formeln II und VIII sind neu resp. z. T. neu und speziell für die Synthese von Verbindungen der Formel I entwickelt worden. Diese Zwischenprodukte bilden einen weiteren Aspekt der vorliegenden Erfindung.

Die Phenylsulfonylisocyanate der Formel IV können durch Umsetzungen der Sulfonamide der Formel II mit Phosgen in Anwesenheit von Butylisocyanat in einem chlorierten Kohlenwasserstoff als Lösungsmittel, bei Rückflusstemperatur erhalten werden. Aehnliche Darstellungen sind in « Newer Methods of Preparative Organic Chemistry » Band VI, 223-241, Academic Press New York und London, beschrieben.

Die Isothiocyanate der Formel IV werden durch Behandlung der Sulfonamide der Formel II mit Schwefelkohlenstoff und Kaliumhydroxid und anschliessender Umsetzung des Dikaliumsalzes mit Phosgen erhalten. Solche Verfahren sind in Arch. Pharm. *299*, 174 (1966) beschrieben.

Die N-Phenylsulfonylcarbamate der Formel VII werden durch Umsetzung der Sulfonamide der Formel II mit Diphenylcarbamat in Gegenwart einer Base erhalten. Aehnliche Verfahren sind in der japanischen Patentschrift 61 169 erwähnt.

Die Ausgangsmaterialien der Formeln III, V und VI sind bekannt oder können nach bekannten Methoden hergestellt werden.

Durch Umsetzung von Aminen der Formel V mit Oxalylchlorid in chlorierten Kohlenwasserstoffen als Lösungsmittel, lassen sich Isocyanate der Formel VI herstellen. Amine der Formel V sind bekannt und zum Teil im Handel erhältlich oder sie können nach bekannten Methoden hergestellt werden, siehe « The Chemistry of Heterocyclic Compounds » Band XIV, Interscience Publishers, New York, London.

Die Umsetzungen zu Verbindungen der Formel I werden vorteilhafterweise in aprotischen, inerten, organischen Lösungsmitteln vorgenommen wie Methylenchlorid, Tetrahydrofuran, Acetonitril, Dioxan, Toluol.

Die Reaktionstemperaturen liegen vorzugsweise zwischen $-20°$ und $+120$ °C. Die Umsetzungen verlaufen im allgemeinen leicht exotherm und können bei Raumtemperetur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base als Reaktionskatalysator beschleunigt werden.

Die Endprodukte können durch Einengen und/oder Verdampfen des Lösungsmittels isoliert und durch Umkristallisieren oder Zerreiben des festen Rückstandes in Lösungsmitteln in denen sie sich nicht gut lösen, wie Aether, aromatischen Kohlenwasserstoffen oder chlorierten Kohlenwasserstoffen gereinigt werden.

Die Wirkstoffe der Formel I sind stabile Verbindungen. Ihre Handhabung bedarf keiner vorsoglicher Massnahmen.

Die Verbindungen der Formel I haben starke pflanzenwuchsregulierende, insbesondere pflanzenwuchshemmende, Eigenschaften. Es werden sowohl Monokotyledonen als auch Dikotyledonen in ihrem Wachstum beeinträchtigt.

So können z. B. die in der Landwirtschaft in tropischen Gegenden häufig als « cover crops » (Bodenbedecker) angepflanzten Leguminosen durch die Verbindungen der Formel I in ihrem Wachstum selektiv gehemmt werden, so dass zwar die Bodenerosion zwischen den Kulturpflanzen verhindert wird, die « cover crops » jedoch nicht zur Konkurrenz für die Kultur werden können.

Weiter eignen sich die Verbindungen der Formel I um das Keimen von eingelagerten Kartoffeln zu verhindern. Bei Kartoffeln entwickeln sich bei der Einlagerung über den Winter häufig Keime, die Schrumpfen, Gewichtsverlust und Faulen zur Folge haben.

Bei grösseren Aufwandmengen werden alle getesteten Pflanzen in ihrer Entwicklung so geschädigt, dass sie absterben.

Bei geringeren Aufwendmengen zeichnen sich die Verbindungen der Formel I durch gute selektivwuchshemmende und selektiv-herbizide Eigenschaften aus, die sie ausgezeichnet zum Einsatz in Kulturen von Nutzpflanzen, insbesondere in Getreide, Baumwolle, Soja, Mais und Reis, befähigen. Es werden dabei teilweise auch Unkräuter geschädigt, welchen bisher nur mit Totalherbiziden beizukommen war.

Die Wirkungsart dieser Wirkstoffe ist unüblich. Sie sind in vielen Fällen translozierbar, d. h. sie werden von der Pflanze aufgenommen und an andere Stellen transportiert, wo sie dann zur Wirkung kommen. Das unübliche daran ist, dass die Wirkstoffe nicht nur den Weg durch die Gefässbündel im Holzteil von den Wurzeln in die Blätter nehmen, sondern auch durch die Siebröhren im Bastteil von den Blättern zurück in die Wurzel transloziert werden können. So gelingt es beispielsweise durch Oberflächenbehandlung perenierende Unkräuter bis in die Wurzeln zu schädigen. Die neuen Verbindungen der Formel I wirken bereits bei — im Vergleich zu anderen Herbiziden und Wuchsregulatoren — sehr geringen Aufwandmengen.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d. h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen : Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dicotylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl ; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder Hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstande verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenefalls substituierte Ammoniumsalze von höheren Fettsäuren ($C_{10}$-$C_{22}$), wie z. B. die Na- oder K-Salze der Oel- oder Stearinsäure, oder von natürlichen Fettsäuregemischen, die z. B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazol-derivate oder Alkylarylsulfonate.

Die Fettsulfonate oder -sulfate liegen in der Regel als Alkali-, Erdalkali- oder gegebenenfalls

substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfsonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazol-derivate enthalten vorzugswiese 2 Sulfonsäuregruppen und einen Fettsäurerest mit 8-22 C-Atomen. Alkylarylsulfonate sind z. B. die Na-, Ca- oder Triäthanolaminsalze der Dodecylbenzosulfonsäure, der Dibutylnaphthalinsulfonsäure oder eines Napthalinsulfonsäure-Formaldehydkondensationsproduktes.

Ferner kommen auch entsprechende Phosphate wie z. B. Salze des Phosphorsäureesters eines p-Nonylphenol-(4-14)-Aethylenoxid-Adduktes in Frage.

Als nicht ionische Tenside kommen in erster Linie Polyglykoläther-derivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthanol, Polyäthylenglykol und Octylphenoxypolyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z. B. das Stearyltrimethylammoniumchlorid oder das Benzyldi(2-chloräthyl)äthylammoniumbromid.

Die in der Formulierungstechnik gebräuchlichen Tenside sind u. a. in folgenden Publikationen beschrieben :

« Mc Cutcheon's Detergents and Emulsifiers Annual » MC Publishing Corp., Ringwood, New Jersey, 1979. Sisely and Wood, « Encyclopedia of Surface Active Agents », Chemical Publishing Co., Inc. New York, 1964.

Die pestiziden Zubereitungen enthalten in der Regel 0,1 bis 95 %, insbesondere 1 bis 80 %, Wirkstoff der Formel I, 1 bis 99 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen : (% = Gewichtsprozent).

Lösungen
Aktiver Wirkstoff : 1 bis 30 %, vorzugsweise 5 bis 20 %
Lösungsmittel : 99 bis 0 %, vorzugsweise 95 bis 0 %
oberflächenaktives Mittel : 0 bis 99 %, vorzugsweise 0 bis 95 %.

Emulgierbare Konzentrate
Aktiver Wirkstoff : 1 bis 20 %, bevorzugt 5 bis 10 %
oberflächenaktives Mittel : 5 bis 30 %, vorzugsweise 10 bis 20 %
flüssiges Trägermittel : 50 bis 94 %, vorzugsweise 70 bis 85 %.

Stäube
Aktiver Wirkstoff : 0,1 bis 10 %, vorzugsweise 0,1 bis 1 %
festes Trägermittel : 99,9 bis 90 %, vorzugsweise 99,9 bis 99 %.

Suspension-Konzentrate
Aktiver Wirkstoff : 5 bis 75 %, vorzugsweise 10 bis 50 %
Wasser : 94 bis 25 %, vorzugsweise 90 bis 30 %
oberflächenaktives Mittel : 1 bis 40 %, vorzugsweise 2 bis 30 %.

Benetzbare Pulver
Aktiver Wirkstoff : 0,5 bis 90 %, vorzugsweise 1 bis 80 %
oberflächenaktives Mittel : 0,5 bis 20 %, vorzugsweise 1 bis 15 %
festes Trägermittel : 5 bis 95 %, vorzugsweise 15 bis 90 %.

Granulate
Aktiver Wirkstoff : 0,5 bis 30 %, vorzugsweise 3 bis 15 %
festes Trägermittel : 99,5 bis 70 %, vorzugsweise 97 bis 85 %.

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die aufwandmengen betragen in der Regel 0,01 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder ander Wirkstoffe zur Erzielung spezieller Effekte enthalten.

In den folgenden Beispielen sind die Temperaturen in Celsiusgraden °C, die Drücke in Millibar mb angegeben.

## Herstellungsbeispiele :

### Beispiel 1 : 2-Hydroxyphenylsulfonamid

Zu einer Suspension von 39 g 2-Methoxyphenylsulfonamid in 210 ml trockenem Methylenchlorid lässt man unter Stickstoffatmosphäre bei Raumtemperatur innerhalb von 15 Minuten 22,2 ml Bortribromid zutropfen. Die Reaktionsmischung wird danach für 1 Stunde bei Raumtemperatur gerührt. Anschliessend werden zur auf 0 °C gekühlten Mischung innerhalb von 15 Minuten 200 ml Methanol zugesetzt und die klare Lösung eingedampft. Der ölige Rückstand wird in Aethylacetat aufgenommen, zweimal mit Wasser gewaschen, mit Natriumsulfat getrocknet und eingedampft. Durch Verreiben des Rückstandes mit Petroläther erhält man 25,1 g 2-Hydroxyphenylsulfonamid vom Schmelzpunkt 136-138 °C.

In analoger Weise werden in in der folgenden Tabelle aufgelisteten Hydroxyphenylsulfonamide der Formel VIII erhalten.

Tabelle 1

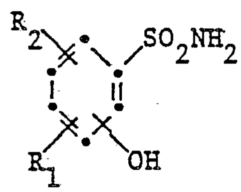

| $R_1$ | $R_2$ | Stellung von -OH | physikal. Daten |
|---|---|---|---|
| 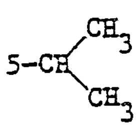 | H | 2 | Smp. 90-91° |
| 2-$CH_3$ | H | 5 | |
| 2-Cl | H | 5 | |
| 5-F | H | 2 | Smp. 141-142° |
| 5-Br | H | 2 | Smp. 169-70° |
| 5-$NO_2$ | H | 2 | Smp. 180-185° |
| 3-$CH_3$ | H | 2 | Smp. 133-134° |
| 2-$CH_3$ | H | 3 | |
| 3-Cl | H | 2 | |
| 6-$CH_3$ | H- | 2 | |
| 3-$OCH_3$ | H | 2 | |
| 2-$OCH_3$ | H | 3 | |
| 3-$CH_3$ | 5-t-$C_4H_9$ | 2 | Smp. 134-137° |

Tabelle 1 (Fortsetzung)

| $R_1$ | $R_2$ | Stellung von -OH | physikal. Daten |
|---|---|---|---|
| 6-$OCH_3$ | H | 2 | |
| 6-OH | H | 2 | |
| 5-$OCH_3$ | H | 2 | Smp. 119-20° |
| 2-$OCH_3$ | H | 5 | Smp. 213-15° |
| 5-$CH_3$ | H | 2 | Smp. 155-156° |
| 6-Cl | H | 2 | Smp. 190-91° |
| 3-$NO_2$ | H | 2 | |
| 3-$COOCH_3$ | H | 2 | |
| 5-$COOCH_3$ | H | 2 | Smp. 171-172° |
| 5-Cl | H | 2 | Smp. 176-179° |
| 5-$NO_2$ | 3-$CF_3$ | 2 | |
| 5-$NO_2$ | 3-Cl | 2 | |
| 5-$CF_3$ | 3-$NO_2$ | 2 | |
| 5-$CH_3$ | 3-$CH_3$ | 2 | |
| 5-Cl | 3-$NO_2$ | 2 | |
| 5-Cl | 3-Cl | 2 | Smp. 156-158° |
| 5-Br | 3-$OCH_3$ | 2 | |
| 5-Br | 2-$OCH_3$ | 3 | Smp. 194-197° |
| 3-$OCH_3$ | 5-$COOCH_3$ | 2 | |
| 2-$OCH_3$ | 5-$COOCH_3$ | 3 | |
| 5-$CH_3$ | 3-Br | 2 | |
| 5-Br | 3-$NO_2$ | 2 | |
| 5-Br | 3-$COOCH_3$ | 2 | |

Tabelle 1 (Fortsetzung)

| $R_1$ | $R_2$ | Stellung von -OH | physikal. Daten |
|---|---|---|---|
| 5-$COOCH_3$ | 3-$NO_2$ | 2 | |
| 5-Cl | 3-Br | 2 | Smp. 247-249° |
| 5-$COOC_3H_7$(i) | 3-$NO_2$ | 2 | |
| 5-$CON(CH_3)_2$ | H | 2 | |
| 6-$NO_2$ | H | 2 | |
| 6-$COOCH_3$ | H | 2 | |

Beispiel 2 : 2-Allyloxyphenylsulfonamid

Eine Mischung von 3,5 g 2-Hydroxyphenylsulfonamid, 5,5 g Pottasche und 1,7 ml Allylbromid in 100 ml Methyläthylketon wird unter Stickstoffatmosphäre bei Rückflusstemperatur während 1 Stunde gerührt. Das Reaktionsgemisch wird auf Raumtemperatur abgekühlt, filtriert und eingedampft. Man erhält nach einmaligem Umkristallisieren aus Aethylacetat 3,27 g 2-Allyloxyphenylsulfonamid vom Schmelzpunkt 104-105 °C.

Analog herzustellende Sulfonamide der Formel II sind in der anschliessenden Tabelle aufgelistet.

Tabelle 2

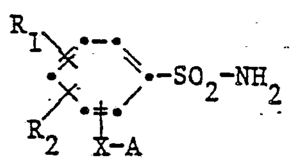

| $R_1$ | $R_2$ | X | A | Stellung von -X-A | phys. Daten |
|---|---|---|---|---|---|
| H | H | O | -CCl=CHCl | 2 | Smp. 147-149° |
| H | H | S | -CCl=CHCl | 2 | |
| H | H | SO | -CCl=CHCl | 2 | |
| H | H | $SO_2$ | -CCl=CHCl | 2 | |
| H | H | O | -$CH=CCl_2$ | 2 | |
| H | H | S | -$CH=CCl_2$ | 2 | |
| H | H | SO | -$CH=CCl_2$ | 2 | |
| H | H | $SO_2$ | -$CH=CCl_2$ | 2 | |
| H | H | O | -$CH_2CH=CH_2$ | 2 | Smp. 104-5° |

Tabelle 2 (Fortsetzung)

| $R_1$ | $R_2$ | X | A | Stellung von-X-A | phys. Daten |
|---|---|---|---|---|---|
| H | H | O | 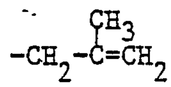 | 2 | Smp. 104-105° |
| H | H | O | $-(CH_2)_2-CH=CH_2$ | 2 | |
| H | H | O | $-(CH_2)_3-CH=CH_2$ | 2 | |
| H | H | O | 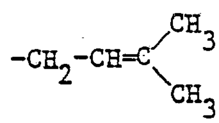 | 2 | |
| H | H | S | 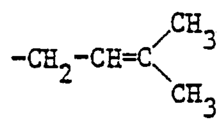 | 2 | |
| H | H | O | $-CH_2-CH=CH-CH_3$ | 2 | Smp. 113-114° |
| H | H | O | 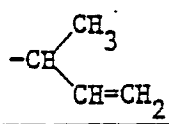 | 2 | |
| H | H | O | $-CH_2-OCH_3$ | 2 | Zers. ab 121° |
| H | H | O | $-CH_2-OC_2H_5$ | 2 | |
| H | H | O | $-CH_2-S-CH_3$ | 2 | |
| H | H | O | $-CH_2CH_2-OCH_3$ | 2 | Smp. 110-112° |
| H | H | S | $-CH_2CH_2-OCH_3$ | 2 | |
| H | H | O | $-CH_2CH_2-OC_2H_5$ | 2 | |
| H | H | O | $-CH_2CH_2-SCH_3$ | 2 | |
| 6-Cl | H | O | $-CH_2CH=CH_2$ | 2 | Smp. 127-128° |
| 6-Cl | H | O | 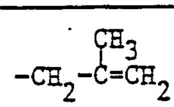 | 2 | |
| 6-Cl | H | O | 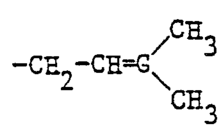 | 2 | |
| 6-Cl | H | O | $-CH_2OCH_3$ | 2 | |
| 6-Cl | H | O | $-(CH_2)_2OCH_3$ | 2 | |
| 6-Cl | H | O | $-CH_2SCH_3$ | 2 | |

Tabelle 2 (Fortsetzung)

| $R_1$ | $R_2$ | X | A | Stellung von -X-A | phys. Daten |
|---|---|---|---|---|---|
| 6-Cl | H | O | $-(CH_2)_2SCH_3$ | 2 | |
| 6-Cl | H | O | $-CH_2-CH=CH-CH_3$ | 2 | |
| $6-OCH_3$ | H | O | $-CH_2-CH=CH_2$ | 2 | |
| $6-OCH_3$ | H | O | 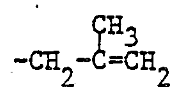 | 2 | |
| $6-OCH_3$ | H | O | $-(CH_2)_2-OC_2H_5$ | 2 | |
| $6-OCH_3$ | H | O | 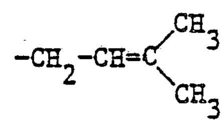 | 2 | |
| $6-OCH_3$ | H | O | $-(CH_2)_2-CH=CH_2$ | 2 | |
| $6-CH_3$ | H | O | $-CH_2-CH=CH_2$ | 2 | |
| $6-CH_3$ | H | O | 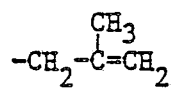 | 2 | |
| $6-CH_3$ | H | O | $-(CH_2)_2-OCH_3$ | 2 | |
| $6-CH_3$ | H | O | $-CH_2SCH_3$ | 2 | |
| H | H | O | $-CH_2CH_2-OCH_2CH=CH_2$ | 2 | |
| $6-NO_2$ | H | O | $-CH_2-CH=CH_2$ | 2 | |
| $6-NO_2$ | H | O | $-CH_2-CH=CH-CH_3$ | 2 | |
| $6-NO_2$ | H | O | $-CH_2-OCH_3$ | 2 | |
| $6-NO_2$ | H | O | $-CH_2-CH_2-OC_2H_5$ | 2 | |
| $6-NO_2$ | H | O | 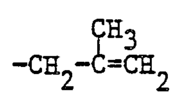 | 2 | |
| $6-COOCH_3$ | H | O | $-CH_2-CH=CH_2$ | 2 | |
| 5-Cl | H | O | $-CH_2-CH=CH_2$ | 2 | Smp. 122° |
| 5-Cl | H | O | 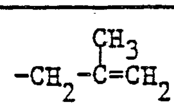 | 2 | |
| 5-Br | H | O | 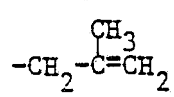 | 2 | |

Tabelle 2 (Fortsetzung)

| $R_1$ | $R_2$ | X | A | Stellung von-X-A | phys. Daten |
|---|---|---|---|---|---|
| 5-Br | H | O | $-CH_2-CH=CH_2$ | 2 | Smp. 110-111° |
| 5-Br | H | O | $-CH_2-OCH_3$ | 2 | |
| 5-F | H | O | $-CH_2-CH=CH_2$ | 2 | Smp. 113-114° |
| 5-F | H | O | 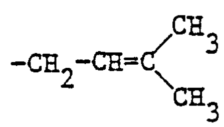 | 2 | |
| 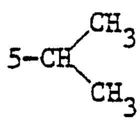 | H | O | 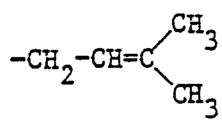 | 2 | |
| 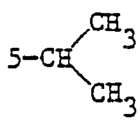 | H | O | $-CH_2-CH=CH_2$ | 2 | Oel |
| 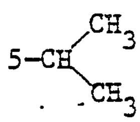 | H | O | $-CH_2CH_2OCH_3$ | 2 | |
| 5-$CH_3$ | H | O | $-CH_2CH_2OCH_3$ | 2 | |
| 5-$CH_3$ | H | O | $-CH_2-CH=CH_2$ | 2 | Smp. 139-140° |
| 5-$CH_3$ | H | O | $-CH_2-CH=CH-CH_3$ | 2 | |
| 5-$NO_2$ | H | O | $-CH_2-CH=CH-CH_3$ | 2 | |
| 5-$NO_2$ | H | O | $-CH_2-S-CH_3$ | 2 | |
| 5-$NO_2$ | H | O | $-CH_2CH=CH_2$ | 2 | Smp. 138-141° |
| 5-$OCH_3$ | H | O | $-CH_2CH=CH_2$ | 2 | Smp. 88-89° |
| 5-$OCH_3$ | H | O | 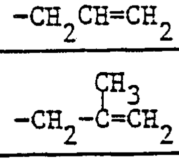 | 2 | |
| 5-$OCH_3$ | H | O | $-CH_2CH=CH-CH_3$ | 2 | |
| 5-$OCH_3$ | H | O | $-CH_2OCH_3$ | 2 | |
| 5-$OCH_3$ | H | O | $-CH_2CH_2-SCH_3$ | 2 | |

Tabelle 2 (Fortsetzung)

| $R_1$ | $R_2$ | X | A | Stellung von-X-A | phys. Daten |
|---|---|---|---|---|---|
| 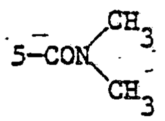 | H | O | $-CH_2-CH=CH_2$ | 2 | |
| 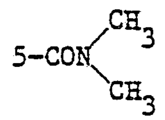 | H | O | 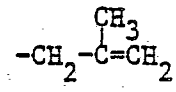 | 2 | |
| $5-COOCH_3$ | H | O | $-CH_2-CH=CH_2$ | 2 | |
| $5-COOCH_3$ | H | O | 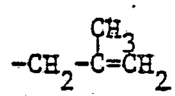 | 2 | |
| $5-COOCH_3$ | H | O | $-CH_2-OC_2H_5$ | 2 | |
| $5-NO_2$ | $3-CF_3$ | O | $-CH_2-CH=CH_2$ | 2 | |
| $5-NO_2$ | 3-Cl | O | $-CH_2-CH=CH_2$ | 2 | |
| $5-NO_2$ | 3-Cl | O | 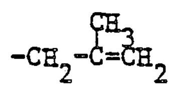 | 2 | |
| $5-CF_3$ | $3-NO_2$ | O | $-CH_2-CH=CH_2$ | 2 | |
| $5-CH_3$ | $3-CH_3$ | O | $-CH_2-CH=CH_2$ | 2 | |
| $5-CH_3$ | $3-CH_3$ | O | $-CH_2-CH=CH-CH_3$ | 2 | |
| 5-Cl | $3-NO_2$ | O | $-CH_2-CH=CH-CH_3$ | | |
| 5-Cl | 3-Cl | O | $-CH_2-CH=CH_2$ | 2 | Smp. 161-162° |
| 5-Cl | 3-Cl | O | $-CH_2-CH_2-OCH_3$ | 2 | |
| 5-Cl | 3-Cl | O | $-CH_2-OCH_3$ | 2 | |
| 5-Br | $3-OCH_3$ | O | $-CH_2-CH=CH_2$ | 2 | |
| 5-Br | $2-OCH_3$ | O | $-CH_2-CH=CH_2$ | 3 | Harz |
| $3-OCH_3$ | $5-COOCH_3$ | O | $-CH_2-CH=CH_2$ | 2 | |
| $2-OCH_3$ | $5-COOCH_3$ | O | 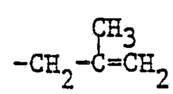 | 3 | |

Tabelle 2 (Fortsetzung)

| $R_1$ | $R_2$ | X | A | Stellung von -X-A | phys. Daten |
|---|---|---|---|---|---|
| $5-CH_3$ | 3-Br | O | $-CH_2-OCH_3$ | 2 | |
| 5-Br | $3-NO_2$ | O | $-CH_2CH=CH_2$ | 2 | |
| 5-Cl | 3-Br | O | $-CH_2CH=CH_2$ | 2 | Smp. 179° |
| 5-Cl | 3-Br | O | $-CH_2-\overset{CH_3}{\overset{\vert}{C}}=CH_2$ | 2 | |
| 5-Cl | 3-Br | O | $-CH_2-CH_2-OCH_3$ | 3 | |
| H | H | O | $-CH_2-SO-CH_3$ | 2 | |
| H | H | O | $-CH_2-SO_2-CH_3$ | 2 | |
| H | H | O | $-CH_2-CH_2-SO-CH_3$ | 2 | |
| H | H | O | $-CH_2-CH_2-SO_2-CH_3$ | 2 | |
| H | H | O | $-CH_2-\overset{Cl}{\overset{\vert}{C}}=CH_2$ | 2 | |
| H | H | O | $-CH_2-\overset{Cl}{\overset{\vert}{C}}=CH-Cl$ | 2 | |
| H | H | O | $-CH_2-CH=\overset{Cl}{\overset{\vert}{C}}-CH_3$ | 2 | |
| H | H | O | $-CH_2-CH=C\begin{smallmatrix}\diagup Cl\\ \diagdown Cl\end{smallmatrix}$ | 2 | |
| $5-CH_3$ | H | O | $-CH_2-\overset{Cl}{\overset{\vert}{C}}=CH_2$ | 2 | |
| $5-NO_2$ | H | O | $-CH_2-\overset{Cl}{\overset{\vert}{C}}=CH-Cl$ | 2 | |
| 5-Br | H | O | $-CH_2-\overset{Cl}{\overset{\vert}{C}}-CH_2$ | 2 | |
| 5-Cl | H | O | $-CH_2-CH=C\begin{smallmatrix}\diagup Cl\\ \diagdown Cl\end{smallmatrix}$ | 2 | |
| $5-CH(CH_3)_2$ | H | O | $-CH_2-CH=\overset{Cl}{\overset{\vert}{C}}-CH_3$ | 2 | |

Tabelle 2 (Fortsetzung)

| $R_1$ | $R_2$ | X | A | Stellung von-X-A | phys. Daten |
|---|---|---|---|---|---|
| 3-$CH_3$ | H | O | 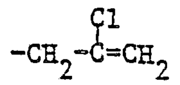 | 2 | |
| 6-$CH_3$ | H | O | 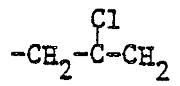 | 2 | |
| 6-$OCH_3$ | H | O | 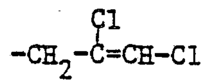 | 2 | |
| 5-$OCH_3$ | H | O | 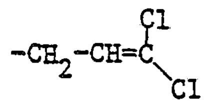 | 2 | |
| 6-Cl | H | O | 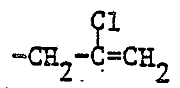 | 2 | |
| 3-$NO_2$ | H | O | 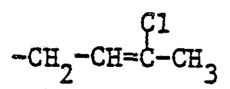 | 2 | |
| 3-$CH_3$ | H | O | $-CH_2-CH=CH_2$ | 2 | Smp. 114-116° |
| 3-$NO_2$ | H | O | $-CH_2-CH=CH_2$ | 2 | |
| 3-Cl | H | O | $-CH_2-CH=CH_2$ | 2 | |
| 3-Cl | H | O | $-CH_2-CH=CH-CH_3$ | 2 | |
| 3-Cl | H | O | 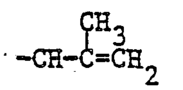 | 2 | |
| 3-$OCH_3$ | H | O | 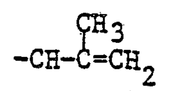 | 2 | |
| 3-$OCH_3$ | H | O | $-CH_2-CH=CH_2$ | 2 | |
| 2-$OCH_3$ | H | O | $-CH_2-CH=CH_2$ | 5 | Smp. 113-114° |

Beispiel 3

a) N-(2-Allyloxyphenylsulfonyl)-phenyl-carbamat.

Zur Suspension von 0,56 g Natriumhydrid (55 %) in 5 ml absolutem Dimethylformamid lässt man bei höchstens 20 °C unter einer Stickstoffatmosphäre innerhalb von 5 Minuten 2,76 g 2-Allyloxyphenylsulfonamid in 20 ml Dimethylformamid zutropfen und rührt vor dem tropfenweisen Zusatz von 2,91 g Diphenylcarbamat in 20 ml Dimethylformamid die Suspension für ca. 10 Minuten. Nach weiterem Rühren für 0,5 Stunden wird das Reaktionsgemisch mit einer Mischung von 80 ml Aethylacetat, 80 g Eis und 12,3 ml 2N Salzsäure aufgenommen. Die organische Phase wird zweimal mit Eiswasser gewaschen, über Natriumsulfat getrocknet und eingedampft. Kristallisation aus Aether/Petroläther (1 : 1) ergibt 3,5 g N-(2-Allyloxyphenylsulfonyl)-phenyl-carbamat, Smp. 140-141 °C.

b) N-(2-Allyloxyphenylsulfonyl)-N'-(4-methoxy-6-methyl-1,3,5-triazin-2-yl)-harnstoff

Eine Mischung von 3,33 g N-(2-Allyloxyphenylsulfonyl)-phenyl-carbamat und 1,4 g 2-Amino-4-methoxy-6-methyl-1,3,5-triazin in 30 ml absolutem Dioxan wird für 0,5 Stunden zum Rückfluss erhitzt, danach auf 20 °C abgekühlt, filtriert, eingedampft und aus Aether kristallisiert. Durch Umkristallissieren aus Aethylacetat/Petroläther (1 : 1) erhält man 2,0 g N-(2-Allyloxyphenylsulfonyl)-N'-(4-methoxy-6-methyl-1,3,5-triazin-2-yl)-harnstoff, Smp. 146-147 °C.

In analoger Weise werden die in der folgenden Tabelle aufgelisteten Verbindungen der Formel I hergestellt.

Tabelle 3

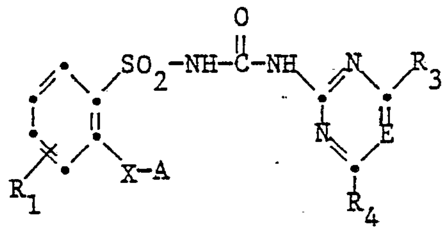

| Nr. | A | $R_1$ | $R_3$ | $R_4$ | X | E |
|---|---|---|---|---|---|---|
| 1 | $-CH_2-CH=CH_2$ | 6-Cl | $CH_3$ | $OCH_3$ | O | N |
| 2 | $-CH_2-CH=CH_2$ | 6-Cl | $CH_3$ | $OCH_3$ | O | CH |
| 3 | $-CH_2-CH=CH_2$ | 6-Cl | $C_2H_5$ | $OCH_3$ | O | N |
| 4 | $-CH_2-CH=CH_2$ | 6-Cl | $OCH_3$ | $OCH_3$ | O | CH |
| 5 | $-CH_2-CH=CH_2$ | 6-Cl | $OCH_3$ | $OCH_3$ | O | N |
| 6 | $CH_3$<br>$-CH_2-C=CH_2$ | 6-Cl | $CH_3$ | $OCH_3$ | O | N |
| 7 | $CH_3$<br>$-CH_2-C=CH_2$ | 6-Cl | $CH_3$ | $OCH_3$ | O | CH |
| 8 | $CH_3$<br>$-CH_2-C=CH_2$ | 6-Cl | $C_2H_5$ | $OCH_3$ | O | N |
| 9 | $CH_3$<br>$-CH_2-C=CH_2$ | 6-Cl | $OCH_3$ | $OCH_3$ | O | N |
| 10 | $CH_3$<br>$-CH_2-C=CH_2$ | 6-Cl | $OCH_3$ | $OCH_3$ | O | CH |
| 11 | $-CH_2-CH=CH-CH_3$ | 6-Cl | $OCH_3$ | $OCH_3$ | O | CH |
| 12 | $-CH_2-CH=CH-CH_3$ | 6-Cl | $OCH_3$ | $OCH_3$ | O | N |
| 13 | $-CH_2-CH=CH-CH_3$ | 6-Cl | $OCH_3$ | $CH_3$ | O | N |
| 14 | $-CH_2-CH=CH-CH_3$ | 6-Cl | $OCH_3$ | $CH_3$ | O | CH |
| 15 | $CH_3$<br>$-CH_2CH=C$<br>$CH_3$ | 6-Cl | $CH_3$ | $OCH_3$ | O | N |

Tabelle 3 (Fortsetzung)

| Nr. | A | $R_1$ | $R_3$ | $R_4$ | X | E |
|---|---|---|---|---|---|---|
| 16 | 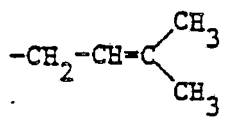 | 6-Cl | $CH_3$ | $OCH_3$ | O | CH |
| 17 | $-CH_2-OCH_3$ | 6-Cl | $CH_3$ | $OCH_3$ | O | CH |
| 18 | $-CH_2-OCH_3$ | 6-Cl | $CH_3$ | $OCH_3$ | O | N |
| 19 | $-CH_2-OCH_3$ | 6-Cl | $C_2H_5$ | $OCH_3$ | O | N |
| 20 | $-(CH_2)_2-OCH_3$ | 6-Cl | $CH_3$ | $OCH_3$ | O | N |
| 21 | $-(CH_2)_2-OCH_3$ | 6-Cl | $CH_3$ | $OCH_3$ | O | CH |
| 22 | $-CH_2-SCH_3$ | 6-Cl | $CH_3$ | $OCH_3$ | O | CH |
| 23 | $-CH_2-SCH_3$ | 6-Cl | $CH_3$ | $OCH_3$ | O | N |
| 24 | $-(CH_2)_2-SCH_3$ | 6-Cl | $CH_3$ | $OCH_3$ | O | N |
| 25 | $-(CH_2)_2-SCH_3$ | 6-Cl | $CH_3$ | $OCH_3$ | O | CH |
| 26 | $-CH_2CH=CH_2$ | 6-$OCH_3$ | $CH_3$ | $OCH_3$ | O | CH |
| 27 | $-CH_2CH=CH_2$ | 6-$OCH_3$ | $CH_3$ | $OCH_3$ | O | N |
| 28 | $-CH_2CH=CH_2$ | 6-$OCH_3$ | $C_2H_5$ | $OCH_3$ | O | N |
| 29 | $-CH_2CH=CH_2$ | 6-$OCH_3$ | $OCH_3$ | $OCH_3$ | O | N |
| 30 | $-CH_2CH=CH_2$ | 6-$OCH_3$ | $OCH_3$ | $OCH_3$ | O | CH |
| 31 | 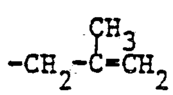 | 6-$OCH_3$ | $OCH_3$ | $OCH_3$ | O | CH |
| 32 | 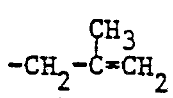 | 6-$OCH_3$ | $CH_3$ | $OCH_3$ | O | N |
| 33 | 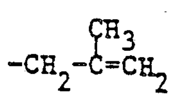 | 6-$OCH_3$ | $CH_3$ | $OCH_3$ | O | CH |
| 34 | $-(CH_2)_2-OC_2H_5$ | 6-$OCH_3$ | $CH_3$ | $OCH_3$ | O | CH |
| 35 | $-(CH_2)_2-OC_2H_5$ | 6-$OCH_3$ | $CH_3$ | $OCH_3$ | O | N |

Tabelle 3 (Fortsetzung)

| Nr. | A | $R_1$ | $R_3$ | $R_4$ | X | E | phys.Daten |
|---|---|---|---|---|---|---|---|
| 36 | 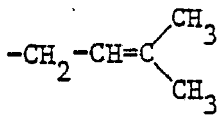 | 6-Cl | $CH_3$ | $OCH_3$ | O | N | |
| 37 | 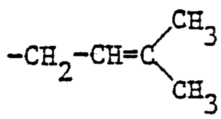 | 6-Cl | $CH_3$ | $OCH_3$ | O | CH | |
| 38 | 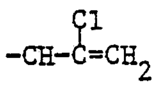 | 6-$OCH_3$ | $CH_3$ | $OCH_3$ | O | N | |
| 39 | 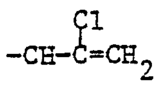 | 6-$OCH_3$ | $CH_3$ | $OCH_3$ | O | CH | |
| 40 | 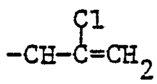 | 6-Cl | $CH_3$ | $OCH_3$ | O | CH | Smp.163-164° |
| 41 | 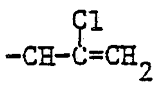 | 6-Cl | $CH_3$ | $OCH_3$ | O | N | Smp.142-145° |
| 42 | $-CH_2-CH=CH_2$ | 6-$CH_3$ | $CH_3$ | $OCH_3$ | O | N | |
| 43 | $-CH_2-CH=CH_2$ | 6-$CH_3$ | $CH_3$ | $OCH_3$ | O | CH | |
| 44 | $-CH_2-CH=CH_2$ | 6-$CH_3$ | $OCH_3$ | $OCH_3$ | O | CH | |
| 45 | $-CH_2-CH=CH_2$ | 6-$CH_3$ | $OCH_3$ | $OCH_3$ | O | N | |
| 46 | 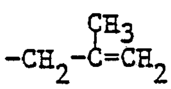 | 6-$CH_3$ | $CH_3$ | $OCH_3$ | O | N | |
| 47 | 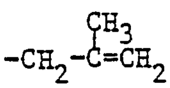 | 6-$CH_3$ | $CH_3$ | $OCH_3$ | O | CH | |
| 48 | 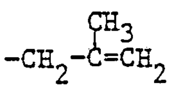 | 6-$CH_3$ | $OCH_3$ | $OCH_3$ | O | N | |
| 49 | 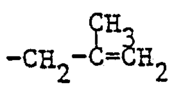 | 6-$CH_3$ | $OCH_3$ | $OCH_3$ | O | CH | |
| 50 | $-CH_2-OCH_3$ | 6-$CH_3$ | $OCH_3$ | $OCH_3$ | O | CH | |
| 51 | $-CH_2-OCH_3$ | 6-$CH_3$ | $OCH_3$ | $CH_3$ | O | CH | |
| 52 | $-CH_2-OCH_3$ | 6-$CH_3$ | $OCH_3$ | $CH_3$ | O | N | |

Tabelle 3 (Fortsetzung)

| Nr. | A | $R_1$ | $R_3$ | $R_4$ | X | E |
|---|---|---|---|---|---|---|
| 53 | $-(CH_2)_2-CH=CH_2$ | $6-OCH_3$ | $OCH_3$ | $CH_3$ | O | N |
| 54 | $-(CH_2)_2-CH=CH_2$ | $6-OCH_3$ | $OCH_3$ | $CH_3$ | O | CH |
| 55 | $-CH_2-SCH_3$ | $6-CH_3$ | $OCH_3$ | $CH_3$ | O | CH |
| 56 | $-CH_2-SCH_3$ | $6-CH_3$ | $OCH_3$ | $CH_3$ | O | N |
| 57 | $-CH_2-CH=CH_2$ | $6-NO_2$ | $CH_3$ | $OCH_3$ | O | N |
| 58 | $-CH_2-CH=CH_2$ | $6-NO_2$ | $CH_3$ | $OCH_3$ | O | CH |
| 59 | $-CH_2-CH=CH_2$ | $6-NO_2$ | $OCH_3$ | $OCH_3$ | O | CH |
| 60 | $-CH_2-CH=CH_2$ | $6-NO_2$ | $OCH_3$ | $OCH_3$ | O | N |
| 61 | $-CH_2-CH=CH-CH_3$ | $6-NO_2$ | $OCH_3$ | $OCH_3$ | O | N |
| 62 | $-CH_2-CH=CH-CH_3$ | $6-NO_2$ | $OCH_3$ | $OCH_3$ | O | CH |
| 63 | $-CH_2-CH=CH-CH_3$ | $6-NO_2$ | $OCH_3$ | $CH_3$ | O | CH |
| 64 | $-CH_2-CH=CH-CH_3$ | $6-NO_2$ | $OCH_3$ | $CH_3$ | O | N |
| 65 | $-CH_2-OCH_3$ | $6-NO_2$ | $OCH_3$ | $CH_3$ | O | N |
| 66 | $-CH_2-OCH_3$ | $6-NO_2$ | $OCH_3$ | $CH_3$ | O | CH |
| 67 | $-CH_2-CH_2-OC_2H_5$ | $6-NO_2$ | $OCH_3$ | $CH_3$ | O | CH |
| 68 | $-CH_2-CH_2-OC_2H_5$ | $6-NO_2$ | $OCH_3$ | $CH_3$ | O | N |
| 69 | $-CH_2-C(CH_3)=CH_2$ | $6-NO_2$ | $OCH_3$ | $CH_3$ | O | N |
| 70 | $-CH_2-C(CH_3)=CH_2$ | $6-NO_2$ | $OCH_3$ | $CH_3$ | O | CH |
| 71 | $-CH_2-CH=CH_2$ | $6-COOCH_3$ | $OCH_3$ | $CH_3$ | O | CH |
| 72 | $-CH_2-CH=CH_2$ | $6-COOCH_3$ | $OCH_3$ | $CH_3$ | O | N |
| 73 | $-CH_2-CH=CH_2$ | $6-COOCH_3$ | $OCH_3$ | $OCH_3$ | O | N |
| 74 | $-CH_2-CH=CH_2$ | $6-COOCH_3$ | $OCH_3$ | $OCH_3$ | O | CH |

Tabelle 3 (Fortsetzung)

| Nr. | A | $R_1$ | $R_3$ | $R_4$ | X | E | phys. Daten |
|---|---|---|---|---|---|---|---|
| 75 | $-CH_2CH=CH_2$ | 5-Cl | $CH_3$ | $OCH_3$ | O | N | |
| 76 | $-CH_2CH=CH_2$ | 5-Cl | $CH_3$ | $OCH_3$ | O | CH | |
| 77 | $-CH_2CH=CH_2$ | 5-Cl | $OCH_3$ | $OCH_3$ | O | N | |
| 78 | $-CH_2CH=CH_2$ | 5-Cl | $OCH_3$ | $OCH_3$ | O | N | |
| 79 | $-CH_2CH=CH_2$ | 5-Cl | $C_2H_5$ | $OCH_3$ | O | N | |
| 80 | $CH_3$<br>$-CH_2-C=CH_2$ | 5-Cl | $CH_3$ | $OCH_3$ | O | N | |
| 81 | $CH_3$<br>$-CH_2-C=CH_2$ | 5-Cl | $CH_3$ | $OCH_3$ | O | CH | |
| 82 | $-CH_2CH=CH_2$ | 5-Br | $CH_3$ | $OCH_3$ | O | CH | |
| 83 | $-CH_2CH=CH_2$ | 5-Br | $CH_3$ | $OCH_3$ | O | N | Smp..190-191° |
| 84 | $-CH_2CH=CH_2$ | 5-Br | $C_2H_5$ | $CH_3$ | O | N | |
| 85 | $CH_3$<br>$-CH-C=CH_2$ | 5-Br | $CH_3$ | $OCH_3$ | O | N | |
| 86 | $CH_3$<br>$-CH-C=CH_2$ | 5-Br | $OCH_3$ | $OCH_3$ | O | N | |
| 87 | $CH_3$<br>$-CH-C=CH_2$ | 5-Br | $CH_3$ | $OCH_3$ | O | CH | |
| 88 | $-CH_2-OCH_3$ | 5-Br | $CH_3$ | $OCH_3$ | O | CH | |
| 89 | $-CH_2-OCH_3$ | 5-Br | $CH_3$ | $OCH_3$ | O | N | |
| 90 | $-CH_2-CH=CH_2$ | 5-F | $CH_3$ | $OCH_3$ | O | N | Smp.159-161° |
| 91 | $-CH_2-CH=CH_2$ | 5-F | $CH_3$ | $OCH_3$ | O | CH | Smp.200-201° |
| 92 | $-CH_2-CH=CH_2$ | 5-F | $OCH_3$ | $C_2H_5$ | O | N | |
| 93 | $-CH_2-CH=CH_2$ | 5-F | $OCH_3$ | $OCH_3$ | O | CH | Smp.162-164° |

Tabelle 3 (Fortsetzung)

| Nr. | A | $R_1$ | $R_3$ | $R_4$ | X | E | phys. Daten |
|---|---|---|---|---|---|---|---|
| 94 | 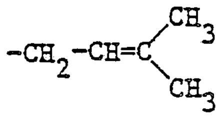 | 5-F | $CH_3$ | $OCH_3$ | O | N | |
| 95 | 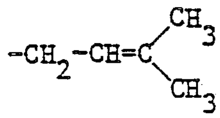 | 5-F | $CH_3$ | $OCH_3$ | O | CH | |
| 96 | 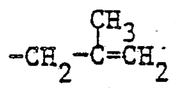 | 5-F | $CH_3$ | $OCH_3$ | O | CH | |
| 97 | 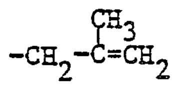 | 5-F | $CH_3$ | $OCH_3$ | O | N | |
| 98 | 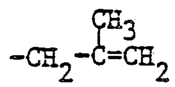 | 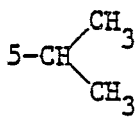 | $CH_3$ | $OCH_3$ | O | N | |
| 99 | 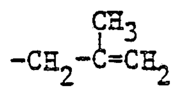 | 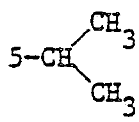 | $CH_3$ | $OCH_3$ | O | CH | |
| 100 | 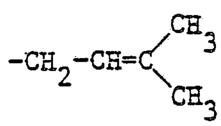 | 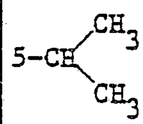 | $CH_3$ | $OCH_3$ | O | CH | |
| 101 | 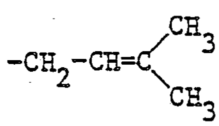 | 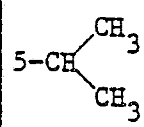 | $CH_3$ | $OCH_3$ | O | N | |
| 102 | $-CH_2-CH=CH_2$ | 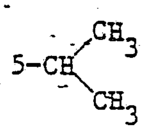 | $CH_3$ | $OCH_3$ | O | N | Smp. 259-260° |
| 103 | $-CH_2-CH=CH_2$ | 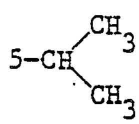 | $CH_3$ | $OCH_3$ | O | CH | Smp. 139-140° |
| 104 | $-CH_2-CH=CH_2$ | 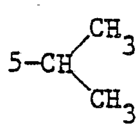 | $C_2H_5$ | $OCH_3$ | O | N | |
| 105 | $-CH_2-CH=CH_2$ | 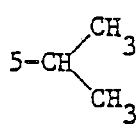 | $OCH_3$ | $OCH_3$ | O | N | Smp. 219-220° |

Tabelle 3 (Fortsetzung)

| Nr. | A | $R_1$ | $R_3$ | $R_4$ | X | E | phys. Daten |
|---|---|---|---|---|---|---|---|
| 106 | $-CH_2-CH_2-OCH_3$ | 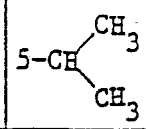 | $CH_3$ | $OCH_3$ | O | N | |
| 107 | $-CH_2-CH_2-OCH_3$ | 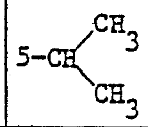 | $CH_3$ | $OCH_3$ | O | CH | |
| 108 | $-CH_2-CH_2-OCH_3$ | $5-CH_3$ | $CH_3$ | $OCH_3$ | O | CH | |
| 109 | $-CH_2-CH_2-OCH_3$ | $5-CH_3$ | $CH_3$ | $OCH_3$ | O | N | |
| 110 | $-CH_2-CH_2-OCH_3$ | $5-CH_3$ | $CH_3$ | Cl | O | CH | |
| 111 | $-CH_2-CH_2-OCH_3$ | $5-CH_3$ | $OCH_3$ | Cl | O | CH | |
| 112 | $-CH_2-CH=CH_2$ | $5-CH_3$ | $CH_3$ | $OCH_3$ | O | N | Smp. 158-162° |
| 113 | $-CH_2-CH=CH_2$ | $5-CH_3$ | $CH_3$ | $OCH_3$ | O | N | |
| 114 | $-CH_2-CH=CH_2$ | $5-CH_3$ | $C_2H_5$ | $OCH_3$ | O | N | |
| 115 | $-CH_2-CH=CH_2$ | $5-CH_3$ | $OCH_3$ | $OCH_3$ | O | N | Smp. 160-165° |
| 116 | $-CH_2-CH=CH_2$ | $5-CH_3$ | $OCH_3$ | $OCH_3$ | O | CH | |
| 117 | $-CH_2-CH=CH-CH_3$ | $5-CH_3$ | $OCH_3$ | $OCH_3$ | O | CH | |
| 118 | $-CH_2-CH=CH-CH_3$ | $5-CH_3$ | $CH_3$ | $OCH_3$ | O | CH | Smp. 179-182° |
| 119 | $-CH_2-CH=CH-CH_3$ | $5-CH_3$ | $CH_3$ | $OCH_3$ | O | N | |
| 120 | $-CH_2-CH=CH-CH_3$ | $5-NO_2$ | $CH_3$ | $OCH_3$ | O | N | |
| 121 | $-CH_2-CH=CH-CH_3$ | $5-NO_2$ | $CH_3$ | $OCH_3$ | O | CH | |
| 122 | $-CH_2-CH=CH-CH_3$ | $5-NO_2$ | $C_2H_5$ | $OCH_3$ | O | N | |
| 123 | $-CH_2-CH=CH-CH_3$ | $5-NO_2$ | $OCH_3$ | $OCH_3$ | O | N | |
| 124 | $-CH_2-CH=CH_2$ | $5-NO_2$ | $OCH_3$ | $OCH_3$ | O | N | |
| 125 | $-CH_2-CH=CH_2$ | $5-NO_2$ | $CH_3$ | $OCH_3$ | O | N | |
| 126 | $-CH_2-CH=CH_2$ | $5-NO_2$ | $CH_3$ | $OCH_3$ | O | CH | |

Tabelle 3 (Fortsetzung)

| Nr. | A | $R_1$ | $R_3$ | $R_4$ | X | E | phys. Daten |
|---|---|---|---|---|---|---|---|
| 127 | $-CH_2-SCH_3$ | $5-NO_2$ | $CH_3$ | $OCH_3$ | O | CH | |
| 128 | $-CH_2-SCH_3$ | $5-NO_2$ | $CH_3$ | $OCH_3$ | O | N | |
| 129 | $-CH_2-SCH_3$ | $5-NO_2$ | $OCH_3$ | $OCH_3$ | O | N | |
| 130 | $-CH_2-SCH_3$ | $5-NO_2$ | $OCH_3$ | $OCH_3$ | O | CH | |
| 131 | $-CH_2-CH=CH_3$ | $5-OCH_3$ | $CH_3$ | $OCH_3$ | O | N | Smp. 142–143° |
| 132 | $-CH_2-CH=CH_3$ | $5-OCH_3$ | $CH_3$ | $OCH_3$ | O | CH | Smp. 138–139° |
| 133 | $-CH_2-CH=CH_3$ | $5-OCH_3$ | $OCH_3$ | $OCH_3$ | O | CH | |
| 134 | $-CH_2-CH=CH_3$ | $5-OCH_3$ | $OCH_3$ | $OCH_3$ | O | N | |
| 135 | $-CH_2-CH=CH_3$ | $5-OCH_3$ | $OCH_3$ | $C_2H_5$ | O | N | |
| 136 | $-CH_2-\overset{CH_3}{\overset{\vert}{C}}=CH_2$ | $5-OCH_3$ | $OCH_3$ | $CH_3$ | O | N | |
| 137 | $-CH_2-\overset{CH_3}{\overset{\vert}{C}}=CH_2$ | $5-OCH_3$ | $OCH_3$ | $CH_3$ | O | CH | |
| 138 | $-CH_2-\overset{CH_3}{\overset{\vert}{C}}=CH_2$ | $5-OCH_3$ | $OCH_3$ | $OCH_3$ | O | N | |
| 139 | $-CH_2-CH=CH-CH_3$ | $5-OCH_3$ | $OCH_3$ | $CH_3$ | O | N | |
| 140 | $-CH_2-CH=CH-CH_3$ | $5-OCH_3$ | $OCH_3$ | $CH_3$ | O | CH | |
| 141 | $-CH_2-CH=CH-CH_3$ | $5-OCH_3$ | $OCH_3$ | $OCH_3$ | O | CH | |
| 142 | $-CH_2-CH=CH-CH_3$ | $5-OCH_3$ | $OCH_3$ | $OCH_3$ | O | N | |
| 143 | $-CH_2-OCH_3$ | $5-OCH_3$ | $OCH_3$ | $OCH_3$ | O | N | |
| 144 | $-CH_2-OCH_3$ | $5-OCH_3$ | $CH_3$ | $OCH_3$ | O | N | |
| 145 | $-CH_2-OCH_3$ | $5-OCH_3$ | $CH_3$ | $OCH_3$ | O | CH | |
| 146 | $-CH_2-OCH_3$ | $5-OCH_3$ | $C_2H_5$ | $OCH_3$ | O | N | |
| 147 | $-CH_2-CH_2-SCH_3$ | $5-OCH_3$ | $CH_3$ | $OCH_3$ | O | N | |

Tabelle 3(Fortsetzung)

| Nr. | A | $R_1$ | $R_3$ | $R_4$ | X | E |
|---|---|---|---|---|---|---|
| 148 | $-CH_2-CH_2-SCH_3$ | $5-OCH_3$ | $CH_3$ | $OCH_3$ | O | CH |
| 149 | $-CH_2-CH_2-SCH_3$ | $5-OCH_3$ | $OCH_3$ | $OCH_3$ | O | N |
| 150 | $-CH_2-CH=CH_2$ | 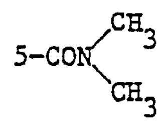 | $CH_3$ | $OCH_3$ | O | N |
| 151 | $-CH_2-CH=CH_2$ | 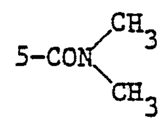 | $CH_3$ | $OCH_3$ | O | CH |
| 152 | 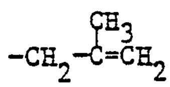 | 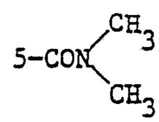 | $CH_3$ | $OCH_3$ | O | N |
| 153 | 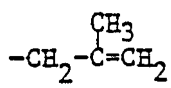 | 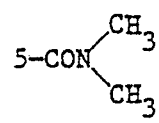 | $CH_3$ | $OCH_3$ | O | CH |
| 154 | 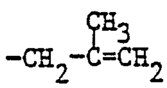 | $5-COOCH_3$ | $CH_3$ | $OCH_3$ | O | CH |
| 155 | 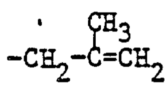 | $5-COOCH_3$ | $CH_3$ | $OCH_3$ | O | N |
| 156 | $-CH_2-CH=CH_2$ | $5-COOCH_3$ | $CH_3$ | $OCH_3$ | O | N |
| 157 | $-CH_2-CH=CH_2$ | $5-COOCH_3$ | $CH_3$ | $OCH_3$ | O | CH |
| 158 | $-CH_2-CH=CH_2$ | $5-COOCH_3$ | $OCH_3$ | $OCH_3$ | O | N |
| 159 | $-CH_2-CH=CH_2$ | $5-COOCH_3$ | $OCH_3$ | $OCH_3$ | O | CH |
| 160 | $-CH_2-OC_2H_5$ | $5-COOCH_3$ | $OCH_3$ | $CH_3$ | O | N |
| 161 | $-CH_2-OC_2H_5$ | $5-COOCH_3$ | $OCH_3$ | $CH_3$ | O | N |
| 162 | 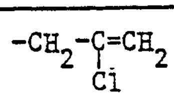 | $5-CH_3$ | $OCH_3$ | $CH_3$ | O | N |
| 163 | 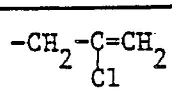 | $5-CH_3$ | $OCH_3$ | $CH_3$ | O | CH |
| 164 | 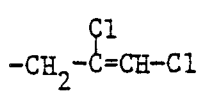 | $5-NO_2$ | $OCH_3$ | $CH_3$ | O | CH |

Tabelle 3 (Fortsetzung)

| Nr. | A | $R_1$ | $R_3$ | $R_4$ | X | E |
|---|---|---|---|---|---|---|
| 165 | Cl<br>$-CH_2-C=CH-Cl$ | $5-NO_2$ | $OCH_3$ | $CH_3$ | O | N |
| 166 | Cl<br>$-CH_2-C=CH-Cl$ | 5-Br | $OCH_3$ | $CH_3$ | O | N |
| 167 | Cl<br>$-CH_2-C=CH-Cl$ | 5-Br | $OCH_3$ | $CH_3$ | O | CH |
| 168 | Cl<br>$-CH_2-C=CH-Cl$ | 5-Cl | $OCH_3$ | $CH_3$ | O | N |
| 169 | Cl<br>$-CH_2-C=CH_2$ | 5-Cl | $CH_3$ | $OCH_3$ | O | CH |
| 170 | Cl<br>$-CH_2-CH=C$<br>$CH_3$ | 5-Cl | $CH_3$ | $OCH_3$ | O | N |
| 171 | Cl<br>$-CH_2-CH=C$<br>$CH_3$ | 5-Cl | $CH_3$ | $OCH_3$ | O | CH |
| 172 | Cl<br>$-CH_2-CH=C$<br>Cl | $CH_3$<br>5-CH<br>$CH_3$ | $CH_3$ | $OCH_3$ | O | CH |
| 173 | Cl<br>$-CH_2-CH=C$<br>Cl | $CH_3$<br>5-CH<br>$CH_3$ | $CH_3$ | $OCH_3$ | O | N |
| 174 | Cl<br>$-CH_2-C=CH_2$ | $3-CH_3$ | $CH_3$ | $OCH_3$ | O | N |
| 175 | Cl<br>$-CH_2-C=CH_2$ | $3-CH_3$ | $CH_3$ | $OCH_3$ | O | CH |
| 176 | Cl<br>$-CH_2-C=CH_2$ | $6-CH_3$ | $CH_3$ | $OCH_3$ | O | N |
| 177 | Cl<br>$-CH_2-C=CH_2$ | $6-CH_3$ | $CH_3$ | $OCH_3$ | O | CH |
| 178 | Cl<br>$-CH_2-C=CH-Cl$ | $6-OCH_3$ | $CH_3$ | $OCH_3$ | O | CH |

Tabelle 3 (Fortsetzung)

| Nr. | A | $R_1$ | $R_3$ | $R_4$ | X | E | phys. Daten |
|---|---|---|---|---|---|---|---|
| 179 | 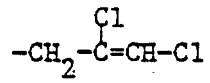 | 6-$OCH_3$ | $CH_3$ | $OCH_3$ | O | N | |
| 180 | 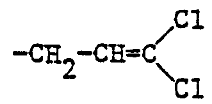 | 5-$OCH_3$ | $CH_3$ | $OCH_3$ | O | N | |
| 181 | 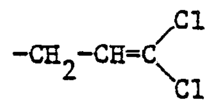 | 5-$OCH_3$ | $CH_3$ | $OCH_3$ | O | CH | |
| 182 | 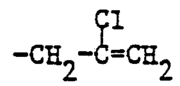 | 6-Cl | $CH_3$ | $OCH_3$ | O | N | |
| 183 | 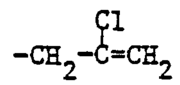 | 6-Cl | $CH_3$ | $OCH_3$ | O | CH | |
| 184 | $-CH_2-CH=CH_2$ | 3-$NO_2$ | $CH_3$ | $OCH_3$ | O | CH | |
| 185 | $-CH_2-CH=CH_2$ | 3-$NO_2$ | $CH_3$ | $OCH_3$ | O | N | |
| 186 | $-CH_2-CH=CH_2$ | 3-$NO_2$ | $C_2H_5$ | $OCH_3$ | O | N | |
| 187 | $-CH_2-CH=CH_2$ | 3-$NO_2$ | $OCH_3$ | $OCH_3$ | O | N | |
| 188 | 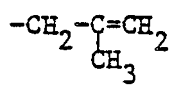 | 3-$NO_2$ | $CH_3$ | $OCH_3$ | O | N | |
| 189 | 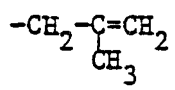 | 3-$NO_2$ | $CH_3$ | $OCH_3$ | O | CH | |
| 190 | 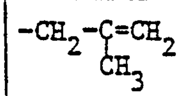 | 3-$NO_2$ | $OCH_3$ | $OCH_3$ | O | N | |
| 191 | 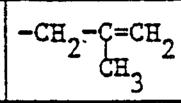 | 3-$NO_2$ | $OCH_3$ | $OCH_3$ | O | CH | |
| 192 | 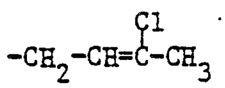 | 3-$NO_2$ | $OCH_3$ | $OCH_3$ | O | CH | |
| 193 | 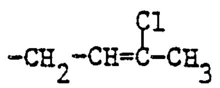 | 3-$NO_2$ | $OCH_3$ | $CH_3$ | O | CH | |
| 194 | 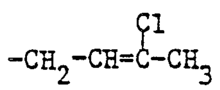 | 3-$NO_2$ | $OCH_3$ | $CH_3$ | O | N | |
| 195 | $-CH_2-CH=CH_2$ | 3-$CH_3$ | $OCH_3$ | $CH_3$ | O | N | Smp. 255-256° |

Tabelle 3 (Fortsetzung)

| Nr. | A | $R_1$ | $R_3$ | $R_4$ | X | E | phys. Daten |
|---|---|---|---|---|---|---|---|
| 196 | $-CH_2-CH=CH_2$ | $3-CH_3$ | $OCH_3$ | $CH_3$ | O | CH | Smp. 163–164° |
| 197 | $-CH_2-CH=CH_2$ | $3-CH_3$ | $C_2H_5$ | $CH_3$ | O | N | |
| 198 | $-CH_2-CH=CH_2$ | $3-CH_3$ | $OCH_3$ | $CH_3$ | O | N | Smp. 183–184° |
| 199 | $-CH_2-CH=CH_2$ | $3-CH_3$ | $OCH_3$ | $CH_3$ | O | CH | |
| 200 | $-CH_2-CH=CH_2$ | 3-Cl | $OCH_3$ | $CH_3$ | O | CH | |
| 201 | $-CH_2-CH=CH_2$ | 3-Cl | $OCH_3$ | $CH_3$ | O | N | |
| 202 | $-CH_2-CH=CH_2$ | 3-Cl | $OCH_3$ | $OCH_3$ | O | N | |
| 203 | $-CH_2-CH=CH_2$ | 3-Cl | $OCH_3$ | $OCH_3$ | O | CH | |
| 204 | $-CH_2-\overset{CH_3}{C}=CH_2$ | 3-Cl | $OCH_3$ | $OCH_3$ | O | N | |
| 205 | $-CH_2-\overset{CH_3}{C}=CH_2$ | 3-Cl | $OCH_3$ | $CH_3$ | O | N | |
| 206 | $-CH_2-\overset{CH_3}{C}=CH_2$ | 3-Cl | $OCH_3$ | $CH_3$ | O | CH | |
| 207 | $-CH_2-CH=CH-CH_3$ | 3-Cl | $CH_3$ | $OCH_3$ | O | N | |
| 208 | $-CH_2-CH=CH-CH_3$ | 3-Cl | $CH_3$ | $OCH_3$ | O | CH | |
| 209 | $-CH_2-\overset{CH_3}{C}=CH_2$ | $3-OCH_3$ | $CH_3$ | $OCH_3$ | O | CH | |
| 210 | $-CH_2-\overset{CH_3}{C}=CH_2$ | $3-OCH_3$ | $CH_3$ | $OCH_3$ | O | N | |
| 211 | $-CH_2-CH=CH_2$ | $3-OCH_3$ | $CH_3$ | $OCH_3$ | O | N | |
| 212 | $-CH_2-CH=CH_2$ | $3-OCH_3$ | $CH_3$ | $OCH_3$ | O | CH | |
| 213 | $-CH_2-CH=CH_2$ | $3-OCH_3$ | $OCH_3$ | $OCH_3$ | O | CH | |
| 214 | $-CH_2-CH=CH_2$ | $3-OCH_3$ | $C_2H_5$ | $OCH_3$ | O | N | |
| 215 | $-CH_2-CH=CH_2$ | $3-OCH_3$ | $OCH_3$ | $OCH_3$ | O | N | |

Tabelle 4

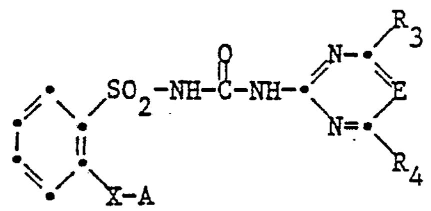

| Nr. | A | $R_3$ | $R_4$ | X | E | phys. Daten |
|---|---|---|---|---|---|---|
| 301 | $-CH_2-\overset{CH_3}{\overset{\vert}{C}}=CH_2$ | $CH_3$ | $CH_3$ | O | N | Smp. 95-103° |
| 302 | $-CH_2-\overset{CH_3}{\overset{\vert}{C}}=CH_2$ | $CH_3$ | $OCH_3$ | O | N | Smp. 142-143° |
| 303 | $-CH_2-\overset{CH_3}{\overset{\vert}{C}}=CH_2$ | $OCH_3$ | $OCH_3$ | O | N | Smp. 145-153° |
| 304 | $-CH_2-\overset{CH_3}{\overset{\vert}{C}}=CH_2$ | $OCH_3$ | $OC_2H_5$ | O | N | |
| 305 | $-CH_2-\overset{CH_3}{\overset{\vert}{C}}=CH_2$ | $-CH_2-CH_3$ | $OCH_3$ | O | N | Smp. 150-152° |
| 306 | $-CH_2-\overset{CH_3}{\overset{\vert}{C}}=CH_2$ | $-CH_2-CH_3$ | $CH_3$ | O | N | Smp. 129-130° |
| 307 | $-CH_2-\overset{CH_3}{\overset{\vert}{C}}=CH_2$ | Cl | $OCH_3$ | O | N | |
| 308 | $-CH_2-\overset{CH_3}{\overset{\vert}{C}}=CH_2$ | $-OCH(CH_3)_2$ | $OCH_3$ | O | N | |
| 309 | $-CH_2-\overset{CH_3}{\overset{\vert}{C}}=CH_2$ | $-OCH(CH_3)_2$ | $OCH_3$ | O | N | |
| 310 | $-CH_2-\overset{CH_3}{\overset{\vert}{C}}=CH_2$ | $-OCH(CH_3)_2$ | $CH_3$ | O | N | |
| 311 | $-CH_2-\overset{CH_3}{\overset{\vert}{C}}=CH_2$ | $OCH_3$ | $SCH_3$ | O | N | |
| 312 | $-CH_2-\overset{CH_3}{\overset{\vert}{C}}=CH_2$ | $OC_2H_5$ | $OC_2H_5$ | O | N | Smp. 118-120° |
| 313 | $-CH_2-\overset{CH_3}{\overset{\vert}{C}}=CH_2$ | $OCH_3$ | $-CH_2OCH_3$ | O | N | |

Tabelle 4 (Fortsetzung)

| Nr. | A | $R_3$ | $R_4$ | X | E | phys. Daten |
|---|---|---|---|---|---|---|
| 314 | $-CH_2-\overset{CH_3}{C}=CH_2$ | $CH_2Cl$ | $OCH_3$ | O | N | |
| 315 | $-CH_2-\overset{CH_3}{C}=CH_2$ | $CH_2F$ | $OCH_3$ | O | N | |
| 316 | $-CH_2-\overset{CH_3}{C}=CH_2$ | $CH_3$ | $OCH_3$ | O | CH | Smp.172-177° |
| 317 | $-CH_2-\overset{CH_3}{C}=CH_2$ | $OCH_3$ | $OCH_3$ | O | CH | |
| 318 | $-CH_2-\overset{CH_3}{C}=CH_2$ | $CH_3$ | $CH_3$ | O | CH | |
| 319 | $-CH_2-\overset{CH_3}{C}=CH_2$ | $C_2H_5$ | $OCH_3$ | O | CH | |
| 320 | $-CH_2-\overset{CH_3}{C}=CH_2$ | $OCH_3$ | Cl | O | CH | Smp.179-180° |
| 321 | $-CH_2-\overset{CH_3}{C}=CH_2$ | $CH_3$ | Cl | O | CH | |
| 322 | $-CH_2-\overset{CH_3}{C}=CH_2$ | $CF_3$ | $OCH_3$ | O | CH | |
| 323 | $-CH_2-\overset{CH_3}{C}=CH_2$ | $-CH_2-OCH_3$ | $OCH_3$ | O | CH | |
| 324 | $-CH_2-\overset{CH_3}{C}=CH_2$ | Br | $OCH_3$ | O | CH | |
| 325 | $-CH_2-\overset{CH_3}{C}=CH_2$ | Br | $CH_3$ | O | CH | |
| 326 | $-CH_2-\overset{CH_3}{C}=CH_2$ | $OCH_3$ | $SCH_3$ | O | CH | |
| 327 | $-CH_2-\overset{CH_3}{C}=CH_2$ | $CH_3$ | $SCH_3$ | O | CH | |
| 328 | $-CH_2-\overset{CH_3}{C}=CH_2$ | $OCH_3$ | $OC_2H_5$ | O | CH | |

Tabelle 4 (Fortsetzung)

| Nr. | A | $R_3$ | $R_4$ | X | E | phys. Daten |
|---|---|---|---|---|---|---|
| 329 | $-CH_2-CH=CH-CH_3$ | $CH_3$ | $OCH_3$ | O | N | Smp.163-164° |
| 330 | $-CH_2-CH=CH-CH_3$ | $CH_3$ | $CH_3$ | O | N | |
| 331 | $-CH_2-CH=CH-CH_3$ | $C_2H_5$ | $OCH_3$ | O | N | Smp.139-140° |
| 332 | $-CH_2-CH=CH-CH_3$ | $C_2H_5$ | $CH_3$ | O | N | Smp.148-151° |
| 333 | $-CH_2-CH=CH-CH_3$ | $OCH_3$ | $OCH_3$ | O | N | Smp.148-149° |
| 334 | $-CH_2-CH=CH-CH_3$ | $OCH_3$ | $OC_2H_5$ | O | N | |
| 335 | $-CH_2-CH=CH-CH_3$ | $OC_2H_5$ | $OC_2H_5$ | O | N | |
| 336 | $-CH_2-CH=CH-CH_3$ | $-CH_2-OCH_3$ | $OCH_3$ | O | N | |
| 337 | $-CH_2-CH=CH-CH_3$ | $CH_3$ | $OCH_3$ | O | CH | Smp.185-186° |
| 338 | $-CH_2-CH=CH-CH_3$ | $OCH_3$ | $OCH_3$ | O | CH | |
| 339 | $-CH_2-CH=CH-CH_3$ | $CH_3$ | $CH_3$ | O | CH | |
| 340 | $-CH_2-CH=CH-CH_3$ | $-CH_2-OCH_3$ | $OCH_3$ | O | CH | |
| 341 | $-CH_2-CH=CH-CH_3$ | $CH_3$ | Cl | O | CH | |
| 342 | $-CH_2-CH=CH-CH_3$ | $OCH_3$ | Cl | O | CH | |
| 343 | $-CH_2-CH=CH-CG_3$ | $OCH_3$ | $OC_2H_5$ | O | CH | |
| 344 | 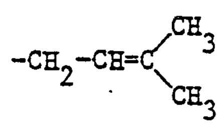 | $CH_3$ | $OCH_3$ | O | N | |
| 345 | 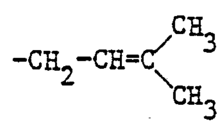 | $CH_3$ | $CH_3$ | O | N | |
| 346 | 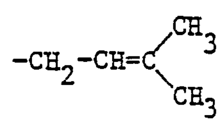 | $C_2H_5$ | $CH_3$ | O | N | |
| 347 | 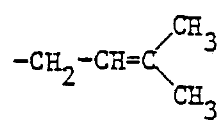 | $C_2H_5$ | $OCH_3$ | O | N | |

Tabelle 4 (Fortsetzung)

| Nr. | A | $R_3$ | $R_4$ | X | E |
|---|---|---|---|---|---|
| 348 | 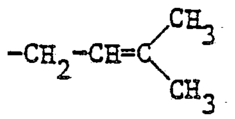 | $OCH_3$ | $OCH_3$ | O | N |
| 349 | 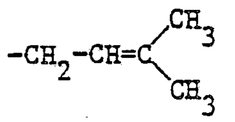 | $OCH_3$ | $OC_2H_5$ | O | N |
| 350 | 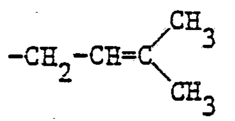 | $OC_2H_5$ | $OC_2H_5$ | O | N |
| 351 | 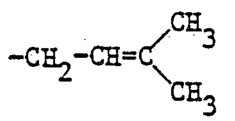 | $-CH_2-OCH_3$ | $OCH_3$ | O | N |
| 352 | 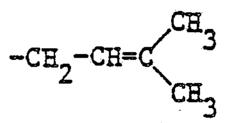 | $CH_3$ | $OCH_3$ | O | CH |
| 353 | 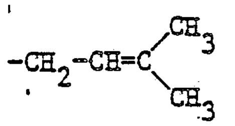 | $OCH_3$ | $OCH_3$ | O | CH |
| 354 | 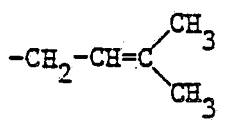 | $CH_3$ | $CH_3$ | O | CH |
| 355 | 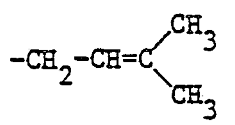 | $OCH_3$ | Cl | O | CH |
| 356 | 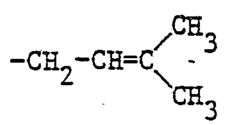 | $CH_3$ | Cl | O | CH |
| 357 | 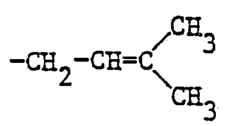 | $OCH_3$ | $OC_2H_5$ | O | CH |
| 358 | 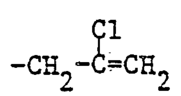 | $CH_3$ | $OCH_3$ | O | N |
| 359 | 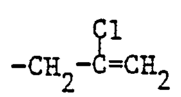 | $CH_3$ | $CH_3$ | O | N |

Tabelle 4 (Fortsetzung)

| Nr. | A | $R_3$ | $R_4$ | X | E | phys. Daten |
|---|---|---|---|---|---|---|
| 360 | $-CH_2-\overset{Cl}{\overset{\vert}{C}}=CH_2$ | $C_2H_5$ | $CH_3$ | O | N | |
| 361 | $-CH_2-\overset{Cl}{\overset{\vert}{C}}=CH_2$ | $C_2H_5$ | $OCH_3$ | O | N | |
| 362 | $-CH_2-\overset{Cl}{\overset{\vert}{C}}=CH_2$ | $OCH_3$ | $OCH_3$ | O | N | |
| 363 | $-CH_2-\overset{Cl}{\overset{\vert}{C}}=CH_2$ | $OCH_3$ | $OC_2H_5$ | O | N | |
| 364 | $-CH_2-\overset{Cl}{\overset{\vert}{C}}=CH_2$ | $OC_2H_5$ | $OC_2H_5$ | O | N | |
| 365 | $-CH_2-\overset{Cl}{\overset{\vert}{C}}=CH_2$ | $-CH_2-OCH_3$ | $OCH_3$ | O | N | |
| 366 | $-CH_2-\overset{Cl}{\overset{\vert}{C}}=CH_2$ | $CH_3$ | $OCH_3$ | O | CH | |
| 367 | $-CH_2-\overset{Cl}{\overset{\vert}{C}}=CH_2$ | $OCH_3$ | $OCH_3$ | O | CH | |
| 368 | $-CH_2-\overset{Cl}{\overset{\vert}{C}}=CH_2$ | $CH_3$ | $CH_3$ | O | CH | |
| 369 | $-CH_2-\overset{Cl}{\overset{\vert}{C}}=CH_2$ | $OCH_3$ | Cl | O | CH | |
| 370 | $-CH_2-\overset{Cl}{\overset{\vert}{C}}=CH_2$ | $CH_3$ | Cl | O | CH | |
| 371 | $-CH_2-\overset{Cl}{\overset{\vert}{C}}=CH_2$ | $OC_2H_5$ | $OCH_3$ | O | CH | |
| 372 | $-CH_2-\overset{Cl}{\overset{\vert}{C}}=CH_2$ | $-CH_2-OCH_3$ | $OCH_3$ | O | CH | |
| 373 | $-CH_2-OCH_3$ | $CH_3$ | $OCH_3$ | O | N | Smp.: 155-157° |
| 374 | $-CH_2-OCH_3$ | $OCH_3$ | $OCH_3$ | O | N | |
| 375 | $-CH_2-OCH_3$ | $C_2H_5$ | $OCH_3$ | O | N | |
| 376 | $-CH_2-OCH_3$ | $C_2H_5$ | $CH_3$ | O | N | |

Tabelle 4 (Fortsetzung)

| Nr. | A | $R_3$ | $R_4$ | X | E | phys. Daten |
|---|---|---|---|---|---|---|
| 377 | $-CH_2-OCH_3$ | $OCH_3$ | $OCH_3$ | O | N | |
| 378 | $-CH_2-OCH_3$ | $OCH_3$ | $OC_2H_5$ | O | N | |
| 379 | $-CH_2-OCH_3$ | $OC_2H_5$ | $OC_2H_5$ | O | N | |
| 380 | $-CH_2-OCH_3$ | $-CH_2-OCH_3$ | $OCH_3$ | O | N | |
| 381 | $-CH_2-OCH_3$ | $OCH_3$ | $SCH_3$ | O | N | |
| 382 | $-CH_2-OCH_3$ | $OCH_3$ | $CH_3$ | O | CH | Smp.: 168-175° |
| 383 | $-CH_2-OCH_3$ | $OCH_3$ | $OCH_3$ | O | CH | |
| 384 | $-CH_2-OCH_3$ | $CH_3$ | $CH_3$ | O | CH | |
| 385 | $-CH_2-OCH_3$ | $CH_3$ | Cl | O | CH | Smp.: 137-143° |
| 386 | $-CH_2-OCH_3$ | $OCH_3$ | Cl | O | CH | |
| 387 | $-CH_2-OCH_3$ | $C_2H_5$ | $OCH_3$ | O | CH | |
| 388 | $-CH_2-OCH_3$ | $OC_2H_5$ | $OCH_3$ | O | CH | |
| 389 | 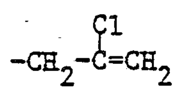 | $C_2H_5$ | $OCH_3$ | O | CH | |
| 390 | 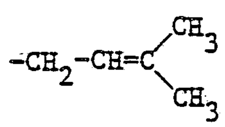 | $C_2H_5$ | $OCH_3$ | O | CH | |
| 391 | $-CH_2-CH=CH-CH_3$ | $C_2H_5$ | $OCH_3$ | O | CH | |
| 392 | $-CH_2-S-CH_3$ | $CH_3$ | $OCH_3$ | O | N | |
| 393 | $-CH_2-S-CH_3$ | $CH_3$ | $CH_3$ | O | N | |
| 394 | $-CH_2-S-CH_3$ | $C_2H_5$ | $CH_3$ | O | N | |
| 295 | $-CH_2-S-CH_3$ | $C_2H_5$ | $OCH_3$ | O | N | |
| 396 | $-CH_2-S-CH_3$ | $OCH_3$ | $OCH_3$ | O | N | |
| 397 | $-CH_2-S-CH_3$ | $OCH_3$ | $OC_2H_5$ | O | N | |
| 398 | $-CH_2-S-CH_3$ | $-CH_2-OCH_3$ | $OCH_3$ | O | N | |

Tabelle 4 (Fortsetzung)

| Nr. | A | $R_3$ | $R_4$ | X | E | phys. Daten |
|---|---|---|---|---|---|---|
| 399 | $-CH_2-S-CH_3$ | $CH_3$ | $OCH_3$ | O | CH | |
| 400 | $-CH_2-S-CH_3$ | $OCH_3$ | $OCH_3$ | O | CH | |
| 401 | $-CH_2-S-CH_3$ | $CH_3$ | $CH_3$ | O | CH | |
| 402 | $-CH_2-S-CH_3$ | $C_2H_5$ | $OCH_3$ | O | CH | |
| 403 | $-CH_2-S-CH_3$ | $OCH_3$ | Cl | O | CH | |
| 404 | $-CH_2-S-CH_3$ | $CH_3$ | Cl | O | CH | |
| 405 | $-CH_2-S-CH_3$ | $OCH_3$ | $OC_2H_5$ | O | CH | |
| 406 | $-CH_2-CH_2-OC_2H_5$ | $CH_3$ | $OCH_3$ | O | N | |
| 407 | $-CH_2-CH_2-OC_2H_5$ | $CH_3$ | $CH_3$ | O | N | |
| 408 | $-CH_2-CH_2-OC_2H_5$ | $C_2H_5$ | $CH_3$ | O | N | |
| 409 | $-CH_2-CH_2-OC_2H_5$ | $C_2H_5$ | $OCH_3$ | O | N | |
| 410 | $-CH_2-CH_2-OC_2H_5$ | $OCH_3$ | $OCH_3$ | O | N | |
| 411 | $-CH_2-CH_2-OC_2H_5$ | $CH_3$ | $OCH_3$ | O | CH | |
| 412 | $-CH_2-CH_2-OC_2H_5$ | $OCH_3$ | $OCH_3$ | O | CH | |
| 413 | $-CH_2-CH_2-OC_2H_5$ | $OCH_3$ | Cl | O | CH | Smp. 166-168° |
| 414 | $-CH_2-CH_2-OC_2H_5$ | $CH_3$ | Cl | O | CH | |

Tabelle 4 (Fortsetzung)

| Nr. | A | $R_3$ | $R_4$ | X | E |
|---|---|---|---|---|---|
| 432 | $-CH_2-SO-CH_3$ | $CH_3$ | $OCH_3$ | O | N |
| 433 | $-CH_2-SO-CH_3$ | $CH_3$ | $OCH_3$ | O | CH |
| 434 | $-CH_2-SO-CH_3$ | $OCH_3$ | $OCH_3$ | O | N |
| 435 | $-CH_2-SO-CH_3$ | $OCH_3$ | $OCH_3$ | O | CH |
| 436 | $-CH_2-SO_2-CH_3$ | $OCH_3$ | $OCH_3$ | O | CH |
| 437 | $-CH_2-SO_2-CH_3$ | $CH_3$ | $OCH_3$ | O | CH |
| 438 | $-CH_2-SO_2-CH_3$ | $CH_3$ | $CH_3$ | O | CH |

Tabelle 4 (Fortsetzung)

| Nr. | A | $R_3$ | $R_4$ | X | E |
|---|---|---|---|---|---|
| 439 | $-CH_2-SO_2-CH_3$ | $CH_3$ | $OCH_3$ | O | N |
| 440 | $-CH_2-SO_2-CH_3$ | $OCH_3$ | $OCH_3$ | O | N |
| 441 | $-CH_2-CH_2-SO-CH_3$ | $OCH_3$ | $OCH_3$ | O | N |
| 442 | $-CH_2-CH_2-SO-CH_3$ | $OCH_3$ | $CH_3$ | O | N |
| 443 | $-CH_2-CH_2-SO-CH_3$ | $OCH_3$ | $CH_3$ | O | CH |
| 444 | $-CH_2-CH_2-SO_2-CH_3$ | $OCH_3$ | $CH_3$ | O | CH |
| 445 | $-CH_2-CH_2-SO_2-CH_3$ | $OCH_3$ | $CH_3$ | O | N |
| 446 | $-CH_2-CH_2-SO_2-CH_3$ | $CH_3$ | $OCH_3$ | O | N |
| 449 | 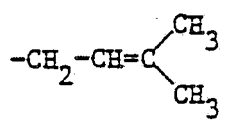 | $CH_3$ | $OCH_3$ | S | CH |
| 450 | 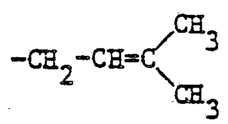 | $CH_3$ | $OCH_3$ | S | N |
| 453 | $-CH_2-CH_2-OCH_3$ | $CH_3$ | $OCH_3$ | S | CH |
| 454 | $-CH_2-CH_2-OCH_3$ | $CH_3$ | $OCH_3$ | S | N |
| 455 | $-(CH_2)_2-CH=CH_2$ | $CH_3$ | $OCH_3$ | O | N |
| 456 | $-(CH_2)_2-CH=CH_2$ | $CH_3$ | $OCH_3$ | O | CH |
| 457 | $-(CH_2)_2-CH=CH_2$ | $CH_3$ | $CH_3$ | O | N |
| 458 | $-(CH_2)_2-CH=CH_2$ | $CH_3$ | $CH_3$ | O | CH |
| 459 | $-(CH_2)_3-CH=CH_2$ | $CH_3$ | $OCH_3$ | O | N |
| 460 | $-(CH_2)_3-CH=CH_2$ | $CH_3$ | $OCH_3$ | O | CH |
| 461 | 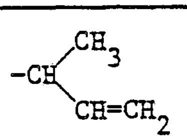 | $CH_3$ | $OCH_3$ | O | N |

Tabelle 4 (Fortsetzung)

| Nr. | A | $R_3$ | $R_4$ | X | E | phys. Daten |
|---|---|---|---|---|---|---|
| 462 | 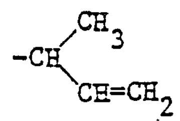 | $CH_3$ | $OCH_3$ | O | CH | |
| 463 | $-CH_2-OC_2H_5$ | $CH_3$ | $OCH_3$ | O | CH | |
| 464 | $-CH_2-OC_2H_5$ | $CH_3$ | $OCH_3$ | O | N | |
| 465 | $-CH_2-OC_2H_5$ | $C_2H_5$ | $OCH_3$ | O | N | |
| 466 | $-CH_2-OC_2H_5$ | $CH_3$ | $CH_3$ | O | N | |
| 467 | $-CH_2-OC_2H_5$ | $CH_3$ | $CH_3$ | O | CH | |
| 468 | $-CH_2-CH_2-OCH_3$ | $CH_3$ | $CH_3$ | O | CH | |
| 469 | $-CH_2-CH_2-OCH_3$ | $CH_3$ | $CH_3$ | O | N | |
| 470 | $-CH_2-CH_2-OCH_3$ | $CH_3$ | $OCH_3$ | O | N | Smp. 134-138° |
| 471 | $-CH_2-CH_2-OCH_3$ | $CH_3$ | $OCH_3$ | O | CH | Smp. 160-162° |
| 472 | $-CH_2-CH_2-SCH_3$ | $CH_3$ | $OCH_3$ | O | CH | |
| 473 | $-CH_2-CH_2-SCH_3$ | $CH_3$ | $OCH_3$ | O | N | |
| 474 | 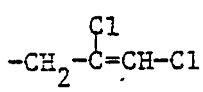 | $CH_3$ | $OCH_3$ | O | N | |
| 475 | 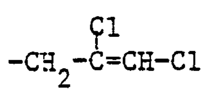 | $CH_3$ | $OCH_3$ | O | CH | |
| 476 | 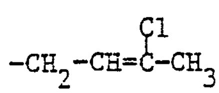 | $CH_3$ | $OCH_3$ | O | CH | |
| 477 | 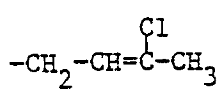 | $CH_3$ | $OCH_3$ | O | N | |
| 479 | 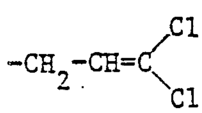 | $CH_3$ | $OCH_3$ | O | N | |
| 479 | 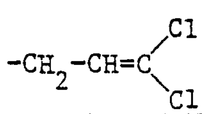 | $CH_3$ | $OCH_3$ | O | CH | |

Tabelle 4 (Fortsetzung)

| Nr. | A | $R_3$ | $R_4$ | X | E | phys. Daten |
|---|---|---|---|---|---|---|
| 480 | $-CH_2-CH_2-O-CH_2-CH=CH_2$ | $CH_3$ | $OCH_3$ | O | CH | |
| 481 | $-CH_2-CH_2-O-CH_2-CH=CH_2$ | $CH_3$ | $OCH_3$ | O | N | |
| 482 | $-CCl=CCl_2$ | $CH_3$ | $OCH_3$ | O | N | |
| 483 | $-CCl=CHCl$ | $OCH_3$ | $OCH_3$ | O | CH | |
| 484 | $-CCl=CCl_2$ | $OCH_3$ | $OCH_3$ | O | N | |
| 485 | $-CCl=CCl_2$ | $CH_3$ | $OCH_3$ | O | CH | |
| 486 | $-CCl=CCl_2$ | $OCH_3$ | $OCH_3$ | O | CH | |
| 487 | $-CCl=CCl_2$ | $CH_3$ | Cl | O | CH | |
| 488 | $-CCl=CCl_2$ | $OCH_3$ | Cl | O | N | |
| 489 | $-CF=CF_2$ | $OCH_3$ | Cl | O | N | |
| 490 | $-CF=CF_2$ | $CH_3$ | Cl | O | CH | |
| 491 | $-CH=CCl_2$ | $OCH_3$ | $OCH_3$ | O | N | |
| 492 | $-CH=CCl_2$ | $CH_3$ | $OCH_3$ | O | CH | |
| 493 | $-CH=CCl_2$ | $OCH_3$ | $OCH_3$ | O | CH | |
| 494 | $-CH=CCl_2$ | $C_2H_5$ | $OCH_3$ | O | N | |
| 495 | $-CF=CF_2$ | $C_2H_5$ | $OCH_3$ | O | N | |
| 496 | $-CCl=CHCl$ | $OCH_3$ | $OCH_3$ | S | N | |
| 497 | $-CCl=CHCl$ | $CH_3$ | $OCH_3$ | O | N | Smp. 169-172° |
| 498 | $-CCl=CHCl$ | $C_2H_5$ | $OCH_3$ | O | N | |
| 499 | $-CCl=CHCl$ | $CH_3$ | $CH_3$ | O | N | |
| 500 | $-CCl=CHCl$ | $OCH_3$ | Cl | O | CH | Smp. 196-198° |
| 501 | $-CCl=CHCl$ | $OCH_3$ | $OC_2H_5$ | O | N | |
| 502 | $-CCl=CHCl$ | $OCH_3$ | $OCH_3$ | O | CH | |

Tabelle 4 (Fortsetzung)

| Nr. | A | $R_3$ | $R_4$ | X | E | phys. Daten |
|---|---|---|---|---|---|---|
| 503 | -CCl=CHCl | $C_2H_5$ | $OCH_3$ | O | CH | |
| 504 | -CCl=CHCl | $CH_3$ | Cl | O | CH | |
| 505 | -CCl=CHCl | $CH_3$ | $OCH_3$ | S | CH | |
| 506 | -CCl=CHCl | $OCH_3$ | $OCH_3$ | S | CH | |
| 507 | $-CF=CF_2$ | $CH_3$ | $OCH_3$ | O | N | |
| 508 | $-CF=CF_2$ | $OCH_3$ | $OCH_3$ | O | N | |
| 509 | $-CF=CF_2$ | $CH_3$ | $OCH_3$ | O | CH | |
| 510 | $-CF=CF_2$ | $OCH_3$ | $OCH_3$ | O | CH | |
| 511 | $-CH_2-CH=CH_2$ | $-CH(CH_3)_2$ | $OCH_3$ | O | CH | |
| 512 | $-CH_2-CH=CH_2$ | $OCH_3$ | $SC_2H_5$ | O | CH | |
| 513 | $-CH_2-CH=CH_2$ | $OCH_3$ | $SC_2H_5$ | O | N | |
| 514 | $-CH_2-CH=CH_2$ | $OCH_3$ | $-SCH(CH_3)_2$ | O | N | |
| 515 | $-CH_2-CH=CH_2$ | Cl | $CH_3$ | O | N | Smp. 128-130° |
| 516 | $-CH_2-CH=CH_2$ | $CH_3$ | Br | O | N | |
| 517 | $-CH_2-CH=CH_2$ | $CHF_2$ | $OCH_3$ | O | N | |
| 518 | $-CH_2-CH=CH_2$ | $CHF_2$ | $CH_3$ | O | N | |
| 519 | $-CH_2-CH=CH_2$ | $-CH_2CF_3$ | Cl | O | N | |
| 520 | $-CH_2-CH=CH_2$ | $-CH_2CF_3$ | $OCH_3$ | O | N | |
| 521 | $-CH_2-CH=CH_2$ | $-CH_2CF_3$ | $CH_3$ | O | N | |
| 522 | $-CH_2-CH=CH_2$ | $OC_2H_5$ | $OC_2H_5$ | O | N | Smp.138-142° |
| 523 | $-CH_2-CH=CH_2$ | $OC_2H_5$ | $OC_2H_5$ | O | CH | |
| 524 | $-CH_2-CH=CH_2$ | $C_2H_5$ | $OCH_3$ | O | N | Smp. 206° |
| 525 | $-CH_2-CH=CH_2$ | $C_2H_5$ | $OC_2H_5$ | O | N | |

Tabelle 4 (Fortsetzung)

| Nr. | A | $R_3$ | $R_4$ | X | E | phys. Daten |
|---|---|---|---|---|---|---|
| 526 | $-CH_2-CH=CH_2$ | $CH_3$ | $OC_2H_5$ | O | N | Smp. 129-131° |
| 527 | $-CH_2-CH=CH_2$ | $CH_3$ | $CH_3$ | O | CH | Smp. 180-182° |
| 528 | $-CH_2-CH=CH_2$ | $CH_3$ | $OCH_3$ | O | CH | Smp. 98-100° |
| 529 | $-CH_2-CH=CH_2$ | $CH_3$ | Br | O | N | |
| 530 | $-CH_2-CH=CH_2$ | $CH_3$ | H | O | N | |
| 531 | $-CH_2-CH=CH_2$ | $C_2H_5$ | Cl | O | CH | |
| 532 | $-CH_2-CH=CH_2$ | $CH_3$ | Cl | O | CH | Smp. 153-154° |
| 533 | $-CH_2-CH=CH_2$ | $CH_3$ | $SCH_3$ | O | CH | |
| 534 | $-CH_2-CH=CH_2$ | $CH_3$ | F | O | CH | |
| 535 | $-CH_2-CH=CH_2$ | $CH_3$ | Br | O | CH | |
| 536 | $-CH_2-CH=CH_2$ | $C_2H_5$ | $OC_2H_5$ | O | CH | |
| 537 | $-CH_2-CH=CH_2$ | $C_2H_5$ | $SCH_3$ | O | CH | |
| 538 | $-CH_2-CH=CH_2$ | $CF_3$ | $CH_3$ | O | CH | |
| 539 | $-CH_2-CH=CH_2$ | $CH_2Cl$ | $CH_3$ | O | CH | |
| 540 | $-CH_2-CH=CH_2$ | $CH_2Cl$ | $OCH_3$ | O | CH | |
| 541 | $-CH_2-CH=CH_2$ | $OCH_3$ | Cl | O | CH | Smp. 173-179° |
| 542 | $-CH_2-CH=CH_2$ | Cl | Cl | O | CH | |
| 543 | $-CH_2-CH=CH_2$ | $OCH_3$ | $SCH_3$ | O | CH | |
| 544 | $-CH_2-CH=CH_2$ | $OCH_3$ | $-OCH(CH_3)_2$ | O | CH | |
| 545 | $-CH_2-CH=CH_2$ | $CH_2F$ | $OCH_3$ | O | CH | |
| 546 | $-CH_2-CH=CH_2$ | $CH_2F$ | $CH_3$ | O | CH | |
| 547 | $-CH_2-CH=CH_2$ | $CF_3$ | $OCH_3$ | O | CH | |
| 548 | $-CH_2-CH=CH_2$ | $C_2H_5$ | $-OCH(CH_3)_2$ | O | N | |

Tabelle 4 (Fortsetzung)

| Nr. | A | $R_3$ | $R_4$ | X | E | phys. Daten |
|---|---|---|---|---|---|---|
| 549 | $-CH_2-CH=CH_2$ | $C_2H_5$ | Cl | O | N | |
| 550 | $-CH_2-CH=CH_2$ | $C_2H_5$ | $SCH_3$ | O | N | |
| 551 | $-CH_2-CH=CH_2$ | $C_2H_5$ | $CH_3$ | O | N | Smp. 128-131° |
| 552 | $-CH_2-CH=CH_2$ | $C_2H_5$ | $C_2H_5$ | O | N | |
| 553 | $-CH_2-CH=CH_2$ | $OCH_3$ | $-OCH(CH_3)_2$ | O | N | Smp. 119-120° |
| 554 | $-CH_2-CH=CH_2$ | $OCH_3$ | $-OCH(CH_3)-CH_2CH_3$ | O | N | |
| 555 | $-CH_2-CH=CH_2$ | $CH_3$ | $-CH(CH_3)_2$ | O | N | Smp. 96-98° |
| 556 | $-CH_2-CH=CH_2$ | $-CH(CH_3)_2$ | Cl | O | N | |
| 557 | $-CH_2-CH=CH_2$ | $-CH(CH_3)_2$ | $OCH_3$ | O | N | Smp. 124-128° |
| 558 | $-CH_2-CH=CH_2$ | $-CH(CH_3)_2$ | $OC_2H_5$ | O | N | |
| 559 | $-CH_2-CH=CH_2$ | $-CH(CH_3)_2$ | $SCH_3$ | O | N | |
| 560 | $-CH_2-CH=CH_2$ | $CH_2Cl$ | $CH_3$ | O | N | |
| 561 | $-CH_2-CH=CH_2$ | $CH_2Cl$ | $OCH_3$ | O | N | |
| 562 | $-CH_2-CH=CH_2$ | $CH_2F$ | $CH_3$ | O | N | |
| 563 | $-CH_2-CH=CH_2$ | $CH_2F$ | $OCH_3$ | O | N | |
| 564 | $-CH_2-CH=CH_2$ | $CH_2F$ | $OC_2H_5$ | O | N | |
| 565 | $-CH_2-CH=CH_2$ | $-CH_2-OCH_3$ | $C_2H_5$ | O | N | |
| 571 | $-CH_2-CH=CH_2$ | $SCH_3$ | Cl | O | N | |

Tabelle 4 (Fortsetzung)

| Nr. | A | $R_3$ | $R_4$ | X | E | phys. Daten |
|---|---|---|---|---|---|---|
| 572 | $-CH_2-CH=CH_2$ | $SCH_3$ | $OCH_3$ | O | N | |
| 573 | $-CH_2-CH=CH_2$ | $SCH_3$ | $OC_2H_5$ | O | N | |
| 574 | $-CH_2-CH=CH_2$ | $SCH_3$ | 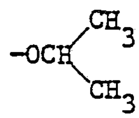 | O | N | |
| 575 | $-CH_2-CH=CH_2$ | $-OCH(CH_3)_2$ | Cl | O | N | |
| 576 | $-CH_2-CH=CH_2$ | $CF_3$ | $OCH_3$ | O | N | |
| 577 | $-CH_2-CH=CH_2$ | $CF_3$ | $CH_3$ | O | N | |
| 578 | $-CH_2-CH=CH_2$ | $CF_3$ | $OC_2H_5$ | O | N | |
| 579 | $-CH_2-CH=CH_2$ | $CCl_3$ | $OCH_3$ | O | N | |
| 580 | $-CH_2-CH=CH_2$ | $CCl_3$ | $SCH_3$ | O | N | |
| 581 | $-CH_2-CH=CH_2$ | $CH_3$ | Cl | O | N | Smp.128-130° |
| 582 | $-CH_2-CH=CH_2$ | $OCH_3$ | Cl | O | N | |
| 583 | $-CH_2-CH=CH_2$ | $OCH_3$ | F | O | N | |
| 584 | $-CH_2-CH=CH_2$ | $OCH_3$ | Br | O | N | |
| 585 | $-CH_2-CH=CH_2$ | $CH_3$ | F | O | N | |
| 586 | $-CH_2-CH=CH_2$ | $OCH_3$ | $OCH_3$ | O | CH | |
| 587 | $-CH_2-CH=CH_2$ | $CH_3$ | $CH_3$ | O | N | Smp.139.141° |
| 588 | $-CH_2-CH=CH_2$ | $OCH_3$ | $OC_2H_5$ | O | N | |
| 589 | $-CH_2-CH=CH_2$ | $OCH_3$ | $OC_2H_5$ | O | CH | |
| 590 | $-CH_2-CH=CH_2$ | $CH_2OCH_3$ | $OCH_3$ | O | CH | |
| 591 | $-CH_2-CH=CH_2$ | $CH_2OCH_3$ | $OCH_3$ | O | N | |
| 592 | $-CH_2-CH=CH_2$ | $C_2H_5$ | $OCH_3$ | O | CH | |

Tabelle 4 (Fortsetzung)

| Nr. | A | $R_3$ | $R_4$ | X | E | phys. Daten |
|---|---|---|---|---|---|---|
| 593 | $-CH_2-CH=CH_2$ | $CH_2OCH_3$ | $CH_3$ | O | N | |
| 595 | $-CH_2-CH=CH_2$ | $OCH_3$ | $OCH_3$ | O | N | Smp. 148-149° |
| 596 | -CH=CHCl | $CH_3$ | $OCH_3$ | O | N | |
| 597 | -CH=CHCl | $CH_3$ | $OCH_3$ | S | N | |
| 598 | -CCl=CHCl | $CH_3$ | $OCH_3$ | O | CH | |
| 599 | $-CH=CCl_2$ | $CH_3$ | $OCH_3$ | O | N | |
| 600 | -CCl=CHCl | $OCH_3$ | $OCH_3$ | O | N | |
| 601 | $-CH_2-CH=CH_2$ | $CH_3$ | $OCH_3$ | O | N | Smp. 146-147° |

Tabelle 5

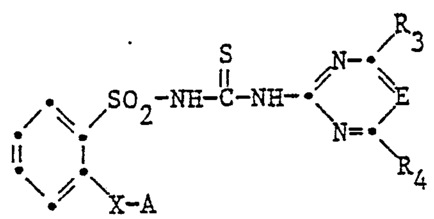

| Nr. | A | $R_3$ | $R_4$ | X | E |
|---|---|---|---|---|---|
| 650 | $-CH_2-CH=CH_2$ | $CH_3$ | $OCH_3$ | O | N |
| 651 | $-CH_2-CH_2-OCH_3$ | $CH_3$ | $OCH_3$ | O | N |
| 652 | -CCl=CH-Cl | $CH_3$ | $OCH_3$ | O | N |
| 653 | $-CH_2-OCH_3$ | $CH_3$ | $OCH_3$ | O | N |
| 654 | $-CH_2-CH=CH-CH_3$ | $OCH_3$ | $OCH_3$ | O | N |
| 655 | $-CH_2-C(CH_3)=CH_2$ | $CH_3$ | $OCH_3$ | O | CH |

(Siehe Tabelle 6 Seite 44 ff.)

Tabelle 6

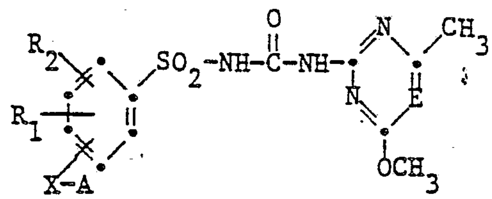

| Nr. | A | Stellung von-X-A | $R_1$ | $R_2$ | X | E |
|---|---|---|---|---|---|---|
| 701 | $-CH_2-CH=CH_2$ | 5 | $2-CH_3$ | H | O | N |
| 702 | $-CH_2-CH=CH_2$ | 5 | $2-CH_3$ | H | O | CH |
| 703 | $-CH_3-\overset{CH_3}{\overset{\vert}{C}}=CH_2$ | 5 | $2-CH_3$ | H | O | CH |
| 704 | $-CH_3-\overset{CH_3}{\overset{\vert}{C}}=CH_2$ | 5 | $2-CH_3$ | H | O | N |
| 705 | $-CH_2-CH=CH-CH_3$ | 5 | $2-CH_3$ | H | O | N |
| 706 | $-CH_2-CH=CH-CH_3$ | 5 | $2-CH_3$ | H | O | CH |
| 707 | $-CH_2-\overset{Cl}{\overset{\vert}{C}}=CH_2$ | 5 | $2-CH_3$ | H | O | CH |
| 708 | $-CH_2-\overset{Cl}{\overset{\vert}{C}}=CH_2$ | 5 | $2-CH_3$ | H | O | N |
| 709 | $-CH_2-CH=CH_2$ | 5 | 2-Cl | H | O | N |
| 710 | $-CH_2-CH=CH_2$ | 5 | 2-Cl | H | O | CH |
| 711 | $-CH_2-\overset{Cl}{\overset{\vert}{C}}=CH_2$ | 5 | 2-Cl | H | O | CH |
| 712 | $-CH_2-\overset{Cl}{\overset{\vert}{C}}=CH_2$ | 5 | 2-Cl | H | O | N |
| 713 | $-CH_2-\overset{CH_3}{\overset{\vert}{C}}=CH_2$ | 5 | 2-Cl | H | O | N |
| 714 | $-CH_2-\overset{CH_3}{\overset{\vert}{C}}=CH_2$ | 5 | 2-Cl | H | O | CH |
| 715 | $-CH_2-O-CH_3$ | 5 | 2-Cl | H | O | CH |
| 716 | $-CH_2-O-CH_3$ | 5 | 2-Cl | H | O | N |

Tabelle 6 (Fortsetzung)

| Nr. | A | Stellung von -X-A | $R_1$ | $R_2$ | X | E |
|---|---|---|---|---|---|---|
| 717 | $-CH_2-S-CH_3$ | 5 | 2-Cl | H | O | N |
| 718 | $-CH_2-S-CH_3$ | 5 | 2-Cl | H | O | CH |
| 719 | $-CH_2-CH=CH-CH_3$ | 5 | 2-Cl | H | O | CH |
| 720 | $-CH_2-CH=CH-CH_3$ | 5 | 2-Cl | H | O | N |
| 721 | $-CH_2-CH_2-OCH_3$ | 5 | 2-Cl | H | O | N |
| 722 | $-CH_2-CH_2-OCH_3$ | 5 | 2-Cl | H | O | CH |
| 723 | $-CH_2-OC_2H_5$ | 5 | 2-Cl | H | O | N |
| 724 | $-CH_2-OC_2H_5$ | 5 | 2-Cl | H | O | CH |
| 725 | $-CH_2-CH_2-SCH_3$ | 5 | 2-Cl | H | O | CH |
| 726 | $-CH_2-CH_2-SCH_3$ | 5 | 2-Cl | H | O | N |
| 727 | $-CH_2-CH=CH_2$ | 3 | $2-CH_3$ | H | O | N |
| 728 | $-CH_2-CH=CH_2$ | 3 | $2-CH_3$ | H | O | CH |
| 729 | $CH_3$<br>$-CH_2-C=CH_3$ | 3 | $2-CH_3$ | H | O | CH |
| 730 | $CH_3$<br>$-CH_2-C=CH_3$ | 3 | $2-CH_3$ | H | O | N |
| 731 | $-CH_2-CH=CH_2$ | 3 | $2-OCH_3$ | H | O | N |
| 732 | $-CH_2-CH=CH_2$ | 3 | $2-OCH_3$ | H | O | CH |
| 733 | $CH_3$<br>$-CH_2-C=CH_2$ | 3 | $2-OCH_3$ | H | O | CH |
| 734 | $CH_3$<br>$-CH_2-C=CH_2$ | 3 | $2-OCH_3$ | H | O | N |
| 735 | Cl<br>$-CH_2-C=CH_2$ | 3 | $2-OCH_3$ | H | O | N |
| 736 | Cl<br>$-CH_2-C=CH_2$ | 3 | $2-OCH_3$ | H | O | CH |

Tabelle 6 (Fortsetzung)

| Nr. | A | Stellung von -X-A | $R_1$ | $R_2$ | X | E | phys. Daten |
|---|---|---|---|---|---|---|---|
| 737 | $-CH_2-CH=CH-CH_3$ | 3 | 2-$OCH_3$ | H | O | CH | |
| 738 | $-CH_2-CH=CH-CH_3$ | 3 | 2-$OCH_3$ | H | O | N | |
| 739 | $-CH_2-CH=CH_2$ | 5 | 2-$OCH_3$ | H | O | N | |
| 740 | $-CH_2-CH=CH_2$ | 5 | 2-$OCH_3$ | H | O | CH | |
| 741 | $CH_3$<br>$-CH_2-C=CH_2$ | 5 | 2-$OCH_3$ | H | O | CH | |
| 742 | $CH_3$<br>$-CH_2-C=CH_2$ | 5 | 2-$OCH_3$ | H | O | N | |
| 743 | $-CH_2-CH=CH-CH_3$ | 5 | 2-$OCH_3$ | H | O | N | |
| 744 | $-CH_2-CH=CH-CH_3$ | 5 | 2-$OCH_3$ | H | O | CH | |
| 745 | Cl<br>$-CH_2-C=CH_2$ | 5 | 2-$OCH_3$ | H | O | CH | |
| 746 | Cl<br>$-CH_2-C=CH_2$ | 5 | 2-$OCH_3$ | H | O | N | |
| 747 | $-CH_2-CH=CH_2$ | 3 | 5-Br | 2-$OCH_3$ | O | N | Smp. 258-259° |
| 748 | $-CH_2-CH=CH_2$ | 3 | 5-Br | 2-$OCH_3$ | O | CH | Smp. 156-167° |
| 749 | $CH_3$<br>$-CH_2-C=CH_2$ | 3 | 5-Br | 2-$OCH_3$ | O | CH | |
| 750 | $CH_3$<br>$-CH_2-C=CH_2$ | 3 | 5-Br | 2-$OCH_3$ | O | N | |
| 751 | $-CH_2-CH=CH_2$ | 3 | 5-$COOCH_3$ | 2-$OCH_3$ | O | N | |
| 752 | $-CH_2-CH=CH_2$ | 3 | 5-$COOCH_3$ | 2-$OCH_3$ | O | CH | |
| 753 | $CH_3$<br>$-CH_2-C=CH_2$ | 3 | 5-$COOCH_3$ | 2-$OCH_3$ | O | CH | |
| 754 | $CH_3$<br>$-CH_2-C=CH_2$ | 3 | 5-$COOCH_3$ | 2-$OCH_3$ | O | N | |

Tabelle 6 (Fortsetzung)

| Nr. | A | Stellung von-X-A | $R_1$ | $R_2$ | X | E |
|---|---|---|---|---|---|---|
| 755 | $-CH_2-CH=CH-CH_3$ | 3 | $5-COOCH_3$ | $2-OCH_3$ | O | N |
| 756 | $-CH_2-CH=CH-CH_3$ | 3 | $5-COOCH_3$ | $2-OCH_3$ | O | CH |
| 757 | $-CH_2-CH=CH_2$ | 2 | $5-NO_2$ | $3-CF_3$ | O | N |
| 758 | $-CH_2-CH=CH_2$ | 2 | $5-NO_2$ | $3-CF_3$ | O | CH |
| 759 | $-CH_2-CH=CH_2$ | 2 | $5-NO_2$ | 3-Cl | O | CH |
| 760 | $-CH_2-CH=CH_2$ | 2 | $5-NO_2$ | 3-Cl | O | N |
| 761 | $-CH-C(CH_3)=CH_2$ | 2 | $5-NO_2$ | 3-Cl | O | N |
| 762 | $-CH-C(CH_3)=CH_2$ | 2 | $5-NO_2$ | 3-Cl | O | CH |
| 763 | $-CH_2-CH=CH_2$ | 2 | $5-CF_3$ | $3-NO_2$ | O | CH |
| 764 | $-CH_2-CH=CH_2$ | 2 | $5-CF_3$ | $3-NO_2$ | O | N |
| 765 | $-CH_2-CH=CH_2$ | 2 | $5-CH_3$ | $3-CH_3$ | O | N |
| 766 | $-CH_2-CH=CH_2$ | 2 | $5-CH_3$ | $3-CH_3$ | O | CH |
| 767 | $-CH_2-CH=CH-CH_3$ | 2 | $5-CH_3$ | $3-CH_3$ | O | CH |
| 768 | $-CH_2-CH=CH-CH_3$ | 2 | $5-CH_3$ | $3-CH_3$ | O | N |
| 769 | $-CH_2-CH=CH-CH_3$ | 2 | 5-Cl | $3-NO_2$ | O | N |
| 770 | $-CH_2-CH=CH-CH_3$ | 2 | 5-Cl | $3-NO_2$ | O | CH |
| 771 | $-CH_2-CH=CH_2$ | 2 | 5-Cl | $3-NO_2$ | O | CH |
| 772 | $-CH_2-CH=CH_2$ | 2 | 5-Cl | $3-NO_2$ | O | N |
| 773 | $-CH_2-CH=CH_2$ | 2 | 5-Cl | 3-Cl | O | N |
| 774 | $-CH_2-CH=CH_2$ | 2 | 5-Cl | 3-Cl | O | CH |
| 775 | $-CH_2-CH_2-OCH_3$ | 2 | 5-Cl | 3-Cl | O | CH |
| 776 | $-CH_2-CH_2-OCH_3$ | 2 | 5-Cl | 3-Cl | O | N |

Tabelle 6 (Fortsetzung)

| Nr. | A | Stellung von-X-A | $R_1$ | $R_2$ | X | E |
|---|---|---|---|---|---|---|
| 777 | $-CH_2-OCH_3$ | 2 | 5-Cl | 3-Cl | O | N |
| 778 | $-CH_2-OCH_3$ | 2 | 5-Cl | 3-Cl | O | CH |
| 779 | $-CH_2-\overset{CH_3}{\overset{\mid}{C}}=CH_2$ | 2 | 5-Cl | 3-Cl | O | CH |
| 780 | $-CH_2-\overset{CH_3}{\overset{\mid}{C}}=CH_2$ | 2 | 5-Cl | 3-Cl | O | N |
| 781 | $-CH_2-CH=CH_2$ | 2 | 5-Br | $3-OCH_3$ | O | N |
| 782 | $-CH_2-CH=CH_2$ | 2 | 5-Br | $3-OCH_3$ | O | CH |
| 783 | $-CH_2-CH=CH_2$ | 3 | 5-Br | $2-OCH_3$ | O | CH |
| 784 | $-CH_2-CH=CH_2$ | 3 | 5-Br | $2-OCH_3$ | O | N |
| 785 | $-CH_2-CH=CH_2$ | 2 | $5-COOCH_3$ | $3-OCH_3$ | O | N |
| 786 | $-CH_2-CH=CH_2$ | 2 | $5-COOCH_3$ | $3-OCH_3$ | O | CH |
| 787 | $-CH_2-CH=CH_2$ | 3 | $5-COOCH_3$ | $2-OCH_3$ | O | CH |
| 788 | $-CH_2-CH=CH_2$ | 3 | $5-COOCH_3$ | $2-OCH_3$ | O | N |
| 789 | $-CH_2-\overset{CH_3}{\overset{\mid}{C}}=CH_2$ | 3 | $5-COOCH_3$ | $2-OCH_3$ | O | N |
| 790 | $-CH_2-\overset{CH_3}{\overset{\mid}{C}}=CH_2$ | 3 | $5-COOCH_3$ | $2-OCH_3$ | O | CH |
| 791 | $-CH_2-CH=CH_2$ | 2 | $5-CH_3$ | 3-Br | O | CH |
| 792 | $-CH_2-CH=CH_2$ | 2 | $5-CH_3$ | 3-Br | O | N |
| 793 | $-CH_2-OCH_3$ | 2 | $5-CH_3$ | 3-Br | O | N |
| 794 | $-CH_2-OCH_3$ | 2 | $5-CH_3$ | 3-Br | O | CH |
| 795 | $-CH_2-CH=CH_2$ | 2 | 5-Br | $3-NO_2$ | O | N |
| 796 | $-CH_2-CH=CH_2$ | 2 | 5-Br | $3-NO_2$ | O | CH |
| 797 | $-CH_2-CH=CH_2$ | 2 | 5-Cl | 3-Br | O | CH |

Tabelle 6 (Fortsetzung)

| Nr. | A | Stellung von -X-A | $R_1$ | $R_2$ | X | E | physikalische Daten |
|---|---|---|---|---|---|---|---|
| 798 | $-CH_2-CH=CH_2$ | 2 | 5-Cl | 3-Br | O | N | Smp. 175° |
| 799 | $-CH_2-C(CH_3)=CH_2$ | 2 | 5-Cl | 3-Br | O | N | |
| 800 | $-CH_2-C(CH_3)=CH_2$ | 2 | 5-Cl | 3-Br | O | CH | |
| 801 | $-CH_2-CH_2-O-CH_3$ | 2 | 5-Cl | 3-Br | O | CH | |
| 802 | $-CH_2-CH_2-O-CH_3$ | 2 | 5-Cl | 3-Br | O | N | |
| 803 | $-CH_2-O-CH_3$ | 5 | $2-NO_2$ | H | O | CH | |
| 804 | $-CH_2-O-CH_3$ | 3 | H | H | S | CH | |
| 805 | $-CH_2-CH_2-OCH_3$ | 2 | $5-OC_2H_4-OOH_3$ | H | O | N | |
| 806 | $-CH_2-CH=CH_2$ | 2 | $5-OCH_2-CH=CH_2$ | H | O | N | |
| 807 | $-CH_2-OCH_3$ | 2 | $5-OCH_2-OCH_3$ | H | O | N | |
| 808 | $-CH_2-CH_2-OCH_3$ | 2 | $5-S-C_2H_4-OCH_3$ | H | S | N | |
| 809 | $-CH_2-SCH_3$ | 2 | $5-S-CH_2-SCH_3$ | H | S | N | |
| 810 | $-CH_2SO-CH_3$ | 2 | $5-S-CH_2-SOCH_3$ | H | S | N | |
| 811 | $-CH_2-SO_2-CH_3$ | 2 | $5-CH_2-SO_2-CH_3$ | H | S | N | |

Formulierungsbeispiele

Beispiel 4 :

Formulierungsbeispiele für flüssige Wirkstoffe der Formel I (% = Gewichtsprozent)

| a) Emulsionskonzentrate | a) | b) | c) |
|---|---|---|---|
| Wirkstoff | 20 % | 10 % | 1 % |
| Ca-Dodecylbenzolsulfonat | 5 % | 4 % | 2 % |
| Ricinusöl-polyäthylenglykoläther (36 Mol AeO) | 5 % | 4 % | 4 % |
| Cyclohexanon | 30 % | 20 % | — |
| Xylolgemisch | 40 % | 62 % | 93 % |

Aus solchen Konzentraten können durch Verdünnen mit Wasser Emulsionen jeder gewünschten Konzentration hergestellt werden.

| b) Lösungen | a) | b) | c) | c) |
|---|---|---|---|---|
| Wirkstoff | 20 % | 10 % | 5 % | 1 % |
| Aethylenglykol-monomethyl-äther | 30 % | — | 50 % | 50 % |
| Polyäthylenglykol M G 400 | — | 70 % | — | 20 % |
| N-Methyl-2-pyrrolidon | 50 % | 20 % | 44 % | 28 % |
| Epoxidiertes Kokosnussöl | — | — | 1 % | 1 % |

Die Lösungen sind zur Anwendung in Form kleinster Tropfen geeignet.

| c) Granulate | a) | b) |
|---|---|---|
| Wirkstoff | 5 % | 10 % |
| Kaolin | 94 % | — |
| Hochdisperse Kieselsäure | 1 % | — |
| Attapulgit | — | 90 % |

Der Wirkstoff wird in Methylenchlorid gelöst, auf den Träger aufgesprüht und das Lösungsmittel anschliessend im Vakuum abgedampft.

| d) Stäubemittel | a) | b) |
|---|---|---|
| Wirkstoff | 91 % | 1 % |
| Hochdisperse Kieselsäure | 1 % | 5 % |
| Talkum | 98,9 % | — |
| Kaolin | — | 94 % |

Durch inniges Vermischen der Trägerstoffe mit dem Wirkstoff erhält man gebrauchsfertige Stäubemittel.

Beispiel 5

Formulierungsbeispiele für feste Wirkstoffe der Formel I (% = Gewichtsprozent)

| a) Spritzpulver | a) | b) | c) |
|---|---|---|---|
| Wirkstoff | 20 % | 60 % | 0,5 % |
| Na-Ligninsulfonat | 5 % | 5 % | 5 % |
| Na-Laurylsulfat | 3 % | — | — |
| Na-Diisobutylnaphthalinsulfonat | — | 6 % | 6 % |
| Octylphenolpolyäthylenglykoläther (7-8 Mol AeO) | — | 2 % | 2 % |
| Hochdisperse Kieselsäure | 5 % | 27 % | 27 % |
| Kaolin | 67 % | — | — |
| Natriumchlorid | — | — | 59,5 % |

Der Wirkstoff wird mit den Zusatzstoffen gut vermischt und in einer geeignete Mühle gut vermahlen. Man erhält Spritzpulver, die sich mit Wasser zu Suspensionen jeden gewünschten Konzentration verdünnen lassen.

| b) Emulsions-Konzentrat | a) | b) |
|---|---|---|
| Wirkstoff | 10 % | 1 % |
| Octylphenolpolyäthylenglykolähter (4-5 Mol AeO) | 3 % | 3 % |
| Ca-Dodecylbenzolsulfonat | 3 % | 3 % |
| Ricinusölpolyglykoläther (36 Mol AeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeden gewünschten Konzentration hergestellt werden.

| c) Stäubemittel | a) | b) |
|---|---|---|
| Wirkstoff | 0,1 % | 1 % |
| Talkum | 99,9 % | — |
| Kaolin | — | 99 % |

Man erhält anwendungsfertige Stäubemittel, indem der Wirkstoff mit dem Träger vermischt und auf einer geeigneten Mühle vermahlen wird.

| d) Extruder Granulat | a) | b) |
|---|---|---|
| Wirkstoff | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

| e) Umhüllungs-Granulat | |
|---|---|
| Wirkstoff | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| f) Suspensions-Konzentrat | a) | b) |
|---|---|---|
| Wirkstoff | 40 % | 5 % |
| Aethylenglykol | 10 % | 10 % |
| Nonylphenolpolyäthylenglykoläther (15 Mol AeO) | 6 % | 1 % |
| Na-Ligninsulfonat | 10 % | 5 % |
| Carboxymethylcellulose | 1 % | 1 % |
| 37 %-ige wässrige Formaldehyd-Lösung | 0,2 % | 0,2 % |
| Silikonöl in Form einer 75 %-igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

| g) Salzlösung | |
|---|---|
| Wirkstoff | 5 % |
| Isopropylamin | 1 % |
| Octylphenolpolyäthylenglykoläther (7-8 Mol AeO) | 3 % |
| Wasser | 91 % |

## Biologische Beispiele

### Beispiel 6

Nachweis der Herbizidwirkung vor dem Auflaufen der Pflanzen

Im Gewächshaus werden Pflanzensamen in Blumentöpfen von 12-15 cm Durchmesser gesät. Unmittelbar danach wird die Erdoberfläche mit einer wässrigen Dispersion oder Lösung der Wirkstoffe behandelt. Es werden verschiedene Konzentrationen mit Wirkstoffmengen pro Hextar angewendet. Die Töpfe werden dann im Gewächshaus bei einer Temperatur von 22-25 °C und 50-70 % relative Luftfeuchtigkeit gehalten. Nach 3 Wochen wird der Versuch ausgewertet und die Wirkung nach folgendem Massstab beurteilt :

1 : Pflanzen nicht gekeimt oder total abgestorben,
2-3 : sehr starke Wirkung,
4-6 : mittlere Wirkung,
7-8 : geringe Wirkung,
9 : keine Wirkung (wie unbehandelte Kontrolle).

Versuchsergebnisse (preemergent) :

| Wirkung Aufwandmenge kg AS/ha | Verb. Nr. 601 | | | |
|---|---|---|---|---|
| Testpflanze | 0,25 | 0,12 | 0,06 | 0,03 |
| Weizen | 4 | 7 | 9 | 9 |
| Avena fatua | 2 | 2 | 3 | 4 |
| Alopecurus myos. | 2 | 2 | 2 | 2 |
| Echinochloa c. g. | 2 | 2 | 2 | 2 |
| Rottboellia ex. | 2 | 3 | 3 | 3 |
| Cyperus escul. | 3 | 3 | 3 | 3 |
| Baumwolle | 2 | 2 | 2 | 3 |
| Abutilon | 1 | 1 | 2 | 2 |
| Xanthium Sp. | 2 | 3 | 3 | 3 |
| Chenopodium Sp. | 1 | 1 | 2 | 2 |
| Ipomoea | 2 | 2 | 2 | 2 |
| Sinapis | 2 | 2 | 2 | 2 |
| Galium aparine | 2 | 2 | 2 | 3 |
| Viola tricolor | 2 | 2 | 2 | 2 |

Beispiel 7

Nachweis der Herbizidwirkung nach dem Auflaufen der Pflanzen (Kontaktwirkung)

Eine Anzahl Unkräuter und Kulturpflanzen, sowohl monocotyle wie dicotyle, wurden nach dem Auflaufen, im 4- bis 6-Blattstadium mit einer wässrigen Wirkstoffdispersion in verschiedenen Dosierungen gespritzt und dann bei 24° bis 26 °C und 45-60 % relativer Luftfeuchtigkeit gehalten. 15 Tage nach der Behandlung wird der Versuch ausgewertet und die Wirkung nach dem gleichen Massstab wie im Beispiel 6 bewertet.

(Siehe Tabelle Seite 53 f.)

Versuchsergebnisse (postemergent) :

| Wirkung Aufwandmenge kg/AS/ha | Verb. Nr. 601 | | | |
|---|---|---|---|---|
| Testpflanze | 0,25 | 0,12 | 0,06 | 0,03 |
| Weizen | 7 | 9 | 9 | 9 |
| Mais | 3 | 4 | 7 | 9 |
| Reis trocken | 4 | 5 | 8 | 9 |
| Avena fatua | 4 | 5 | 8 | 9 |
| Alopecurus myos. | 1 | 2 | 2 | 2 |
| Echinochloa c. g. | 2 | 2 | 6 | 6 |
| Cyperus escul. | 4 | 5 | 9 | 9 |
| Baumwolle | 4 | 6 | 7 | 9 |
| Abutilon | 2 | 2 | 2 | 2 |
| Xanthium Sp. | 1 | 1 | 1 | 1 |
| Chenopodium Sp. | 1 | 2 | 2 | 2 |
| Ipomoea | 2 | 3 | 3 | 4 |
| Sinapis | 1 | 1 | 1 | 1 |
| Galium aparine | 1 | 2 | 2 | 2 |
| Viola tricolor | 2 | 2 | 2 | 2 |

Beispiel 8

Nachweis der Keimhemmung an Lagerkartoffeln.

Eine Anzahl im Handel erhältliche Kartoffeln der Sorte « Urgenta » ohne Keime werden gewaschen und abgetrocknet. Danach werden die Kartoffeln für jeweils eine Minute in Wirkstoffemulsionen verschiedener Konzentration getaucht, in Kunststoffschalen auf Filterpapier ausgelegt und bei Temperaturen von 14° und 21 °C im Dunkeln bei 50 % relativer Luftfeuchtigkeit gehalten. Die Auswertung erfolgte 34 Tage nach der Applikation Gleichzeitig wird der Gewichtsverlust der Knollen und das Gewicht der Keime im Vergleich zur unbehandelten Kontrolle ermittelt. Einige der Verbindungen der Forme I zeigten in diesem Versuch eine vollständige Verhinderung der Keimbildung. Gleichzeitig betrug der Gewichtsverlust der Kartoffeln weniger als 10 % des Gewichtsverlustes der Kontrollkartoffeln.

Beispiel 9

Nachweis der Wuchshemmung bei tropischen Bodenbedeckern-Leguminosen (cover crops).

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70 % relativer Luftfeuchtigkeit und 6 000 lux Kunstlicht, pro Tag 14 Stunden, bei Temperaturen von 27° bei Tag und 21 °C bei Nacht gehalten. 4 Wochen nach der Applikation wird der Versuch ausgewertet. Es werden daber der Neuzuwachs im Vergleich zur Kontrolle abgeschätzt und gewochen und die Phytotoxizität bewertet.

In diesem Versuch zeigen die mit einigen der Wirkstoffe der Formel I behandelten Pflanzen eine deutliche Reduktion des Neuzuwachses (weniger als 20 %) ohne dass dabei die Versuchspflanzen geschädigt wurden.

**Patentansprüche** (für die Vertragsstaaten : BE, DE, FR, GB, IT, NL, SE)

1. N-Phenylsulfonyl-N'-diazinyl- und triazinyl-harnstoffe der allgemeinen Formel I

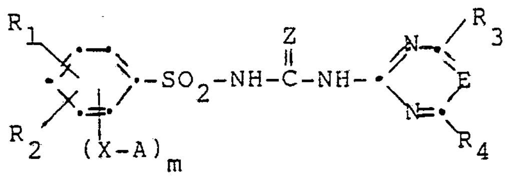

(I)

worin

A einen durch $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Halogenalkoxy, $C_1$-$C_4$-Halogenalkylthio, $C_1$-$C_4$-Halogenalkylsulfinyl oder $C_1$-$C_4$-Halogenalkylsulfonyl substituiertern $C_1$-$C_6$-Alkylrest oder einen gegebenenfalls durch die aufgezählten Substituenten substituierten $C_2$-$C_6$-Alkenylrest oder einen $C_2$-$C_6$-Halogenalkenylrest,

E die Methingruppe oder Stickstoff,

X Sauerstoff, Schwefel, eine Sulfinyl- oder Sulfonylbrücke,

Z Sauerstoff oder Schwefel,

m die Zahl eins oder zwei

$R_1$ Wasserstoff, Halogen, $C_1$-$C_5$-Alkyl, $C_2$-$C_5$-Alkenyl oder einen Rest —Y—$R_5$,

$R_2$ Wasserstoff, Halogen, $C_1$-$C_5$-Alkyl, $C_2$-$C_5$-Alkenyl, $C_1$-$C_4$-Halogenalkyl, oder einen Rest —Y—$R_5$, —$COOR_6$, —$NO_2$ oder —CO—$NR_7R_8$,

$R_3$ und $R_4$ unabhängig voneinander Wasserstoff, $C_1$-$C_4$-Alkyl, $C_1$-$C_4$-Alkoxy, $C_1$-$C_4$-Alkylthio, $C_1$-$C_4$-Halogenalkyl, Halogen oder Alkoxyalkyl mit höchstens 4 Kohlenstoffatomen,

$R_5$ und $R_6$ je $C_1$-$C_5$-Alkyl, $C_2$-$C_5$-Alkenyl oder $C_2$-$C_6$-Alkinyl,

$R_7$ und $R_8$ unabhängig voneinander Wasserstoff, $C_1$-$C_5$-Alkyl, $C_2$-$C_5$-Alkenyl oder $C_2$-$C_6$-Alkinyl und

Y Sauerstoff, Schwefel, eine Sulfinyl- oder Sulfonylbrücke bedeuten,

mit der Massgabe, dass m die Zahl zwei bedeutet, wenn gleichzeitig X für Schwefel, eine Sulfinyl- oder Sulfonylbrücke und A für $C_3$-$C_4$-Alkenyl stehen, sowie die Salze dieser Verbindungen.

2. Verbindungen gemäss Anspruch 1, dadurch gekennzeichnet, dass Z Sauerstoff bedeutet.

3. Verbindungen gemäss Anspruch 1, dadurch gekennzeichnet, dass die Reste $R_3$ und $R_4$ zusammen nicht mehr als 4 Kohlenstoffatome enthalten.

4. Verbindungen gemäss Anspruch 1, dadurch gekennzeichnet, dass Z Sauerstoff und m die Zahl eins bedeuten.

5. Verbindungen gemäss Anspruch 1, dadurch gekennzeichnet, dass Z Sauerstoff und m die Zahl zwei bedeuten.

6. Verbindungen gemäss Anspruch 4, dadurch gekennzeichnet, dass der Rest —X—A in 2- oder 3-Position zur Sulfonylgruppe steht.

7. Verbindungen gemäss Anspruch 6, dadurch gekennzeichnet, dass der Rest —X—A in der 2-Position steht.

8. Verbindungen gemäss Anspruch 5, dadurch gekennzeichnet, dass die beiden Reste —X—A in 2- und in 5-Stellung zur Sulfonylgruppe stehen.

9. Verbindungen gemäss Anspruch 2, dadurch gekennzeichnet, dass nur ein Rest —X—A in der 2-Stellung zum Sulfonylrest vorhanden ist und die Reste $R_3$ und $R_4$ zusammen nicht mehr als 4 Kohlenstoffatome enthalten.

10. Verbindungen gemäss Anspruch 2, dadurch gekennzeichnet, dass zwei Reste —X—A in 2- und 5-Stellung zum Sulfonylrest vorhanden sind und die Reste $R_3$ und $R_4$ zusammen nicht mehr als 4 Kohlenstoffatome enthalten.

11. Verbindungen gemäss Anspruch 9, dadurch gekennzeichnet, dass $R_1$ Wasserstoff bedeutet, und der Rest $R_2$ in der 5- oder 6-Stellung zur Sulfonylgruppe steht.

12. Verbindungen gemäss Anspruch 11, dadurch gekennzeichnet, dass $R_2$ für Wasserstoff, Halogen, $C_1$-$C_4$-Alkoxy, Nitro oder —$COOR_6$ steht.

13. Verbindungen gemäss Anspruch 12, dadurch gekennzeichnet, dass $R_2$ Wasserstoff, Fluor, Nitro oder $C_1$-$C_4$-Alkoxy und $R_3$ und $R_4$ jeweils Wasserstoff, $C_1$-$C_4$-Alkyl, $C_1$-$C_4$-Alkoxy, $C_1$-$C_4$-Alkylthio, Halogen oder Alkoxyalkyl bedeuten, wobei $R_3$ und $R_4$ zusammen höchstens 4 Kohlenstoffatome enthalten.

14. Verbindungen gemäss Anspruch 13, dadurch gekennzeichnet, dass $R_2$ Wasserstoff und $R_3$ und $R_4$ jeweils $C_1$-$C_4$-Alkyl, $C_1$-$C_4$-Alkoxy, Methylthio, Halogen oder Alkoxyalkyl bedeuten.

15. Verbindungen gemäss Anspruch 14, dadurch gekennzeichnet, dass X für Sauerstoff oder Schwefel steht.

16. Verbindungen gemäss Anspruch 15, dadurch gekennzeichnet, dass A für $C_2$-$C_8$-Alkoxyalkyl, $C_2$-$C_4$-Halogenalkenyl oder $C_2$-$C_6$-Alkenyl steht.

17. Verbindungen gemäss Anspruch 16, dadurch gekennzeichnet, dass A für $C_2$-$C_8$-Alkoxyalkyl und $R_3$ und $R_4$ jeweils für Methyl, Aethyl, Chlor oder Methoxy stehen.

18. Verbindungen gemäss Anspruch 16, dadurch gekennzeichnet, dass A für $C_2$-$C_6$-Alkenyl und $R_3$ und $R_4$ jeweils für Methyl, Aethyl, Chlor oder Methoxy stehen.

19. Verbindungen gemäss Anspruch 16, dadurch gekennzeichnet, dass A für durch ein bis drei Halogenatome substituiertes Vinyl und $R_3$ und $R_4$ jeweils für Methyl, Aethyl, Chlor, oder Methoxy stehen.

20. Verbindungen gemäss Anspruch 1, dadurch gekennzeichnet, dass Z Schwefel, X Sauerstoff, $R_3$ und $R_4$ jeweils unabhängig voneinander $C_1$-$C_3$-Alkyl, $C_1$-$C_3$-Alkoxy oder $C_1$-$C_3$-Alkylthio mit zusammen höchstens 4 Kohlenstoffatomen bedeuten und A für $-CH_2-CH=CH_2$, $-CH_2-CH=CH-CH_3$, $-CH_2-CH(CH_3)=CH_2$, Methoxyäthyl, Methoxymethyl oder $-CCl=CHCl$ steht und der Rest —X—A die 2-Stellung besetzt und m die Zahl 1 bedeutet.

21. N-(2-Allyloxyphenylsulfonyl)-N'-(4-methoxy-6-methyl-1,3,5-triazin-2-yl)-harnstoff gemäss Anspruch 1.

22. Verfahren zur Herstellung der Verbindungen der Formel I, Anspruch 1, dadurch gekennzeichnet, dass man ein Phenylsulfonamid der Formel II

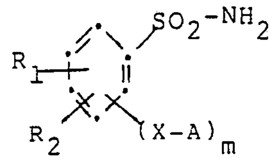
(II)

worin A, $R_1$, $R_2$, X und m die unter Formel I gegebene Bedeutung haben, in Gegenwart einer Base mit einem N-Pyrimidinyl- oder -Triazinyl-carbamat der Formel

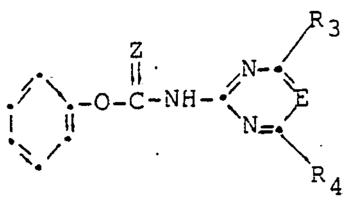
(III)

worin E, $R_3$, $R_4$ und Z die unter Formel I gegebene Bedeutung haben, umsetzt und gegebenenfalls in ihre Salze überführt.

23. Verfahren zur herstellung der Verbindungen der Formel I, Anspruch 1, dadurch gekennzeichnet, dass man ein Phenylsulfonylisocyanat oder -isothiocyanat der Formel IV

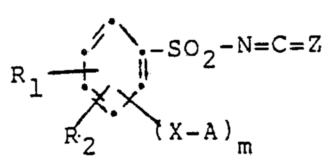
(IV)

worin A, $R_1$, $R_2$, m, X und Z die unter Formel I gegebene Bedeutung haben, gegebenenfalls in Gegenwart einer Base, mit einem Amin der Formel V

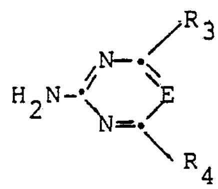
(V)

worin E, $R_3$ und $R_4$ die unter Formel I gegebene Bedeutung haben, umsetzt und gegebenenfalls in ihre Salze überführt.

24. Verfahren zur Herstellung der Verbindungen der Formel I, Anspruch 1, dadurch gekennzeichnet, dass man ein Sulfonamid der oben angegebenen Formel II gegebenenfalls in Gegenwart einer Base mit einem Isocyanat oder Isothiocyanat der Formel VI

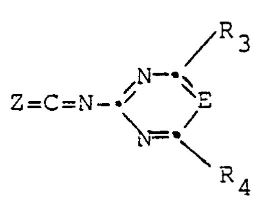
(VI)

worin E, $R_3$, $R_4$ und Z die unter Formel I gegebenen Bedeutung haben, umsetzt und gegebenenfalls in ihre Salze überführt.

25. Verfahren zur Herstellung der Verbindungen der Formel I, Anspruch 1, dadurch gekennzeichnet, dass man N-Phenylsulfonylcarbamat der Formel VII

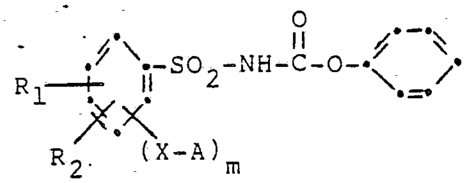

(VII)

worin A, $R_1$, $R_2$, m und X die unter Formel I gegebene Bedeutung haben, mit einem Amin der oben angegebenen Formel V umsetzt und gegebenenfalls in ihre Salze überführt.

26. Verfahren zur Herstellung von Salzen der Formel I gemäss einem der Ansprüche 22 bis 25, dadurch gekennzeichnet, dass man einen Sulfonylharnstoff der Formel I mit einem Amin, einem Alkalimetall- oder Erdalkalimetallhydroxid oder einen quaternären Ammoniumbase umsetzt.

27. Phenylsulfonamide der Formel II

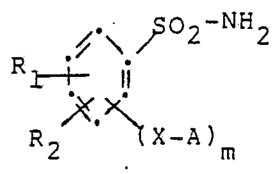

(II)

worin A, $R_1$, $R_2$, m und X die unter Formel I in Anspruch 1 gegebene Bedeutung haben.

28. Phenylsulfonamide der Formel VIII

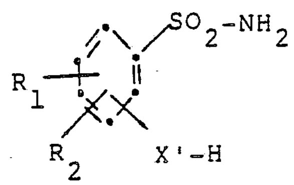

(VIII)

worin $R_1$ und $R_2$ die unter Formel I in Anspruch 1 gegebene Bedeutung haben und X für Sauerstoff oder Schwefel steht, mit der Massgabe, dass $R_2$ nicht Wasserstoff bedeutet und steht einer der Substituenten $R_2$ oder —X'—H in 2-Stellung zur Sulfonamidgruppe steht.

29. Ein herbizides und den Pflanzenwuchs hemmendes Mittel, dadurch gekennzeichnet, dass es neben Träger- und/oder anderen Zuschlagstoffen als Wirkstoff mindestens einen N-Phenylsulfonyl-N'-triazinyl- oder -pyrimidinyl-harnstoff der Formel I, Anspruch 1, enthält.

30. Die Verwendung der N-Phenylsulfonyl-N'-triazinyl- oder -pyrimidinyl-harnstoffe der Formel I, Anspruch 1, oder sie enthaltender Mittel zur Bekämpfung unerwünschten Pflanzenwachstums.

31. Die Verwendung der N-Phenylsulfonyl-N'-triazinyl- oder -pyrimidinyl-harnstoffe der Formel I, Anspruch 1, oder sie enthaltender Mittel zur Hemmung des Pflanzenwachstums.

32. Die Verwendung der N-Phenylsulfonyl-N'-triazinyl- oder -pyrimidinyl-harnstoffe der Formel I, oder sie enthaltender Mittel, gemäss Anspruch 30, zur selektiven pre- oder postemergenten Bekämpfung von Unkräutern in Nutzpflanzenkulturen.

33. Die Verwendung der N-Phenylsulfonyl-N'-triazinyl- oder -pyrimidinyl-harnstoffe der Formel I, oder sie enthaltender Mittel, gemäss Anspruch 31, zur Unterdrückung des Pflanzenwachstums über das 2-Blattstadium hinaus, dadurch gekennzeichnet, dass die Wirkstoffe preemergent angewendet werden.

34. N-(2-Allyloxy-5-methyl-phenylsulfonyl)-N'-(4-methoxy-6-methyl-1,3,5-triazin-2-yl)-harnstoff gemäss Anspruch 1.

35. N-[2-(2-Methallyloxy-phenylsulfonyl]-N'-(4-methoxy-6-methyl-1,3,5-triazin-2-yl)-harnstoff gemäss Anspruch 1.

36. N-[2-(2-Methallyloxy)-phenylsulfonyl]-N'-(4,6-dimethoxy-1,3,5-triazin-2-yl)-harnstoff gemäss Anspruch 1.

37. N-[2-(2-Methallyloxy)-phenylsulfonyl]-N'-(4-äthyl-6-methoxy-1,3,5-triazin-2-yl)-harnstoff gemäss Anspruch 1.

38. N-[2-(2-Methallyloxy)-phenylsulfonyl]-N'-(4,6-diäthoxy-1,3,5-triazin-2-yl)-harnstoff gemäss Anspruch 1.

39. N-[2-(2-Butenyloxy)-phenylsulfonyl]-N'-(4-methoxy-6-methyl-1,3,5-triazin-2-yl)-harnstoff gemäss Anspruch 1.

40. N-[2-(2-Butenyloxy)-phenylsulfonyl]-N'-(4,6-dimethoxy-1,3,5-triazin-2-yl)-harnstoff gemäss Anspruch 1.

41. N-[2-(1,2-Dichlorvinyloxy)-phenylsulfonyl]-N'-(4-methoxy-6-methyl-1,3,5-triazin-2-yl)-harnstoff gemäss Anspruch 1.

42. N-(2-Allyloxy-phenylsulfonyl)-N'-(4,6-diäthoxy-1,3,5-triazin-2-yl)-harnstoff gemäss Anspruch 1.

43. N-(2-Allyloxy-phenylsulfonyl)-N'-(4-äthoxy-6-methyl-1,3,5-triazin-2-yl)-harnstoff gemäss Anspruch 1.

44. N-(2-Allyloxy-phenylsulfonyl)-N'-(4-methoxy-6-isopropyloxy-1,3,5-triazin-2-yl)-harnstoff gemäss Anspruch 1.

45. N-(2-Allyloxy-phenylsulfonyl)-N'-(4,6-dimethoxy-1,3,5-triazin-2-yl)-harnstoff gemäss Anspruch 1.

46. N-[2-(1,2-Dichlorvinyloxy)-phenylsulfonyl]-N'-(4,6-dimethoxy-1,3,5-triazin-2-yl)-harnstoff gemass Anspruch 1.

47. N-[2-(1,2-Dichlorvinyloxy)-phenylsulfonyl]-N'-(4-chlor-6-methoxy-pyrimidin-2-yl)-harnstoff gemäss Anspruch 1.

48. N-(2-Methoxyäthoxy-phenylsulfonyl)-N'-(4-methoxy-6-methyl-1,3,5-triazin-2-yl)-harnstoff gemäss Anspruch 1.

49. N-(2-Methoxymethoxy-phenylsulfonyl)-N'-(4-methoxy-6-methyl-1,3,5-triazin-2-yl)-harnstoff gemäss Anspruch 1.

50. N-(2-Methoxyäthylthio-phenylsulfonyl)-N'-(4-methoxy-6-methyl-1,3,5-triazin-2-yl)-harnstoff gemäss Anspruch 1.

51. N-(2-Methoxyäthoxy-phenylsulfonyl)-N'-(4-chlor-6-methoxy-pyrimidin-2-yl)-harnstoff gemäss Anspruch 1.

52. N-(2-Methoxyäthoxy-phenylsulfonyl)-N'-(4-methoxy-6-methyl-pyrimidin-2-yl)-harnstoff gemäss Anspruch 1.

**Patentansprüche** (für den Vertragsstaat AT)

1. Ein herbizides und den Pflanzenwuchs hemmendes Mittel, dadurch gekennzeichnet, dass es neben Träger- und/oder anderen Zuschlagstoffen als Wirkstoff mindestens einen N-Phenylsulfonyl-N'-diazinyl- und triazinyl-harnstoff der allgemeinen Formel I

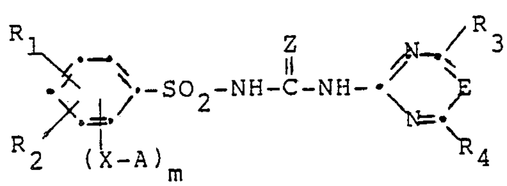

(I)

oder ein Salz dieser Verbindungen enthält, worin

A einen durch $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Halogenalkoxy, $C_1$-$C_4$-Halogenalkylthio, $C_1$-$C_4$-Halogenalkylsulfonyl substituierten $C_1$-$C_6$-Alkylrest oder einen gegebenenfalls durch die aufgezählten Substituenten substituierten $C_2$-$C_6$-Alkenylrest oder einen $C_2$-$C_6$-Halogenalkenylrest,

E die Methingruppe oder Stickstoff,

X Sauerstoff, Schwefel, eine Sulfinyl- oder Sulfonylbrücke,

Z Sauerstoff oder Schwefel,

m die Zahl eins oder zwei

$R_1$ Wasserstoff, Halogen, $C_1$-$C_5$-Alkyl, $C_2$-$C_5$-Alkenyl oder einen Rest —Y—$R_5$,

$R_2$ Wasserstoff, Halogen, $C_1$-$C_5$-Alkyl, $C_2$-$C_5$-Alkenyl, $C_1$-$C_4$-Halogenalkyl, oder einen Rest —Y—$R_5$, —$COOR_6$, —$NO_2$ oder —CO—$NR_7R_8$,

$R_3$ und $R_4$ unabhängig voneinander Wasserstoff, $C_1$-$C_4$-Alkyl, $C_1$-$C_4$-Alkoxy, $C_1$-$C_4$-Alkylthio, $C_1$-$C_4$-Halogenalkyl, Halogen oder Alkoxyalkyl mit höchstens 4 Kohlenstoffatomen,

$R_5$ und $R_6$ je $C_1$-$C_5$-Alkyl, $C_2$-$C_5$-Alkenyl oder $C_2$-$C_6$-Alkinyl,

$R_7$ und $R_8$ unabhängig voneinander Wasserstoff, $C_1$-$C_5$-Alkyl, $C_2$-$C_5$-Alkenyl oder $C_2$-$C_6$-Alkinyl und

Y Sauerstoff, Schwefel, eine Sulfinyl- oder Sulfonylbrücke bedeuten,

mit der Massgabe, dass m die Zahl zwei bedeutet, wenn gleichzeitig X für Schwefel, eine Sulfinyl- oder Sulfonylbrücke und A für $C_3$-$C_4$-Alkenyl stehen.

2. Mittel gemäss Anspruch 1, dadurch gekennzeichnet, dass Z Sauerstoff bedeutet.

3. Mittel gemäss Anspruch 1, dadurch gekennzeichnet, dass die Reste $R_3$ und $R_4$ zusammen nicht mehr als 4 Kohlenstoffatome enthalten.

4. Mittel gemäss Anspruch 1, dadurch gekennzeichnet, dass Z Sauerstoff und m die Zahl eins bedeuten.

5. Mittel gemäss Anspruch 1, dadurch gekennzeichnet, dass Z Sauerstoff und m die Zahl zwei bedeuten.

6. Mittel gemäss Anspruch 4, dadurch gekennzeichnet, dass der Rest —X—A in 2- oder 3-Position zur Sulfonylgruppe steht.

7. Mittel gemäss Anspruch 6, dadurch gekennzeichnet, dass der Rest —X—A in der 2-Position steht.

8. Mittel gemäss Anspruch 5, dadurch gekennzeichnet, dass die beiden Reste —X—A in 2- und in 5-Stellung zur Sulfonylgruppe stehen.

9. Mittel gemäss Anspruch 2, dadurch gekennzeichnet, dass nur ein Rest —X—A in der 2-Stellung zum Sulfonylrest vorhanden ist und die Reste $R_3$ und $R_4$ zusammen nicht mehr als 4 Kohlenstoffatome enthalten.

10. Mittel gemäss Anspruch 2, dadurch gekennzeichnet, dass zwei Reste —X—A in 2- und 5-Stellung zum Sulfonylrest vorhanden sind und die Reste $R_3$ und $R_4$ zusammen nicht mehr als 4 Kohlenstoffatome enthalten.

11. Mittel gemäss Anspruch 9, dadurch gekennzeichnet, dass $R_1$ Wasserstoff bedeutet, und der Rest $R_2$ in der 5- oder 6-Stellung zur Sulfonylgruppe steht.

12. Mittel gemäss Anspruch 11, dadurch gekennzeichnet, dass $R_2$ für Wasserstoff, Halogen, $C_1$-$C_4$-Alkoxy, Nitro oder —$COOR_6$ steht.

13. Mittel gemäss Anspruch 12, dadurch gekennzeichnet, dass $R_2$ Wasserstoff, Fluor, Nitro oder $C_1$-$C_4$-Alkoxy und $R_3$ und $R_4$ jeweils Wasserstoff, $C_1$-$C_4$-Alkyl, $C_1$-$C_4$-Alkoxy, $C_1$-$C_4$-Alkylthio, Halogen oder Alkoxyalkyl bedeuten, wobei $R_3$ und $R_4$ zusammen höchstens 4 Kohlenstoffatome enthalten.

14. Mittel gemäss Anspruch 13, dadurch gekennzeichnet, dass $R_2$ Wasserstoff und $R_3$ und $R_4$ jeweils $C_1$-$C_4$-Alkyl, $C_1$-$C_4$-Alkoxy, Methylthio, Halogen oder Alkoxyalkyl bedeuten.

15. Mittel gemäss Anspruch 14, dadurch gekennzeichnet, dass X für Sauerstoff oder Schwefel steht.

16. Mittel gemäss Anspruch 15, dadurch gekennzeichnet, dass A für $C_2$-$C_8$-Alkoxyalkyl, $C_2$-$C_4$-Halogenalkenyl oder $C_2$-$C_6$-Alkenyl steht.

17. Mittel gemäss Anspruch 16, dadurch gekennzeichnet, dass A für $C_2$-$C_8$-Alkoxyalkyl und $R_3$ und $R_4$ jeweils für Methyl, Aethyl, Chlor oder Methoxy stehen.

18. Mittel gemäss Anspruch 16, dadurch gekennzeichnet, dass A für $C_2$-$C_6$-Alkenyl und $R_3$ und $R_4$ jeweils für Methyl, Aethyl, Chlor oder Methoxy stehen.

19. Mittel gemäss Anspruch 16, dadurch gekennzeichnet, dass A für durch ein bis drei Halogenatome substituiertes Vinyl und $R_3$ und $R_4$ jeweils für Methyl, Aethyl, Chlor oder Methoxy stehen.

20. Mittel gemäss Anspruch 1, dadurch gekennzeichnet, dass Z Schwefel, X Sauerstoff, $R_3$ und $R_4$ jeweils unabhängig voneinander $C_1$-$C_3$-Alkyl, $C_1$-$C_3$-Alkoxy oder $C_1$-$C_3$-Alkoxy oder $C_1$-$C_3$-Alkylthio mit zusammen höchstens 4 Kohlenstoffatomen bedeuten und A für $—CH_2—CH = CH_2$, $—CH_2—CH = CH—CH_3$, $—CH_2—CH(CH_3) = CH_2$, Methoxyäthyl, Methoxymethyl oder $—CCl = CHCl$ steht und der Rest —X—A die 2-Stellung besetzt und m die Zahl 1 bedeutet.

21. Mittel gemäss Anspruch 1, dadurch gekennzeichnet, dass es als Wirkstoff N-(2-Allyloxyphenylsulfonyl)-N'-(4-methoxy-6-methyl-1,3,5-triazin-2-yl)-harnstoff enthält.

22. Verfahren zur Herstellung der Verbindungen der Formel I, gemäss Anspruch 1, dadurch gekennzeichnet, dass man entweder

a) ein Phenylsulfonamid der Formel II

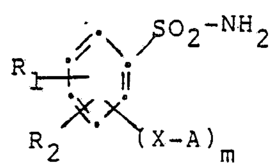

(II)

worin A, $R_1$, $R_2$, X und m die unter Formel I gegebene Bedeutung haben, in Gegenwart einer Base mit einem N-Pyrimidinyl- oder -Triazinylcarbamat der Formel

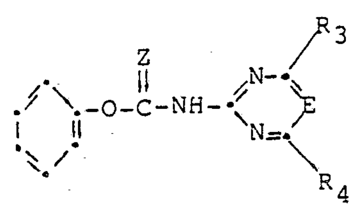

(III)

worin E, $R_3$, $R_4$ und Z die unter Formel I gegebene Bedeutung haben, umsetzt oder

b) ein Phenylsulfonylisocyanat oder -isothiocyanat der Formel IV

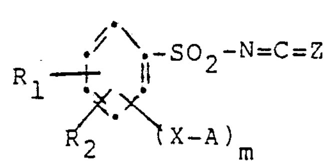

(IV)

worin A, $R_1$, $R_2$, m, X und Z die unter Formel I gegebene Bedeutung haben, gegebenenfalls in Gegenwart einer Base, mit einem Amin der Formel V

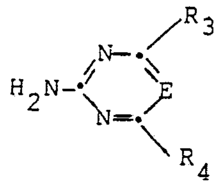

(V)

worin E, $R_3$ und $R_4$ die unter Formel I gegebene Bedeutung haben, umsetzt oder

c) ein Sulfonamid der oben angegebenen Formel II gegebenenfalls in Gegenwart einer Base mit einem Isocyanat oder Isothiocyanat der Formel VI

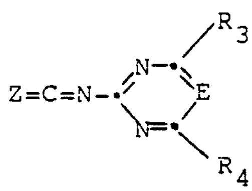

(VI)

worin E, $R_3$, $R_4$ und Z die unter Formel I gegebenen Bedeutung haben, umsetzt oder

d) ein N-Phenylsulfonylcarbamat der Formel VII

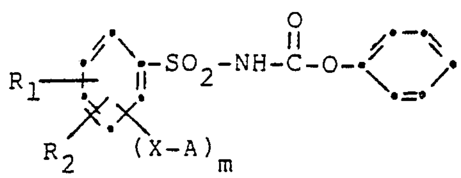

(VII)

worin A, $R_1$, $R_2$, m und X die unter Formel I gegebene Bedeutung haben, mit einem Amin der oben angegebenen Formel V umsetzt und gegebenenfalls in ein Salz überführt, indem man den Sulfonylharnstoff der Formel I mit einem Amin, einem Alkalimetall- oder Erdalkalimetallhydroxid oder einen quaternären Ammoniumbase umsetzt.

23. Verfahren zur Herstellung der Phenylsulfonamide der Formel II

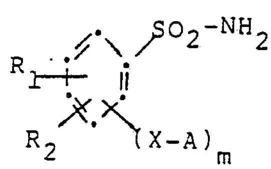

(II)

worin A, $R_1$, $R_2$, m und X die unter Formel I in Anspruch gegebene Bedeutung haben, dadurch gekennzeichnet, dass man in an sich bekannter Weise entsprechende Anilide durch Diazotierung und Austausch der Diazogruppe mit Schwefeldioxid in Gegenwart eines Katalysators in Salzsäure oder Essigsäure in die entsprechenden Phenylsulfonylchloride überführt und diese mit Ammoniumhydroxid-Lösung umsetzt.

24. Verfahren zur Herstellung der Phenylsulfonamide der Formel VIII

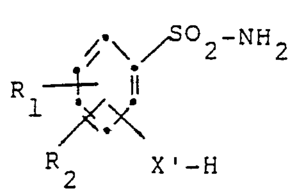

(VIII)

worin $R_1$ und $R_2$ die unter Formel I in Anspruch 1 gegebene Bedeutung haben und X für Sauerstoff oder Schwefel steht, mit der Massgabe, dass $R_2$ nicht Wasserstoff bedeutet und stets einer der Substituenten $R_2$ oder —X'—H in 2-Stellung zur Sulfonamidgruppe steht, dadurch gekennzeichnet, dass man in an sich bekannter Weise entsprechende $C_1$-$C_4$-Alkoxyphenylsulfonamide mittels einer Aetherspaltungsreaktion in die freien Hydroxyphenylsulfonamide überführt oder dass man in an sich bekannter Weise entsprechende Benzyloxyphenylsulfonamide mittels einer Hydrogenolysereaktion in die freien Hydroxyphenylsulfonamide überführt.

25. Ein herbizides und den Pflanzenwuchs hemmendes Mittel, dadurch gekennzeichnet, dass es neben Träger- und/oder anderen Zuschlagstoffen als Wirkstoff mindestens einen N-Phenylsulfonyl-N'-triazinyl- oder -pyrimidinyl-harnstoff der Formel I, Anspruch 1, enthält.

26. Die Verwendung der N-Phenylsulfonyl-N'-triazinyl- oder -pyrimidinyl-harnstoffe der Formel I, Anspruch 1, oder sie enthaltender Mittel zur Bekämpfung unerwünschten Pflanzenwachstums.

27. Die Verwendung der N-Phenylsulfonyl-N'-triazinyl- oder -pyrimidinyl-harnstoffe der Formel I, Anspruch 1, oder sie enthaltender Mittel zur Hemmung des Pflanzenwachstums.

28. Die Verwendung der N-Phenylsulfonyl-N'-triazinyl- oder -pyrimidinyl-harnstoffe der Formel I, oder sie enthaltender Mittel, gemäss Anspruch 26, zur selektiven pre- oder postemergenten Bekämpfung von Unkräutern in Nutzpflanzenkulturen.

29. Die Verwendung der N-Phenylsulfonyl-N'-triazinyl- oder -pyrimidinyl-harnstoffe der Formel I, oder sie enthaltender Mittel, gemäss Anspruch 27, zur Unterdrückung des Pflanzenwachstums über das 2-Blattstadium hinaus, dadurch gekennzeichnet, dass die Wirkstoffe preemergent angewendet werden.

30. Mittel gemäss Anspruch 1, dadurch gekennzeichnet, dass es als Wirkstoff N-(2-Allyloxy-5-methyl-phenylsulfonyl)-N'-(4-methoxy-6-methyl-1,3,5-triazin-2-yl)- harnstoff enthält.

31. Mittel gemäss Anspruch 1, dadurch gekennzeichnet, dass es als Wirkstoff N-[2-(2-Methallyloxy)-phenylsulfonyl]-N'-(4-methoxy-6-methyl-1,3,5-triazin-2-yl)-harnstoff enthält.

32. Mittel gemäss Anspruch 1, dadurch gekennzeichnet, dass es als Wirkstoff N-[2-(2-Methallyloxy)-phenylsulfonyl]-N'-(4,6-dimethoxy-1,3,5-triazin-2-yl)-harnstoff enthält.

33. Mittel gemäss Anspruch 1, dadurch gekennzeichnet, dass es als Wirkstoff N-[2-(2-Methallyloxy)-phenylsulfonyl]-N'-(4-äthyl-6-methoxy-1,3,5-triazin-2-yl)-harnstoff enthält.

34. Mittel gemäss Anspruch 1, dadurch gekennzeichnet, dass es als Wirkstoff N-[2-(2-Methallyloxy)-phenylsulfonyl]-N'-(4,6-diäthoxy-1,3,5-triazin-2-yl)-harnstoff enthält.

35. Mittel gemäss Anspruch 1, dadurch gekennzeichnet, dass es als Wirkstoff N-[2-(2-Butenyloxy)-phenylsulfonyl]-N'-(4-methoxy-6-methyl-1,3,5-triazin-2-yl)-harnstoff enthält.

36. Mittel gemäss Anspruch 1, dadurch gekennzeichnet, dass es als Wirkstoff N-[2-(2-Butenyloxy)-phenylsulfonyl]-N'-(4,6-dimethoxy-1,3,5-triazin-2-yl)-harnstoff enthält.

37. Mittel gemäss Anspruch 1, dadurch gekennzeichnet, dass es als Wirkstoff N-[2-(1,2-Dichlorvinyloxy)-phenylsulfonyl]-N'-(4-methoxy-6-methyl-1,3,5-triazin-2-yl)-harnstoff enthält.

38. Mittel gemäss Anspruch 1, dadurch gekennzeichnet, dass es als Wirkstoff N-(2-Allyloxy-phenylsulfonyl)-N'-(4,6-diäthoxy-1,3,5-2-yl)-harnstoff enthält.

39. Mittel gemäss Anspruch 1, dadurch gekennzeichnet, dass es als Wirkstoff N-(2-Allyloxy-phenylsulfonyl)-N'-(4-äthoxy-6-methyl-1,3,5-triazin-2-yl)-harnstoff enthält.

40. Mittel gemäss Anspruch 1, dadurch gekennzeichnet, dass es als Wirkstoff N-(2-Allyloxy-phenylsulfonyl)-N'-(4-methoxy-6-isopropyloxy-1,3,5-triazin-2-yl)-harnstoff enthält.

41. Mittel gemäss Anspruch 1, dadurch gekennzeichnet, dass es als Wirkstoff N-(2-Allyloxy-phenylsulfonyl)-N'-(4,6-dimethoxy-1,3,5-triazin-2-yl)-harnstoff enthält.

42. Mittel gemäss Anspruch 1, dadurch gekennzeichnet, dass es als Wirkstoff N-[2-(1,2-Dichlorvinyloxy)-phenylsulfonyl]-N'-(4,6-dimethoxy-1,3,5-triazin-2-yl)-harnstoff enthält.

43. Mittel gemäss Anspruch 1, dadurch gekennzeichnet, dass es als Wirkstoff N-[2-(1,2-Dichlorvinyloxy)-phenylsulfonyl]-N'-(4-chlor-6-methoxy-pyrimidin-2-yl)-harnstoff enthält.

44. Mittel gemäss Anspruch 1, dadurch gekennzeichnet, dass es als Wirkstoff N-(2-Methoxyäthoxy-phenylsulfonyl)-N'-(4-methoxy-6-methyl-1,3,5-triazin-2-yl)-harnstoff enthält.

45. Mittel gemäss Anspruch 1, dadurch gekennzeichnet, dass es als Wirkstoff N-(2-Methoxymethoxy-phenylsulfonyl)-N'-(4-methoxy-6-methyl-1,3,5-triazin-2-yl)-harnstoff enthält.

46. Mittel gemäss Anspruch 1, dadurch gekennzeichnet, dass es als Wirkstoff N-(2-Methoxyäthylthio-phenylsulfonyl)-N'-(4-methoxy-6-methyl-1,3,4-triazin-2-yl)-harnstoff enthält.

47. Mittel gemäss Anspruch 1, dadurch gekennzeichnet, dass es als Wirkstoff N-(2-Methoxyäthoxy-phenylsulfonyl)-N'-(4-chlor-6-methoxy-pyrimidin-2-yl)-harnstoff enthält.

48. Mittel gemäss Anspruch 1, dadurch gekennzeichnet, dass es als Wirkstoff N-(2-Methoxyäthoxy-phenylsulfonyl)-N'-(4-methoxy-6-methyl-pyrimidin-2-yl)-harnstoff enthält.

**Claims** (for the Contracting States : BE, DE, FR, GB, IT, NL, SE)

1. A N-phenylsulfonyl-N'-diazinyl- or -triazinylurea of the general formula I

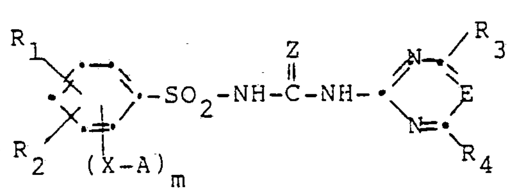

(I)

wherein

A is a $C_1$-$C_6$alkyl radical which is substituted by $C_1$-$C_4$alkoxy, $C_1$-$C_4$alkylthio, $C_1$-$C_4$alkylsulfinyl, $C_1$-$C_4$haloalkoxy, $C_1$-$C_4$haloalkylthio, $C_1$-$C_4$haloalkylsulfinyl or $C_1$-$C_4$haloalkylsulfonyl, or a $C_2$-$C_6$alkenyl radical which is unsubstituted or substituted by the above substituents, or is a $C_2$-$C_6$haloalkenyl radical,

E is the methine group or nitrogen,

X is oxygen, sulfur, a sulfinyl or sulfonyl bridge,

Z is oxygen or sulfur,

m is 1 or 2,

$R_1$ is hydrogen, halogen, $C_1$-$C_5$alkyl, $C_2$-$C_5$alkenyl or a radical —Y—$R_5$,

$R_2$ is hydrogen, halogen, $C_1$-$C_5$alkyl, $C_2$-$C_5$alkenyl, $C_1$-$C_4$-haloalkyl, or a radical —Y—$R_5$, —$COOR_6$, —$NO_2$ or —CO—$NR_7$—$R_8$,

$R_3$ and $R_4$, each independently of the other, are hydrogen, $C_1$-$C_4$alkyl, $C_1$-$C_4$alkoxy, $C_1$-$C_4$alkylthio, $C_1$-$C_4$haloalkyl, halogen or alkoxyalkyl of at most 4 carbon atoms,

$R_5$ and $R_6$, each independently of the other, are $C_1$-$C_5$alkyl, $C_2$-$C_5$alkenyl or $C_2$-$C_6$alkynyl,

$R_7$ and $R_8$, each independently of the other, are hydrogen, $C_1$-$C_5$alkyl, $C_2$-$C_5$alkenyl or $C_2$-$C_6$alkenyl, and

Y is oxygen, sulfur, a sulfinyl or sulfonyl bridge,

with the proviso that m is 2 if simultaneously X is sulfur, a sulfinyl or sulfonyl bridge and A is $C_3$-$C_4$alkenyl ; or a salt thereof.

2. A compound according to claim 1, wherein Z is oxygen.

3. A compound according to claim 1, wherein $R_3$ and $R_4$ together contain not more than 4 carbon atoms.

4. A compound according to claim 1, wherein Z is oxygen and m is 1.

5. A compound according to claim 1, wherein Z is oxygen and m is 2.

6. A compound according to claim 4, wherein the radical —X—A is in the 2- or 3-position relative to the sulfonyl group.

7. A compound according to claim 6, wherein the radical —X—A is in the 2-position.

8. A compound according to claim 5, wherein both radicals —X—A are in the 2- and 5-position relative to the sulfonyl group.

9. A compound according to claim 2, wherein only one radical —X—A is in the 2-position relative to the sulfonyl radical and $R_3$ and $R_4$ together contain not more than 4 carbon atoms.

10. A compound according to claim 2, wherein two radicals —X—A are in the 2- or 5-position relative to the sulfonyl radical and $R_3$ and $R_4$ together contain not more than 4 carbon atoms.

11. A compound according to claim 9, wherein $R_1$ is hydrogen and $R_2$ is in the 5- or 6-position relative to the sulfonyl group.

12. A compound according to claim 11, wherein $R_2$ is hydrogen, halogen, $C_1$-$C_4$alkoxy, nitro or —$COOR_6$.

13. A compound according to claim 12, wherein $R_2$ is hydrogen, fluorine, nitro or $C_1$-$C_4$alkoxy and each of $R_3$ and $R_4$ is hydrogen, $C_1$-$C_4$alkyl, $C_1$-$C_4$alkoxy, $C_1$-$C_4$alkylthio, halogen or alkoxyalkyl, and $R_3$ and $R_4$ together contain at most 4 carbon atoms.

14. A compound according to claim 13, wherein $R_2$ is hydrogen, and each of $R_3$ and $R_4$ is $C_1$-$C_4$alkyl, $C_1$-$C_4$alkoxy, methylthio, halogen, or alkoxyalkyl.

15. A compound according to claim 14, wherein X is oxygen or sulfur.

16. A compound according to claim 15, wherein A is $C_2$-$C_8$alkoxyalkyl, $C_2$-$C_4$haloalkenyl or $C_2$-$C_6$alkenyl.

17. A compound according to claim 16, wherein A is $C_2$-$C_8$alkoxyalkyl and each of $R_3$ and $R_4$ is methyl, ethyl, chlorine or methoxy.

18. A compound according to claim 16, wherein A is $C_2$-$C_6$alkenyl and each of $R_3$ and $R_4$ is methyl, ethyl, chlorine or methoxy.

19. A compound according to claim 16, wherein A is vinyl substituted by 1 to 3 halogen atoms, and each of $R_3$ and $R_4$ is methyl, ethyl, chlorine or methoxy.

20. A compound according to claim 1, wherein Z is sulfur, X is oxygen, each of $R_3$ and $R_4$ independently is $C_1$-$C_3$alkyl, $C_1$-$C_3$alkoxy or $C_1$-$C_3$alkylthio, containing together at most 4 carbon atoms, and A is $—CH_2—CH=CH_2$, $—CH_2—CH=CH—CH_3$, $—CH_2—CH(CH_3)=CH_2$, methoxyethyl, methoxymethyl or $—CCl=CHCl$, and the radical —X—A is in the 2-position and m is 1.

21. N-(2-Allyloxyphenylsulfonyl)-N'-(4-methoxy-6-methyl-1,3,5-triazin-2-yl)urea according to claim 1.

22. A process for the preparation of a compound of the formula I according to claim 1, which process comprises reacting a phenylsulfonamide of the formula II

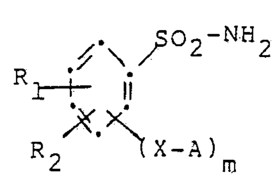

(II)

wherein A, $R_1$, $R_2$, X and m are as defined for formula I, in the presence of a base, with a N-pyrimidinyl- or N-triazinyl-carbamate of the formula III.

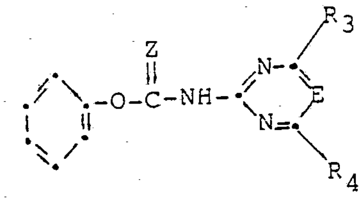

(III)

wherein E, $R_3$, $R_4$ and Z are as defined for formula I, and, if desired, converting the compound of formula I into a salt thereof.

23. A process for the preparation of a compound of the formula I according to claim 1, which process comprises reacting a phenylsulfonylisocyanate or phenylsulfonylisothiocyanate of the formula IV

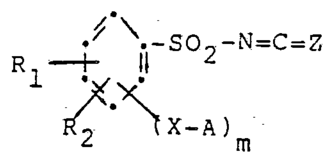

(IV)

wherein A, $R_1$, $R_2$, m, X and Z are as defined for formula I, optionally in the presence of a base, with an amine of the formula V

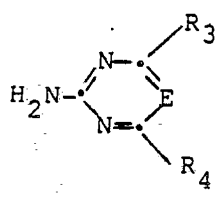

(V)

wherein E, $R_3$ and $R_4$ are as defined for formula I, and, if desired, converting the compound of formula I into a salt thereof.

24. A process for the preparation of a compound of the formula I according to claim 1, which process comprises reacting a sulfonamide of the formula II above, optionally in the presence of a base, with an isocyanate or isothiocyanate of the formula VI

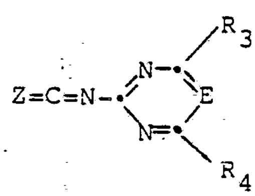

(VI)

wherein E, $R_3$, $R_4$ and Z are as defined for formula I, and, if desired, converting the compound of formula I into a salt thereof.

25. A process for the preparation of a compound of the formula I according to claim 1, which process comprises reacting a N-phenylsulfonylcarbamate of the formula VII

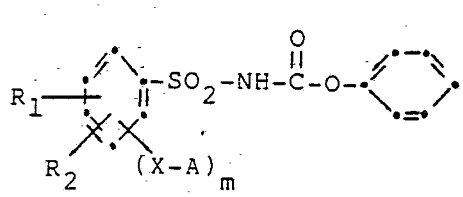

(VII)

wherein A, $R_1$, $R_2$, m and X are as defined for formula I, with an amine of the formula V above, and, if desired, converting the compound of formula I into a salt thereof.

26. A process for the preparation of a salt of the formula I according to any one of claims 22 to 25, which process comprises reacting a sulfonylurea of the formula I with an amine, an alkali metal hydroxide or alkaline earth metal hydroxide or a quaternary ammonium base.

27. A phenylsulfonamide of the formula II

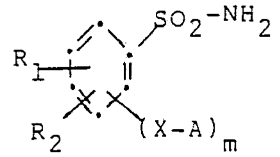

(II)

wherein A, $R_1$, $R_2$, m and X are as defined for formula I in claim 1.

28. A phenylsulfonamide of the formula VIII

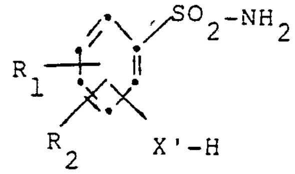

(VIII)

wherein $R_1$ and $R_2$ are as defined for formula I in claim 1, and X is oxygen or sulfur, with the proviso that $R_2$ is not hydrogen and one of the substituents $R_2$ or —X'—H is always in the 2-position relative to the sulfonamido group.

29. A herbicidal and plant growth regulating composition which contains at least one N-phenylsulfonyl-N'-triazinyl- or -N'-pyrimidinylurea of the formula I as claimed in claim 1, together with carriers and/or other adjuvants.

30. A method of controlling undesired plant growth, which comprises the use of a N-phenylsulfonyl-N'-triazinylurea or N-phenylsulfonyl-N'-pyrimidinylurea of the formula I according to claim 1, or of a composition containing such a compound.

31. A method of inhibiting plant growth, which comprises the use of a N-phenylsulfonyl-N'-triazinylurea or N-phenylsulfonyl-N'-pyrimidinylurea of the formula I according to claim 1, or of a composition containing such a compound.

32. A method according to claim 30 of selectively controlling weeds pre- or postemergence in crops of useful plants, which method comprises the use of a N-phenylsulfonyl-N'-triazinylurea or n-phenylsulfonyl-N'-pyrimidinylurea of the formula I, or of a composition containing such a compound.

33. A method according to claim 31 of inhibiting plant growth beyond the 2-leaf stage preemergence, which method comprises the use of a N-phenylsulfonyl-N'-triazinylurea or N-phenylsulfonyl-N'-pyrimidinylurea of the formula I, or of a composition containing such a compound.

34. N-(2-Allyloxy-5-methylphenylsylfonyl)-N'-(4-methoxy-6-methyl-1,3,5-triazin-2-yl)urea according to claim 1.

35. N-[2-(2-Methallyloxy)phenylsulfonyl]-N'-(4-methoxy-6-methyl-1,3,5-triazin-2-yl)urea according to claim 1.

36. N-[2-(2-Methallyloxy)phenylsulfonyl]-N'-(4,6-dimethoxy-1,3,5-triazin-2-yl)urea according to claim 1.

37. N-[2-(2-Methallyloxy)phenylsulfonyl]-N'-(4-ethyl-6-methoxy-1,3,5-triazin-2-yl)urea according to claim 1.

38. N-[2-(2-Methallyloxy)phenylsulfonyl]-N'-(4,6-diethoxy-1,3,5-triazin-2-yl)urea according to claim 1.

39. N-[2-(2-Butenyloxy)phenylsulfonyl]-N'-(4-methoxy-6-methyl-1,3,5-triazin-2-yl)urea according to claim 1.

40. N-[2-(2-Butenyloxy)phenylsulfonyl]-N'-(4,6-dimethoxy-1,3,5-triazin-2-yl)urea according to claim 1.

41. N-[2-(1,2-Dichlorovinyloxy)phenylsulfonyl]-N'-(4-methoxy-6-methyl-1,3,5-triazin-2-yl)urea according to claim 1.

42. N-(2-Allyloxyphenylsulfonyl)-N'-(4,6-diethoxy-1,3,5-triazin-2-yl)urea according to claim 1.

43. N-(2-Allyloxyphenylsulfonyl)-N'-(4-ethoxy-6-methyl-1,3,5-triazin-2-yl)urea according to claim 1.

44. N-(2-Allyloxyphenylsulfonyl)-N'-(4-methoxy-6-isopropoxy-1,3,5-triazin-2-yl)urea according to claim 1.

45. N-(2-Allyloxyphenylsulfonyl)-N'-(4,6-dimethoxy-1,3,5-triazin-2-yl)urea according to claim 1.

46. N-[2-(1,2-Dichlorovinyloxy)phenylsulfonyl]-N'-4,6-dimethoxy-1,3,5-triazin-2-yl)urea according to claim 1.

47. N-[2-(1,2-Dichlorovinyloxy)phenylsulfonyl]-N'-(4-chloro-6-methoxypyrimidin-2-yl)urea according to claim 1.

48. N-(2-Methoxyethoxyphenylsulfonyl)-N'-(4-methoxy-6-methyl-1,3,5-triazin-2-yl)urea according to claim 1.

49. N-(2-Methoxymethoxyphenylsulfonyl)-N'-(4-methoxy-6-methyl-1,3,5-triazin-2-yl)urea according to claim 1.

50. N-(2-Methoxyethylthiophenylsulfonyl)-N'-(4-methoxy-6-methyl-1,3,5-triazin-2-yl)urea according to claim 1.

51. N-(2-Methoxyethoxyphenylsulfonyl)-N'-(4-chloro-6-methoxypyrimidin-2-yl)urea according to claim 1.

52. N-[2-Methoxyethoxyphenylsulfonyl)-N'-(4-methoxy-6-methylpyrimidin-2-yl)urea according to claim 1.

**Claims** (for the Contracting State AT)

1. A herbicidal and plant growth regulating composition which contains at least one N-phenylsulfonyl-N'-diazinyl- or -triazinylurea of the general formula I

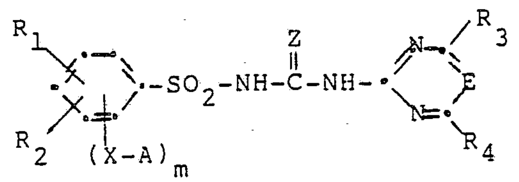

(I)

wherein

A is a $C_1$-$C_6$alkyl radical which is substituted by $C_1$-$C_4$alkoxy, $C_1$-$C_4$alkylthio, $C_1$-$C_4$alkylsulfinyl, $C_1$-$C_4$-alkylsulfonyl, $C_1$-$C_4$haloalkoxy, $C_1$-$C_4$haloalkylthio, $C_1$-$C_4$haloalkylsulfinyl or $C_1$-$C_4$haloalkylsulfonyl, or a $C_2$-$C_6$-alkenyl radical which is unsubstituted or substituted by the above substituents, or is a $C_2$-$C_6$haloalkenyl radical,

E is the methine group or nitrogen,

X is oxygen, sulfur, a sulfinyl or sulfonyl bridge,

Z is oxygen or sulfur,

m is 1 or 2

$R_1$ is hydrogen, halogen, $C_1$-$C_5$alkyl, $C_2$-$C_5$alkenyl or a radical —Y—$R_5$,

$R_2$ is hydrogen, halogen, $C_1$-$C_5$alkyl, $C_2$-$C_5$alkenyl, $C_1$-$C_4$-haloalkyl, or a radical —Y—$R_5$, —$COOR_6$, —$NO_2$ or —CO—$NR_7$—$R_8$,

$R_3$ and $R_4$, each independently of the other, are hydrogen, $C_1$-$C_4$alkyl, $C_1$-$C_4$alkoxy, $C_1$-$C_4$alkylthio, $C_1$-$C_4$haloalkyl, halogen or alkoxyalkyl of at most 4 carbon atoms,

$R_5$ and $R_6$, each independently of the other, are $C_1$-$C_5$alkyl, $C_2$-$C_5$alkenyl or $C_2$-$C_6$alkynyl,

$R_7$ and $R_8$, each independently of the other, are hydrogen, $C_1$-$C_5$alkyl, $C_2$-$C_5$alkenyl or $C_2$-$C_6$alkynyl, and

Y is oxygen, sulfur, a sulfinyl or sulfonyl bridge,

with the proviso that m is 2 if simultaneously X is sulfur, a sulfinyl or sulfonyl bridge and A is $C_3$-$C_4$alkenyl ; or a salt thereof, together with carriers and/or other adjuvants.

2. A composition according to claim 1, wherein Z is oxygen.

3. A composition according to claim 1, wherein $R_3$ and $R_4$ together contain not more than 4 carbon atoms.

4. A composition according to claim 1, wherein Z is oxygen and m is 1.

5. A composition according to claim 1, wherein Z is oxygen and m is 2.

6. A composition according to claim 4, wherein the radical —X—A is in the 2- or 3-position to the sulfonyl group.

7. A composition according to claim 6, wherein the radical —X—A is in the 2-position.

8. A composition according to claim 5, wherein both radicals —X—A are in the 2- and 5-position to the sulfonyl group.

9. A composition according to claim 2, wherein only one radical —X—A is in the 2-position to the sulfonyl radical and $R_3$ and $R_4$ together contain not more than 4 carbon atoms.

10. A composition according to claim 2, wherein two radicals —X—A are in the 2- and 5-position to the sulfonyl radical and $R_3$ and $R_4$ together contain not more than 4 carbon atoms.

11. A composition according to claim 9, wherein $R_1$ is hydrogen and $R_2$ is in the 5- or 6-position to the sulfonyl group.

12. A composition according to claim 11, wherein $R_2$ is hydrogen, halogen, $C_1$-$C_4$alkoxy, nitro or —$COOR_6$.

13. A composition according to claim 12, wherein $R_2$ is hydrogen, fluorine, nitro or $C_1$-$C_4$alkoxy and each of $R_3$ and $R_4$ is hydrogen, $C_1$-$C_4$alkyl, $C_1$-$C_4$alkoxy, $C_1$-$C_4$alkylthio, halogen or alkoxyalkyl, whilst $R_3$ and $R_4$ together contain at most 4 carbon atoms.

14. A composition according to claim 13, wherein $R_2$ is hydrogen, and each of $R_3$ and $R_4$ is $C_1$-$C_4$alkyl, $C_1$-$C_4$alkoxy, methylthio, halogen, or alkoxyalkyl.

15. A composition according to claim 14, wherein X is oxygen or sulfur.

16. A composition according to claim 15, wherein A is $C_2$-$C_8$alkoxyalkyl, $C_2$-$C_4$haloalkenyl or $C_2$-$C_6$alkenyl.

17. A composition according to claim 16, wherein A is $C_2$-$C_8$alkoxyalkyl and each of $R_3$ and $R_4$ is methyl, ethyl, chlorine or methoxy.

18. A composition according to claim 16, wherein A is $C_2$-$C_6$alkenyl and each of $R_3$ and $R_4$ is methyl, ethyl, chlorine or methoxy.

19. A composition according to claim 16, wherein A is vinyl substituted by 1 to 3 halogen atoms, and each of $R_3$ and $R_4$ is methyl, ethyl, chlorine or methoxy.

20. A composition according to claim 1, wherein Z is sulfur, X is oxygen, each of $R_3$ and $R_4$ independently is $C_1$-$C_3$alkyl, $C_1$-$C_3$alkoxy or $C_1$-$C_3$alkylthio, containing together at most 4 carbon atoms, and A is $-CH_2-CH=CH_2$, $-CH_2-CH=CH-CH_3$, $-CH_2-CH(CH_3)=CH_2$, methoxyethyl, methoxymethyl or $-CCl=CHCl$, and the radical —X—A is in the 2-position and m is 1.

21. A composition according to claim 1, which contains N-(2-allyloxyphenylsulfonyl)-N'-(4-methoxy-6-methyl-1,3,5-triazin-2-yl)urea.

22. A process for the preparation of a compound of the formula I according to claim 1, which process comprises reacting either

a) a phenylsulfonamide of the formula II

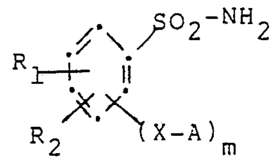

(II)

wherein A, $R_1$, $R_2$, X and m are as defined for formula I, in the presence of a base, with a N-pyrimidinyl- or N-triazinyl-carbamate of the formula III

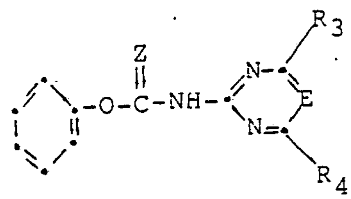

(III)

wherein E, $R_3$, $R_4$ and Z are as defined for formula I, or

b) reacting a phenylsulfonylisocyanate or phenylsulfonylisothiocyanate of the formula IV

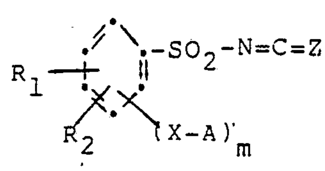

(IV)

wherein A, $R_1$, $R_2$, m, X and Z are as defined for formula I, optionally in the presence of a base, with an amine of the formula V

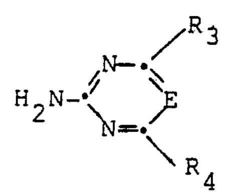

(V)

wherein E, $R_3$ and $R_4$ are as defined for formula I, or

c) reacting a sulfonamide of the formula II above, optionally in the presence of a base, with an isocyanate or isothiocyanate of the formula VI

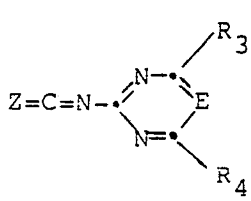

(VI)

wherein E, $R_3$, $R_4$ and Z are as defined for formula I, or

d) reacting a N-phenylsulfonylcarbamate of the formula

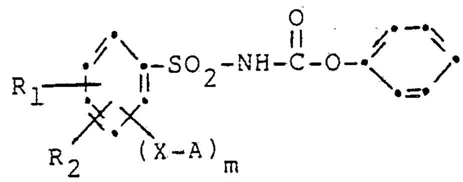

(VII)

wherein A, $R_1$, $R_2$, m and X are as defined for formula I, with an amine of the formula V above, and, if desired, converting said compound of formula I into a salt thereof, by reacting the sulfonyl urea of the formula I with an amine, an alkali metal hydroxide or alkaline earth metal hydroxide or a quaternary ammonium base.

23. A process for the preparation of a phenylsulfonamide of the formula II

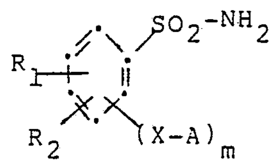

(II)

wherein A, $R_1$, $R_2$, m and X are as defined for formula I in claim 1, which process comprises converting, in a manner known per se, a corresponding anilide into the phenylsulfonyl chloride by diazotisation and replacement of the diazo group with sulfur dioxide, in the presence of a catalyst and in hydrochloric acid or acetic acid, and reacting said phenylsulfonyl chloride with ammonium hydroxide solution.

24. A process for the preparation of a phenylsulfonamide of the formula VIII

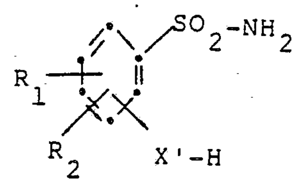

(VIII)

wherein $R_1$ and $R_2$ are as defined for formula I in claim 1 and X is oxygen or sulfur, with the proviso that $R_2$ is not hydrogen and one of the substituents $R_2$ or —X'—H is always in the 2-position relative to the sulfonamido group, which process comprises converting, in a manner known per se, a corresponding $C_1$-$C_4$alkoxyphenylsulfonamide into the free hydroxyphenylsulfonamide by an ether cleavage reaction, or converting, in a manner known per se, a corresponding benzyloxyphenylsulfonamide into the free hydroxyphenylsulfonamide by hydrogenolysis.

25. A herbicidal and plant growth regulating composition which contains at least one N-phenylsulfonyl-N'-triazinyl- or -N'-pyrimidinylurea of the formula I as claimed in claim 1, together with carriers and/or other adjuvants.

26. A method of controlling undesired plant growth, which comprises the use of a N-phenylsulfonyl-N'-triazinylurea or N-phenylsulfonyl-N'-pyrimidinylurea of the formula I according to claim 1, or of a composition containing such a compound.

27. A method of inhibiting plant growth, which comprises the use of a N-phenylsulfonyl-N'-triazinylurea or N-phenylsulfonyl-N'-pyrimidinylurea of the formula I according to claim 1, or of a composition containing such a compound.

28. A method according to claim 26 of selectively controlling weeds pre- or postemergence in crops of useful plants, which method comprises the use of a N-phenylsulfonyl-N'-triazinylurea or n-phenylsulfonyl-N'-pyrimidinylurea of the formula I, or of a composition containing such a compound.

29. A method according to claim 27 of inhibiting plant growth beyond the 2-leaf stage preemergence, which method comprises the use of a N-phenylsulfonyl-N'-triazinylurea or N-phenylsulfonyl-N'-pyrimidinylurea of the formula I, or of a composition containing such a compound.

30. A composition according to claim 1, which contains N-(2-allyloxy-5-methylphenylsulfonyl)-N'-(4-methoxy-6-methyl-1,4,5-triazin-2-yl)urea.

31. A composition according to claim 1, which contains N-[2-(2-methallyloxy)phenylsulfonyl]-N'-(4-methoxy-6-methyl-1,3,5-triazin-2-yl)urea.

32. A composition according to claim 1, which contains N-[2-(2-)methallyloxy)phenylsulfonyo-]-N'-(4,6-dimethoxy-1,3-5-triazin-2-yl)urea.

33. A composition according to claim 1, which contains N-[2(2-methallyloxy)phenylsulfonyl]-N'-(4-ethyl-6-methoxy-1,3,5-triazin-2-yl)urea.

34. A composition according to claim 1, which contains N-[2-(2-methallyloxy)phenylsulfonyl]-N'-(4,6-diethoxy-1,3,5-triazin-2-yl)urea.

35. A composition according to claim 1, which contains N-[2-(2-butenyloxy)phenylsulfonyl]-N'-(4-methoxy-6-methyl-1,3,5-triazin-2-yl)urea.

36. A composition according to claim 1, which contains N-[2-(2-butenyloxy)phenylsulfonyl]-N'-(4,6-dimethoxy-1,3,5-triazin-2-yl)urea.

37. A composition according to claim 1, which contains N-[2-(1,2-dichlorovinyloxy)phenylsulfonyl]-N'-(4-methoxy-6-methyl-1,3,5-triazin-2-yl)urea.

38. A composition according to claim 1, which contains N-(2-allyloxyphenylsulfonyl)-N'-(4,6-diethoxy-1,3,5-triazin-2-yl)urea.

39. A composition according to claim 1, which contains N-(2-allyloxyphenylsulfonyl)-N'-(4-ethoxy-6-methyl-1,3,5-triazin-2-yl)urea..

40. A composition according to claim 1, which contains N-(2-allyloxyphenylsulfonyl)-N'-(4-methoxy-6-isopropoxy-1,3,5-triazin-2-yl)urea.

41. A composition according to claim 1, which contains N-(2-allyloxyphenylsulfonyl)-N'-(4,6-dimethoxy-1,3,5-triazin-2-yl)-urea.

42. A composition according to claim 1, which contains N-[2-(1,2-dichlorovinyloxy)phenylsulfonyl]-N'-(4,6-dimethoxy-1,3,5-triazin-2-yl)urea.

43. A composition according to claim 1, which contains N-[2-(1,2-dichlorovinyloxy)phenylsulfonyl]-N'-(4-chloro-6-methoxypyrimidin-2-yl)urea.

44. A composition according to claim 1, which contains N-(2-methoxyethoxyphenylsulfonyl)-N'-(4-methoxy-6-methyl-1,3,5-triazin-2-yl)urea.

45. A composition according to claim 1, which contains N-(2-methoxymethoxyphenylsulfonyl)-N'-(4-methoxy-6-methyl-1,3,5-triazin-2-yl)urea.

46. A composition according to claim 1, which contains N-(2-methoxyethylthiophenylsulfonyl)-N'-(4-methoxy-6-methyl-1,3,5-triazin-2-yl)urea.

47. A composition according to claim 1, which contains N-(2-methoxyethoxyphenylsulfonyl)-N'-(4-chloro-6-methoxy-pyrimidin-2-yl)urea.

48. A composition according to claim 1, which contains N-(2-methoxyethoxyphenylsulfonyl)-N'-(4-methoxy-6-methyl-pyrimidin-2-yl)urea.

**Revendications** (pour les Etats contractants : BE, DE, FR, GB, IT, NL, SE)

1. N-phénylsulfonyl-N'-diazinyl- et -triazinyl-urées de formule générale I

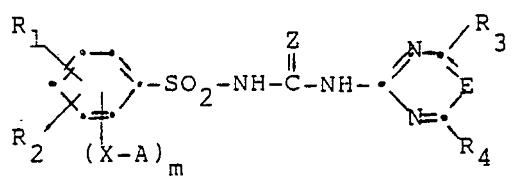

(I)

dans laquelle

A représente un groupe alkyle en C1-C6 substitué par des groupes alcoxy en C1-C4, alkylthio en C1-C4, alkylsulfinyle en C1-C4, alkylsulfonyle en C1-C4, halogénoalcoxy en C1-C4, halogénoalkylthio en C1-C4, halogénoalkylsulfinyle en C1-C4 ou halogénoalkylsulfonyle en C1-C4, ou un groupe alcényle en C2-C6 portant éventuellement les substituants énumérés ou un groupe halogénoalcényle en C2-C6,

E représente le groupe méthine ou l'azote,

X représente l'oxygène, le soufre, un pont sulfinyle ou sulfonyle,

Z représente l'oxygène ou le soufre,

m est égal à 1 ou 2,

$R_1$ représente l'hydrogène, un halogène, un groupe alkyle en C1-C5, alcényle en C2-C5 ou $-Y-R_5$,

$R_2$ représente l'hydrogène, un halogène, un groupe alkyle en C1-C5, alcényle en C2-C5, halogénoalkyle en C1-C4 ou un reste $-Y-R_5$, $-COOR_6$, $-NO_2$ ou $-CO-NR_7R_8$,

$R_3$ et $R_4$ représentent, indépendamment l'un de l'autre, l'hydrogène, un groupe alkyle en C1-C4, alcoxy en C1-C4, alkylthio en C1-C4, halogénoalkyle en C1-C4, un halogène ou un groupe alcoxyalkyle contenant au maximum 4 atomes de carbone,

$R_5$ et $R_6$ représentent chacun un groupe alkyle en C1-C5, alcényle en C2-C5 ou alcynyle en C2-C6,

$R_7$ et $R_8$ représentent chacun, indépendamment l'un de l'autre, l'hydrogène, un groupe alkyle en C1-C5, alcényle en C2-C5 ou alcynyle en C2-C6 et

Y représente l'oxygène, le soufre, un pont sulfinyle ou sulfonyle,

étant spécifié que m est égal à 2, lorsque, simultanément, X représente le soufre, un pont sulfinyle ou sulfonyle et A représente un groupe alcényle en C3-C4, ainsi que les sels de ces composés.

2. Composés selon la revendication 1, caractérisés en ce que Z représente l'oxygène.

3. Composés selon la revendication 1, caractérisés en ce que les groupes $R_3$ et $R_4$, ensemble, ne contiennent pas plus de 4 atomes de carbone.

4. Composés selon la revendication 1, caractérisés en ce que Z représente l'oxygène et m est égal à 1.

5. Composés selon la revendication 1, caractérisés en ce que Z représente l'oxygène et m est égal à 2.

6. Composés selon la revendication 4, caractérisés en ce que le reste —X—A est en position 2 ou 3 par rapport au groupe sulfonyle.

7. Composés selon la revendication 6, caractérisés en ce que le reste —X—A est en position 2.

8. Composés selon la revendication 5, caractérisés en ce que les deux restes —X—A sont dans les positions 2 et 5 par rapport au groupe sulfonyle.

9. Composés selon la revendication 2, caractérisés en ce qu'il n'y a qu'un seul reste —X—A en position 2 par rapport au groupe sulfonyle et en ce que les restes $R_3$ et $R_4$, ensemble, ne contiennent pas plus de 4 atomes de carbone.

10. Composés selon la revendication 2, caractérisés en ce qu'il y a deux restes —X—A dans les positions 2 et 5 par rapport au groupe sulfonyle et en ce que les restes $R_3$ et $R_4$, ensemble, ne contiennent pas plus de 4 atomes de carbone.

11. Composés selon la revendication 9, caractérisés en ce que $R_1$ représente l'hydrogène et en ce que le reste $R_2$ est en position 5 ou 6 par rapport au groupe sulfonyle.

12. Composés selon la revendication 11, caractérisés en ce que $R_2$ représente l'hydrogène, un halogène, un groupe alcoxy en C1-C4, nitro ou $—COOR_6$.

13. Composés selon la revendication 12, caractérisés en ce que $R_2$ représente l'hydrogène, le fluor, un groupe nitro ou alcoxy en C1-C4 et $R_3$ et $R_4$ représentent chacun l'hydrogène, un groupe alkyle en C1-C4, alcoxy en C1-C4, alkylthio en C1-C4, un halogène ou un groupe alcoxyalkyle, et $R_3$ et $R_4$, ensemble, contiennent au maximum 4 atomes de carbone.

14. Composés selon la revendication 13, caractérisés en ce que $R_2$ représente l'hydrogène et $R_3$ et $R_4$ représentent chacun un groupe alkyle en C1-C4, alcoxy en C1-C4, méthylthio, un halogène ou un groupe alcoxyalkyle.

15. Composés selon la revendication 14, caractérisés en ce que X représente l'oxygène ou le soufre.

16. Composés selon la revendication 15, caractérisés en ce que A représente un groupe alcoxyalkyle en C2-C8, halogénoalcényle en C2-C4 ou alcényle en C2-C6.

17. Composés selon la revendication 16, caractérisés en ce que A représente un groupe alcoxyalkyle en C2-C8 et $R_3$ et $R_4$ représentent chacun un groupe méthyle, éthyle, le chlore ou un groupe méthoxy.

18. Composés selon la revendication 16, caractérisés en ce que A représente un groupe alcényle en C2-C6 et $R_3$ et $R_4$ représentent chacun un groupe méthyle, éthyle, le chlore ou un groupe méthoxy.

19. Composés selon la revendication 16, caractérisés en ce que A représente un groupe vinyle substitué par un à trois atomes d'halogènes et $R_3$ et $R_4$ représentent chacun un groupe méthyle, éthyle, le chlore ou un groupe méthoxy.

20. Composés selon la revendication 1, caractérisés en ce que Z représente le soufre, X l'oxygène, $R_3$ et $R_4$ représentent chacun, indépendamment l'un de l'autre, un groupe alkyle en C1-C3, alcoxy en C1-C3 ou alkylthio en C1-C3 contenant ensemble au maximum 4 atomes de carbone, et A représente un groupe $—CH_2—CH=CH_2$, $—CH_2—CH=CH—CH_3$, $—CH_2—CH(CH_3)=CH_2$, méthoxyéthyle, méthoxyméthyle ou $—CCl=CHCl$, le reste —X—A occupe la position 2 et m est égal à 1.

21. La N-(2-allyloxyphénylsulfonyl)-N'-(4-méthoxy-6-Méthyl-1,3,5-triazine-2-l)urée selon la revendication 1.

22. Procédé de préparation des composés de formule I, revendication 1, caractérisé en ce que l'on fait réagir un phénylsulfonamide de formule II

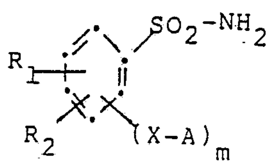

(II)

dans laquelle A, $R_1$, $R_2$, X et m ont les significations indiquées en référence à la formule I, en présence d'une base, avec un N-pyrimidinyl- ou -triazinyl-carbamate de formule

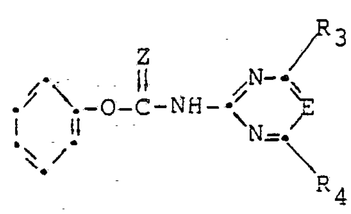

(III)

dans laquelle E, $R_3$, $R_4$ et Z ont les significations indiquées en référence à la formule I, et on convertit le cas échéant le composé obtenu en ses sels.

23. Procédé de préparation des composés de formule I, revendication 1, caractérisé en ce que l'on

fait réagir un phénylsulfonylisocyanate- ou -isothiocyanate de formule IV

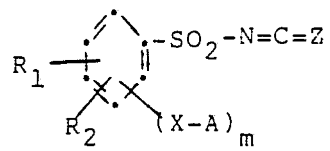

(IV)

dans laquelle A, $R_1$, $R_2$, m, X et Z ont les significations indiquées en référence à la formule I, éventuellement en présence d'une base, avec une amine de formule V

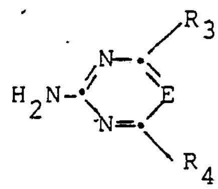

(V)

dans laquelle E, $R_3$ et $R_4$ ont les significations indiquées en référence à la formule I, et on convertit éventuellement le composé obtenu en ses sels.

24. Procédé de préparation des composés de formule I, revendication 1, caractérisé en ce que l'on fait réagir un sulfonamide de formule II ci-dessus, éventuellement en présence d'une base, avec un isocyanate ou isothiocyanate de formule VI

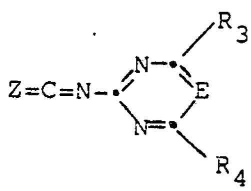

(VI)

dans laquelle E, $R_3$, $R_4$ et Z ont les significations indiquées en référence à la formule I, et on convertit éventuellement le composé obtenu en ses sels.

25. Procédé de préparation des composés de formule I, revendication 1, caractérisé en ce que l'on fait réagir un N-phénylsulfonylcarbamate de formule VII

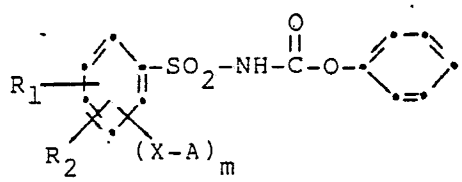

(VII)

dans laquelle A, $R_1$, $R_2$, m et X ont les significations indiquées en référence à la formule I, avec une amine de formule V ci-dessus, et on convertit éventuellement le composé obtenu en ses sels.

26. Procédé de préparation des sels de formule I selon l'une des revendications 22 à 25, caractérisé en ce que l'on fait réagir une sulfonylurée de formule I avec une amine, un hydroxyde de métal alcalin ou alcalino-terreux ou une base d'ammonium quaternaire.

27. Phénylsulfonamides de formule II

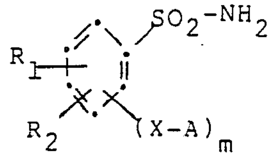

(II)

dans laquelle A, $R_1$, $R_2$, m et X ont les significations indiquées en référence à la formule I dans la revendication 1.

28. Phénylsulfonamides de formule VIII

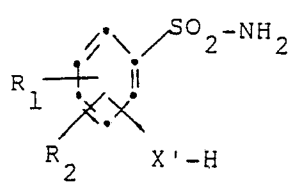

(VIII)

dans laquelle $R_1$ et $R_2$ ont les significations indiquées en référence à la formule I dans la revendication 1 et X représente l'oxygène ou le soufre, étant spécifié que $R_2$ ne peut représenter l'hydrogène et que l'un des substituants $R_2$ ou —X'—H est toujours en position 2 par rapport au groupe sulfonamide.

29. Un agent herbicide et inhibiteur de la croissance des végétaux, caractérisé en ce qu'il contient, en tant que substance active, avec des véhicules et/ou d'autres additifs, au moins une N-phénylsulfonyl-N'-triazinyl- ou -pyrimidinyl-urée de formule I, revendication 1.

30. L'utilisation des N-phénylsulfonyl-N'-triazinyl- ou -pyrimidinyl-urées de formule I, revendication 1, ou de produits en contenant, dans la lutte contre les croissances de végétaux indésirables.

31. L'utilisation des N-phénylsulfonyl-N'-triazinyl- ou -pyrimidinyl-urées de formule I, revendication 1, ou de produits en contenant, pour l'inhibition de la croissance des végétaux.

32. L'utilisation des N-phénylsulfonyl-N'-triazinyl- ou -pyrimidinyl-urées de formule I ou de produits en contenant, selon la revendication 30, pour la lutte sélective en pré-levée ou en post-levée contre les mauvaises herbes dans les cultures de végétaux utiles.

33. L'utilisation des N-phénylsulfonyl-N'-triazinyl- ou -pyrimidinyl-urées de formule I ou de produits en contenant, selon la revendication 31, pour l'inhibition de la croissance des végétaux au-delà du stade deux feuilles, caractérisée en ce que l'on applique les substances actives en pré-levée.

34. La N-(2-allyloxy-5-méthyl-phénylsulfonyl)-N'-(4-méthoxy-6-méthyl-1,3,5-triazine-2-yl)-urée selon la revendication 1.

35. La N-[2-(2-méthallyloxy)-phénylsulfonyl]-N'-(4-méthoxy-6-méthyl-1,3,5-triazine-2-yl)-urée selon la revendication 1.

36. La N-[2-(2-méthallyloxy)-phénylsulfonyl]-N'-(4,6-diméthoxy-1,3,5-triazine-2-yl)-urée, selon la revendication 1.

37. La N-[2-(2-méthallyloxy)-phénylsulfonyl]-N'-(4-éthyl-6-méthoxy-1,3,5-triazine-2-yl)-urée, selon la revendication 1.

38. La N-[2-(2-méthallyloxy)-phénylsulfonyl]-N'-(4,6-diéthoxy-1,3,5-triazine-2-yl)-urée, selon la revendication 1.

39. La N-[2-(2-buténylоxy)-phénylsulfonyl]-N'-(4-méthoxy-6-méthyl-1,3,5-triazine-2-yl)urée, selon la revendication 1.

40. La N-[2-(2-buténylоxy)-phénylsulfonyl]-N'-(4,6-diméthoxy-1,3,5-triazine-2-yl)urée, selon la revendication 1.

41. La N-[2-(1,2-dichlorovinyloxy)-phénylsulfonyl]-N'-(4'-méthoxy-6-méthyl-1,3,5-triazine-2-yl)-urée, selon la revendication 1.

42. La N-(2-allyloxy-phénylsulfonyl)-N'-(4,6-diéthoxy-1,3,5-triazine-2-yl)-urée, selon la revendication 1.

43. La N-(2-allyloxy-phénylsulfonyl)-N'-(4-éthoxy-6-méthyl-1,3,5-triazine-2-yl)-urée, selon la revendication 1.

44. La N-(2-allyloxy-phénylsulfonyl)-N'-(4-méthoxy-6-isopropyloxy-1,3,5-triazine-2-yl)-urée, selon la revendication 1.

45. La N-(2-allyloxy-phénylsulfonyl)-N'-(4,6-diméthoxy-1,3,5-triazine-2-yl)-urée, selon la revendication 1.

46. La N-[2-(1,3-dichlorovinyloxy)-phénylsulfonyl]-N'-(4,6-diméthoxy-1,3,5-triazine-2-yl)-urée, selon la revendication 1.

47. La N-[2-(1,2-dichlorovinyloxy)-phénylsulfonyl]-N'-(4-chloro-6-méthoxy-pyrimidine-2-yl)-urée, selon la revendication 1.

48. La N-(2-méthoxyéthoxy-phénylsulfonyl)-N'-(4-méthoxy-6-méthyl-1,3,5-triazine-2-yl)-urée, selon la revendication 1.

49. La N-(2-méthoxyméthoxy-phénylsulfonyl)-N'-(4-méthoxy-6-méthyl-1,3,5-triazine-2-yl)-urée, selon la revendication 1.

50. La N-(2-méthoxyéthylthio-phénylsulfonyl)-N'-(4-méthoxy-6-méthyl-1,3,5-triazine-2-yl)-urée, selon la revendication 1.

51. La N-(2-méthoxyéthoxy-phénylsulfonyl)-N'-(4-chloro-6-méthoxy-pyrimidine-2-yl)-urée, selon la revendication 1.

52. La N-(2-méthoxyéthoxy-phénylsulfonyl)-N'-(4-méthoxy-6-méthyl-pyrimidine-2-yl)-urée, selon la revendication 1.

**Revendications** (pour l'Etat contractant AT)

1. Un produit herbicide et inhibiteur de la croissance des végétaux, caractérisé en ce qu'il contient en tant que substance active, avec des véhicules et/ou d'autres additifs, au moins une N-phénylsulfonyl-N'-diazinyl- ou -triazinyl-urée de formule générale I

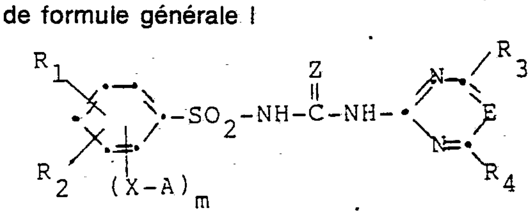

(I)

ou un sel de ces composés,
dans lesquels

A représente un groupe alkyle en C1-C6 substitué par des groupes alcoxy en C1-C4, alkylthio en C1-C4, alkylsulfinyle en C1-C4, alkylsulfonyle en C1-C4, halogénoalcoxy en C1-C4, halogénoalkylthio en C1-C4, halogénoalkylsulfinyle en C1-C4 ou halogénoalkylsulfonyle en C1-C4, ou un groupe alcényle en C2-C6 portant éventuellement les substituants énumérés ou un groupe halogénoalcényle en C2-C6,

E représente le groupe méthine ou l'azote,

X représente l'oxygène, le soufre, un pont sulfinyle ou sulfonyle,

Z représente l'oxygène ou le soufre,

m est égal à 1 ou 2,

$R_1$ représente l'hydrogène, un halogène, un groupe alkyle en C1-C5, alcényle en C2-C5 ou —Y—$R_5$,

$R_2$ représente l'hydrogène, un halogène, un groupe alkyle en C1-C5, alcényle en C2-C5, halogénoalkyle en C1-C4 ou un reste —Y—$R_5$, —$COOR_6$, —$NO_2$ ou —CO—$NR_7R_8$,

$R_3$ et $R_4$ représentent, indépendamment l'un de l'autre, l'hydrogène, un groupe alkyle en C1-C4, alcoxy en C1-C4, alkylthio en C1-C4, halogénoalkyle en C1-C4, un halogène ou un groupe alcoxyalkyle contenant au maximum 4 atomes de carbone,

$R_5$ et $R_6$ représentent chacun un groupe alkyle en C1-C5, alcényle en C2-C5 ou alcynyle en C2-C6,

$R_7$ et $R_8$ représentent chacun, indépendamment l'un de l'autre, l'hydrogène, un groupe alkyle en C1-C5, alcényle en C2-C5 ou alcynyle en C2-C6, et

Y représente l'oxygène, le soufre, un pont sulfinyle ou sulfonyle,

étant spécifié que m est égal à 2, lorsque, simultanément, X représente le soufre, un pont sulfinyle ou sulfonyle et A représente un groupe alcényle en C3-C4.

2. Produit selon la revendication 1, caractérisé en ce que Z représente l'oxygène.

3. Produit selon la revendication 1, caractérisé en ce que les groupes $R_3$ et $R_4$, ensemble, ne contiennent pas plus de 4 atomes de carbone.

4. Produit selon la revendication 1, caractérisé en ce que Z représente l'oxygène et m est égal à 1.

5. Produit selon la revendication 1, caractérisé en ce que Z représente l'oxygène et m est égal à 2.

6. Produit selon la revendication 4, caractérisé en ce que le reste —X—A est en position 2 ou 3 par rapport au groupe sulfonyle.

7. Produit selon la revendication 6, caractérisé en ce que le reste —X—A est en position 2.

8. Produit selon la revendication 5, caractérisé en ce que les deux restes —X—A sont dans les positions 2 et 5 par rapport au groupe sulfonyle.

9. Produit selon la revendiction 2, caractérisé en ce qu'il n'y a qu'un seul reste —X—A en position 2 par rapport au groupe sulfonyle et en ce que les restes $R_3$ et $R_4$, ensemble, ne contiennent pas plus de 4 atomes de carbone.

10. Produit selon la revendication 2, caractérisé en ce qu'il y a deux restes —X—A dans les positions 2 et 5 par rapport au groupe sulfonyle et en ce que les restes $R_3$ et $R_4$, ensemble, ne contiennent pas plus de 4 atomes de carbone.

11. Produit selon la revendication 9, caractérisé en ce que $R_1$ représente l'hydrogène et en ce que le reste $R_2$ est en position 5 ou 6 par rapport au groupe sulfonyle.

12. Produit selon la revendication 11, caractérisé en ce que $R_2$ représente l'hydrogène, un halogène, un groupe alcoxy en C1-C4, nitro ou —$COOR_6$.

13. Produit selon la revendication 12, caractérisé en ce que $R_2$ représente l'hydrogène, le fluor, un groupe nitro ou alcoxy en C1-C4 et $R_3$ et $R_4$ représentent chacun l'hydrogène, un groupe alkyle en C1-C4, alcoxy en C1-C4, alkylthio en C1-C4, un halogène ou un groupe alcoxyalkyle, et $R_3$ et $R_4$, ensemble, contiennent au maximum 4 atomes de carbone.

14. Produit selon la revendication 13, caractérisé en ce que $R_2$ représente l'hydrogène et $R_3$ et $R_4$ représentent chacun un groupe alkyle en C1-C4, alcoxy en C1-C4, méthylthio, un halogène ou un groupe alcoxyalkyle.

15. Produit selon la revendication 14, caractérisé en ce que X représente l'oxygène ou le soufre.

16. Produit selon la revendication 15, caractérisé en ce que A représente un groupe alcoxyalkyle en C2-C8, halogénoalcényle en C2-C4 ou alcényle en C2-C6.

17. Produit selon la revendication 16, caractérisé en ce que A représente un groupe alcoxyalkyle en C2-C8 et $R_3$ et $R_4$ représentent chacun un groupe méthyle, éthyle, le chlore ou un groupe méthoxy.

18. Produit selon la revendication 16, caractérisé en ce que A représente un groupe alcényle en C2-C6 et $R_3$ et $R_4$ représentent chacun un groupe méthyle, éthyle, le chlore ou un groupe méthoxy.

19. Produit selon la revendication 16, caractérisé en ce que A représente un groupe vinyle substitué par un à trois atomes d'halogènes et $R_3$ et $R_4$ représentent chacun un groupe méthyle, éthyle, le chlore ou un groupe méthoxy.

20. Produit selon la revendication 1, caractérisé en ce que Z représente le soufre, X l'oxygène, $R_3$ et $R_4$ représentent chacun, indépendamment l'un de l'autre, un groupe alkyle en C1-C3, alcoxy en C1-C3 ou alkylthio en C1-C3 contenant ensemble au maximum 4 atomes de carbone, et A représente un groupe $—CH_2—CH = CH_2$, $—CH_2—CH = CH—CH_3$, $—CH_2—CH(CH_3) = CH_2$, méthoxyéthyle, méthoxyméthyle ou $—CCl = CHCl$, le reste —X—A occupe la position 2 et m est égal à 1.

21. Produit selon la revendication 1, caractérisé en ce qu'il contient en tant que substance active la N-(2-allyloxyphénylsulfonyl)-N'-(4-méthoxy-6-méthyl-1,3,5-triazine-2-yl)-urée.

22. Procédé de préparation des composés de formule I selon la revendication 1, caractérisé en ce que

a) on fait réagir un phénylsulfonamide de formule II

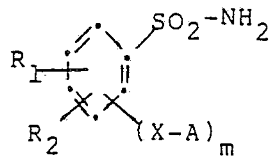

(II)

dans laquelle A, $R_1$, $R_2$, X et m ont les significations indiquées en référence à la formule I, en présence d'une base, avec un N-pyrimidinyl- ou -triazinyl-carbamate de formule

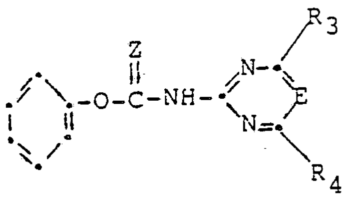

(III)

dans laquelle E, $R_3$, $R_4$ et Z ont les significations indiquées en référence à la formule I, ou bien

b) on fait réagir un phénylsulfonylisocyanate ou -isothiocyanate de formule IV

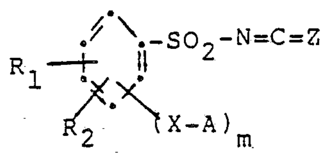

(IV)

dans laquelle A, $R_1$, $R_2$, m, X et Z ont les significations indiquées en référence à la formule I, éventuellement en présence d'une base, avec un amine de formule V

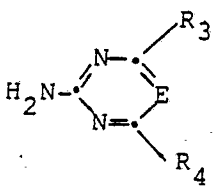

(V)

dans laquelle E, $R_3$ et $R_4$ ont les significations indiquées en référence à la formule I, ou bien

c) on fait réagir un sulfonamide de formule II ci-dessus, éventuellement en présence d'une base, avec un isocyanate ou isothiocyanate de formule VI

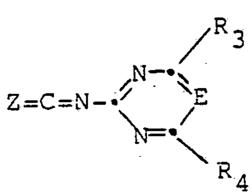

(VI)

dans laquelle E, $R_3$, $R_4$ et Z ont les significations indiquées en référence à la formule I, ou bien

d) on fait réagir un N-phénylsulfonylcarbamate de formule VII

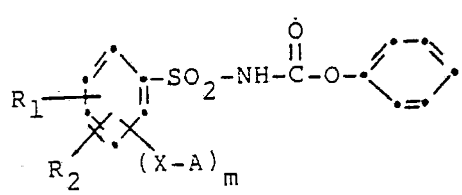

(VII)

dans laquelle A, $R_1$, $R_2$, m et X ont les significations indiquées en référence à la formule I avec une amine de formule V telle que définie ci-dessus, et le cas échéant, on convertit en un sel en faisant réagir la

sulfonylurée de formule I avec une amine, un hydroxyde de métal alcalin ou alcalino-terreux ou une base d'ammonium quaternaire.

23. Procédé de préparation des phénylsulfonamides de formule II

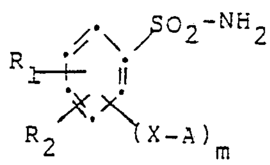

(II)

dans laquelle A, $R_1$, $R_2$, m et X ont les significations indiquées en référence à la formule I dans la revendication 1, caractérisé en ce que l'on convertit de manière connue en soi les anilides correspondants, par diazotation et échange du groupe diazo avec l'anhydride sulfureux en présence d'un catalyseur, dans l'acide chlorhydrique ou l'acide acétique, en les chlorures de phénylsulfonyle correspondants qu'on fait réagir avec une solution d'hydroxyde d'ammonium.

24. Procédé de préparation des phénylsulfonamides de formule VIII

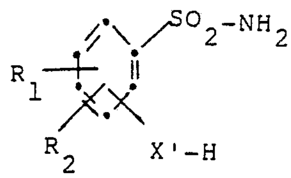

(VIII)

dans laquelle $R_1$ et $R_2$ ont les significations indiquées en référence à la formule I dans la revendication 1 et X représente l'oxygène ou le soufre, étant spécifié que $R_2$ ne peut représenter l'hydrogène et que l'un des substituants $R_2$ ou —X'—H est toujours en position 2 par rapport au groupe sulfonamide, caractérisé en ce que l'on convertit de manière connue en soi, les (alcoxy en C1-C4)-phénylsulfonamides correspondants, par une réaction de scission des éthers, en les hydroxyphénylsulfonamides libres, ou bien en ce que l'on convertit de manière connue en soi les benzyloxyphénylsulfonamides correspondants, par une réaction d'hydrogénolyse, en les hydroxyphénylsulfonamides libres.

25. Un agent herbicide et inhibiteur de la croissance des végétaux, caractérisé en ce qu'il contient en tant que substance active, avec des véhicules et/ou d'autres additifs, au moins une N-phénylsulfonyl-N'-triazinyl- ou -pyrimidinyl-urée de formule I, revendication 1.

26. L'utilisation des N-phénylsulfonyl-N'-triazinyl- ou -pyrimidinyl-urées de formule I, revendication 1, ou de produits en contenant, dans la lutte contre les croissances de végétaux indésirables.

27. L'utilisation des N-phénylsulfonyl-N'-triazinyl- ou -pyrimidinyl-urées de formule I, revendication 1, ou de produits en contenant, pour l'inhibition de la croissance des végétaux.

28. L'utilisation des N-phénylsulfonyl-N'-triazinyl ou -pyrimidinyl-urées de formule I ou de produits en contenant, selon la revendication 26, pour la lutte sélective en pré-levée ou en post-levée contre les mauvaises herbes dans les cultures de végétaux utiles.

29. L'utilisation des N-phénylsulfonyl-N'-triazinyl- ou -pyrimidinyl-urées de formule I ou de produits en contenant, selon la revendication 27, pour l'inhibition de la croissance des végétaux au-delà du stade deux feuilles, caractérisée en ce que l'on applique les substances actives en pré-levée.

30. Produit selon la revendication 1, caractérisé en ce qu'il contient en tant que substance active la N-(2-allyloxy-5-méthyl-phénylsulfonyl)-N'-(4-méthoxy-6-méthyl-1,3,5-triazine-2-yl)-urée.

31. Produit selon la revendication 1, caractérisé en ce qu'il contient en tant que substance active la N-[2-(2-méthallyloxy)-phénylsulfonyl]-N'-(4-méthoxy-6-méthyl-1,3,5-triazine-2-yl)-urée.

32. Produit selon la revendication 1, caractérisé en ce qu'il contient en tant que substance active la N-[2-(2-méthallyloxy)-phénylsulfonyl]-N'-(4,6-diméthoxy-1,3,5-triazine-2-yl)-urée.

33. Produit selon la revendication 1, caractérisé en ce qu'il contient en tant que substance active la N-[2-(2-méthallyloxy)-phénylsulfonyl]-N'-(4-éthyl-6-méthoxy-1,3,5-triazine-2-yl)-urée.

34. Produit selon la revendication 1, caractérisé en ce qu'il contient en tant que substance active la N-[2-(2-méthallyloxy)-phénylsulfonyl]-N'-(4,6-diéthoxy-1,3,5-triazine-2-yl)-urée.

35. Produit selon la revendication 1, caractérisé en ce qu'il contient en tant que substance active la N-[2-(2-buténylоxy)-phénylsulfonyl]-N'-(4-méthoxy-6-méthyl-1,3,5-triazine-2-yl)-urée.

36. Produit selon la revendication 1, caractérisé en ce qu'il contient en tant que substance active la N-[2-(2-buténylоxy)-phénylsulfonyl]-N'-(4,6-diméthoxy-1,3,5-triazine-2-yl)-urée.

37. Produit selon la revendication 1, caractérisé en ce qu'il contient en tant que substance active la N-[2-(1,2-dichlorovinyloxy)-phénylsulfonyl]-N'-(4-méthoxy-6-méthyl-1,3,5-triazine-2-yl)-urée.

38. Produit selon la revendication 1, caractérisé en ce qu'il contient en tant que substance active la N-(2-allyloxyphénylsulfonyl)-N'-(4,6-diéthoxy-1,3,5-triazine-2-yl)-urée.

39. Produit selon la revendication 1, caractérisé en ce qu'il contient en tant que substance active la N-(2-allyloxyphénylsulfonyl)-N'-(4-éthoxy-6-méthyl-1,3,5-triazine-2-yl)-urée.

40. Produit selon la revendication 1, caractérisé en ce qu'il contient en tant que substance active la N-(2-allyloxyphénylsulfonyl)-N'-(4-méthoxy-6-isopropyloxy-1,3,5-triazine-2-yl)-urée.

41. Produit selon la revendication 1, caractérisé en ce qu'il contient en tant que substance active la N-(2-allyloxyphénylsulfonyl)-N'-(4,6-diméthoxy-1,3,5-triazine-2-yl)-urée.

42. Produit selon la revendication 1, caractérisé en ce qu'il contient en tant que substance active la N-[2-(1,2-dichlorovinyloxy)-phénylsulfonyl]-N'-(4,6-diméthoxy-1,3,5-triazine-2-yl)-urée.

43. Produit selon la revendication 1, caractérisé en ce qu'il contient en tant que substance active la N-[2-(1,2-dichlorovinyloxy)-phénylsulfonyl]-N'-(4-chloro-6-méthoxy-pyrimidine-2-yl)-urée.

44. Produit selon la revendication 1, caractérisé en ce qu'il contient en tant que substance active la N-(2-méthoxy-éthoxy-phénylsulfonyl)-N'-(4-méthoxy-6-méthyl-1,3,5-triazine-2-yl)-urée.

45. Produit selon la revendication 1, caractérisé en ce qu'il contient en tant que substance active la N-(2-méthoxy-méthoxy-phénylsulfonyl)-N'-(4-méthoxy-6-méthyl-1,3,5-triazine-2-yl)-urée.

46. Produit selon la revendication 1, caractérisé en ce qu'il contient en tant que substance active la N-(2-méthoxy-éthylthio-phénylsulfonyl)-N'-(4-méthoxy-6-méthyl-1,3,5-triazine-2-yl)-urée.

47. Produit selon la revendication 1, caractérisé en ce qu'il contient en tant que substance active la N-(2-méthoxy-éthoxy-phénylsulfonyl)-N'-(4-chloro-6-méthoxy-pyrimidine-2-yl)-urée.

48. Produit selon la revendication 1, caractérisé en ce qu'il contient en tant que substance active la N-(2-méthoxy-éthoxy-phénylsulfonyl)-N'-(4-méthoxy-6-méthyl-pyrimidine-2-yl)-urée.